(19) Europäisches Patentamt
European Patent Office
Office européen des brevets

(11) EP 4 186 896 A1

(12) EUROPEAN PATENT APPLICATION
published in accordance with Art. 153(4) EPC

(43) Date of publication:
**31.05.2023 Bulletin 2023/22**

(21) Application number: **21847300.7**

(22) Date of filing: **22.07.2021**

(51) International Patent Classification (IPC):
***C07D 401/14** (2006.01)* ***C07D 491/10** (2006.01)*
***C07D 413/14** (2006.01)* ***C07D 513/10** (2006.01)*
***C07D 471/20** (2006.01)* ***C07D 487/04** (2006.01)*
***A61K 31/497** (2006.01)* ***A61P 35/02** (2006.01)*
***A61P 35/00** (2006.01)*

(52) Cooperative Patent Classification (CPC):
**A61K 31/497; A61P 35/00; A61P 35/02; C07D 401/14; C07D 413/14; C07D 471/20; C07D 487/04; C07D 491/10; C07D 513/10**

(86) International application number:
**PCT/CN2021/107808**

(87) International publication number:
**WO 2022/017444 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2020 PCT/CN2020/104473**
**30.09.2020 PCT/CN2020/119590**

(71) Applicant: **Betta Pharmaceuticals Co., Ltd**
**Yuhang**
**Hangzhou**
**Zhejiang 311100 (CN)**

(72) Inventors:
- **WU, Hao**
**Hangzhou, Zhejiang 311100 (CN)**
- **WU, Wenmao**
**Hangzhou, Zhejiang 311100 (CN)**
- **LI, Ling**
**Hangzhou, Zhejiang 311100 (CN)**
- **ZHANG, Zhan**
**Hangzhou, Zhejiang 311100 (CN)**
- **WANG, Feng**
**Hangzhou, Zhejiang 311100 (CN)**
- **YUAN, Ding**
**Hangzhou, Zhejiang 311100 (CN)**
- **WU, Yunfei**
**Hangzhou, Zhejiang 311100 (CN)**
- **CHEN, Qiang**
**Hangzhou, Zhejiang 311100 (CN)**
- **HAN, Han**
**Hangzhou, Zhejiang 311100 (CN)**
- **GUO, Jing**
**Beijing 100176 (CN)**
- **LAN, Hong**
**Beijing 100176 (CN)**
- **DING, Lieming**
**Hangzhou, Zhejiang 311100 (CN)**
- **WANG, Jiabing**
**Beijing 100176 (CN)**

(74) Representative: **IPAZ**
**16, rue Gaillon**
**75002 Paris (FR)**

(54) **SHP2 INHIBITOR AND COMPOSITION AND APPLICATION THEREOF**

(57) A compound (as represented by formula I) serving as an Src homology region 2-containing protein tyrosine phosphatase 2 (SHP2) inhibitor, as well as a pharmaceutical composition thereof, a preparation method therefor, and a use in treating an SHP2 mediation diseases. The compounds of formula I exerts an effect by means of participating in the regulation of multiple processes such as cell proliferation, apoptosis, migration, and angiogenesis.

EP 4 186 896 A1

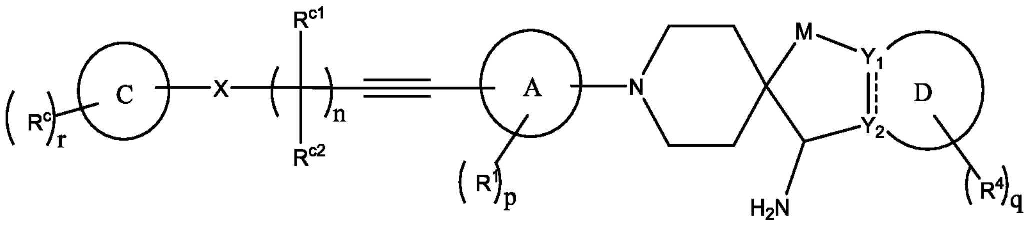

I

## Description

## FIELD OF THE INVENTION

The present invention relates to series of compounds as inhibitors of Src homologyregion 2-containing protein tyrosine phosphatase 2 (SHP2), methods and pharmaceutical compositions thereof. The present invention also relates to the use of the compounds or pharmaceutical compositions thereof for the treatment of SHP2-mediated diseases.

## BACKGROUND OF THE INVENTION

Src homologyregion 2-containing protein tyrosine phosphatase 2 (SHP2) is a non-receptor protein tyrosine phosphatase encoded by the PTPN11 gene. PTPN11 is the first discovered proto-oncogene encoding tyrosine phosphokinase(Chan R J et al. PTPN11 is the first identified proto-oncogene that encodes a tyrosine phosphatase. Blood, 2007, 109:862-867), and its encoded SHP2 protein contains N-terminal SHP2 domain (N-SHP2), C-terminal SHP2 domain (C-SHP2), protein phosphatase catalyzed domain (PTP), two C-terminal tyrosine residues (Y542 and Y580) and a proline-rich (Pro) motif.

In recent years, researches mainly believe that the Ras/ERK pathway is the most important signal transduction pathway for SHP2. The mechanism (Dance M et al. The molecular functions of SHP2 in the RAS/mitogen-activated protein kinase (ERK1/2) pathway. Cell Signal, 2008, 20:453-459) is roughly as follows: after activation of growth factor receptors, its tyrosine residues are autophosphorylated to provide docking sites for the phosphotyrosine-binding region SH2 of Grb2 and SHP2 (the SH2 domain-containing adaptor protein). Binding of Grb2 to phosphorylated growth factor receptors results in the accumulation of SOS proteins at the cell membrane. As a guanine nucleotide exchange factor (GEF), SOS can catalyze the conversion of membrane-bound protein Ras from inactive Ras-GDP to active Ras-GTP. Ras-GTP further connects with the downstream signaling system, activates the Ser/Thr kinase Raf1, etc., and then activates ERK under the action of the regulatory kinase MEK. After activation, ERK directly acts on cytoplasmic target molecules or transfers to the nucleus to regulate genes Transcribe to make cells proliferate or differentiate. This process may also be influenced by SHP2-binding proteins and substrates (SHP substrate-1, SHPS-1), Ras-GTPase activating protein (Ras-GAP), and other Src members.

It is reported that SHP2 protein not only regulates the Ras/ERK signaling pathway, but also regulates multiple signaling pathways such as JAK-STAT3, NF-κB, PI3K/Akt, RHO and NFAT, thereby regulating cell proliferation, differentiation, migration, apoptosis and other physiology Function.

SHP2 has been shown to be associated with various diseases, and Tartaglia et al. (Tartaglia M et al. Mutations in PTPN11, encoding the protein tyrosine phosphatase SHP-2, cause Noonan sydrome. Nat Genet, 2001, 29:465-468) found that about 50% of Patients with Noonan syndrome have missense mutations in PTPN11. In addition, studies have found that PTPN11 mutation is an important cause of JMMLL and various leukemias (Tartaglia M et al. Nat Genet, 2003, 34: 148-150; Loh ML et al. Blood, 2004, 103: 2325-2331; Tartaglia M et al. Br J Haematol, 2005, 129:333-339; Xu R et al. Blood, 2005, 106:3142-3149.). With further study of PTPN11/SHP2, it has been found that it is related to the occurrence of lung cancer, gastric cancer, colon cancer, melanoma, thyroid cancer and other cancers (Tang Chunlan et al. Chinese Journal of Lung Cancer, 2010, 13:98- 101; Higuchi M et al. Cancer Sci, 2004, 95:442-447; Bentires-Alj M et al. Cancer Res, 2004, 64:8816-8820; Martinelli S et al. Cancer Genet Cytogenet, 2006, 166: 124-129).

At present, SHP2 inhibitors have received more and more attention as a potential therapeutic approach. However, this target has not yet been marketed at home and abroad. Therefore, it is of great research significance to develop small-molecule drugs that can target and inhibit the activity of SHP2 and provide patients with safer and more effective SHP2 inhibitors.

In the WO2019183367 patent published on September 26, 2019, the structure of compound 243 is disclosed as follows: According to the patent, the $IC_{50}$ of compound 243 in the SHP2 allosteric inhibition test is greater than 50nM and less than or equal to 1μM, which needs further improvement.

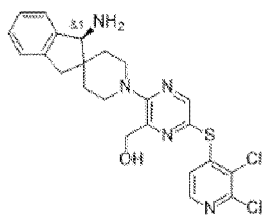

Compound 243 in WO2019183367

**SUMMARY OF INVENTION**

The present invention relates to a compound as an inhibitor of Src homology region 2 protein tyrosine phosphatase 2 (SHP2), or a pharmaceutically acceptable salt, deuterated product, cis-trans isomer and tautomer thereof, solvate, chelate, non-covalent complex or prodrug, the compound is shown in Formula I:

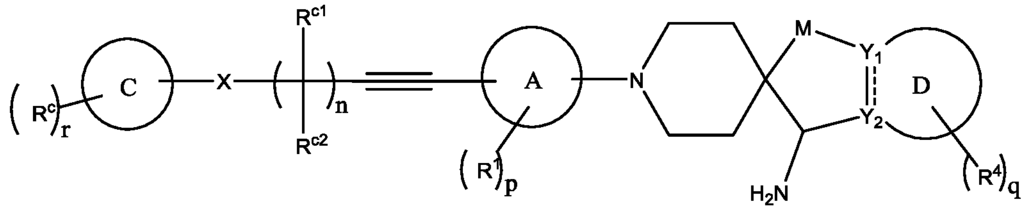

Formula I

wherein,

- - - - - - is a single bond or a double bond;

ring A is selected from 5- to 10- membered heteroaryl (such as 6- membered heteroaryl, 9- membered heteroaryl, etc) or 5- to 10- membered heterocyclyl (such as 6- membered heterocyclyl, 9- membered heterocyclyl, etc.); Wherein, the 5- to 10-membered heteroaryl or the 5- to 10- membered heterocyclyl contains one or more O, N or S heteroatoms; the 5- to 10- membered heterocyclyl contains at least one double bond;

ring C is selected from $C_{3-10}$ carbocyclyl, 3- to 10- membered saturated or partially saturated heterocyclyl, $C_{6-10}$ aryl or 5- to 10- membered heteroaryl;

each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$, $-OC_{2-6}$ alkenyl $C_{1-6}$ alkoxy, $-OC_{2-6}$ alkenyl, $C_{6-10}$ aryl,

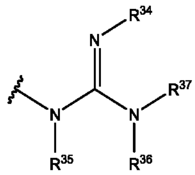 and 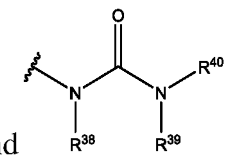 ;

wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, and 5- to 10- membered heteroaryl are optional is substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ Carbocyclyl, 3- to 10- membered heterocyclyl, $C_{6-10}$ aryl, 5- to 10-membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$ $-NR^{28}S(=O)_2R^{29}$, $-OC(=O)R^{30}$, $-S(=O)R^{31}$, $-P(=O)R^{32}R^{33}$,

two $R^c$ together with the atom to which they are attached atoms form $C_{3-8}$ carbocyclyl, 3- to 10- membered heterocyclyl, $C_{6-10}$ aryl, 5- to 10- membered heteroaryl; wherein, the $C_{3-8}$ carbocyclyl, 3- to 10- membered heterocyclyl, $C_{6-10}$ aryl, 5- to 10- heteroaryl is optionally substituted with one or more substituents; the substituents are independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$,$-NR^{20}(CH_2)_tNR^{21}R^{22}$,$-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$, $-OC_{2-6}$ alkenyl, $C_{1-6}$ alkoxy, $-OC_{2-6}$ alkenyl, $-C_{6-10}$ aryl,

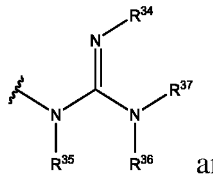 and 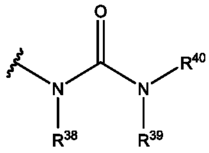 ;

Wherein, the $C_{1-8}$ alkyl, $C_{2-8}$ alkenyl, $C_{2-8}$ alkynyl, $C_{3-8}$ carbocyclyl, 3- to 10-membered heterocyclyl, $C_{6-10}$ aryl, 5- to 10- membered heteroaryl optionally substituted by one or more substituents; the substituents are selected from hydrogen, halogen, -CN, -$NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl, -$OR^5$, -$SR^6$, -$NR^7R^8$, -(C=O)$R^9$, -C(=O)$OR^{10}$, -C(=O)$NR^{11}R^{12}$, -S(=O)$_2R^{13}$, -S(=O)$_2OR^{14}$, -S(=O)$_2NR^{15}R^{16}$, -O$(CH_2)_sOR^{17}$, -O$(CH_2)_sNR^{18}R^{19}$, -$NR^{20}(CH_2)_tNR^{21}R^{22}$, -$NR^{23}(CH_2)_tOR^{24}$, -$NR^{25}C(=O)R^{26}$, -OS(=O)$_2R^{27}$, -$NR^{28}S(=O)_2R^{29}$, -OC(=O)$R^{30}$, -S(=O)$R^{31}$, -$P^1$(=O)$R^{32}R^{33}$;

$R^{c1}$, $R^{c2}$ are independently selected from hydrogen, halogen, $C_{1-4}$ alkyl or -$OR^5$;

or $R^{c1}$ and $R^{c2}$ with connected atoms form $C_{3-6}$ carbocyclyl, 3- to 6- membered heterocyclyl;

each $R^1$ is independently selected from hydrogen, halogen, -CN, -$NO_2$, oxo, $C_{1-4}$ alkyl, $C_{2-4}$ alkenyl, $C_{2-4}$ alkynyl, $C_{3-6}$ carbocyclyl, 3- to 6- membered heterocyclyl, $C_{6-10}$ aryl, 5- to 10- membered heteroaryl, -$OR^5$, -$SR^6$, -$NR^7R^8$, -(C=O)$R^9$, -C(=O)$OR^{10}$, -C(=O)$NR^{11}R^{12}$, -S(=O)$_2R^{13}$, -S(=O)$_2OR^{14}$, -S(=O)$_2NR^{15}R^{16}$, -O$(CH_2)_sOR^{17}$, -O$(CH_2)_sNR^{18}R^{19}$, -$NR^{20}(CH_2)_tNR^{21}R^{22}$, -$NR^{23}(CH_2)_tOR^{24}$, -$NR^{25}C(=O)R^{26}$, -OS(=O)$_2R^{27}$, -$NR^{28}S(=O)_2R^{29}$, -OC(=O)$R^{30}$, -S(=O)$R^{31}$, -P(=O)$R^{32}R^{33}$ ; wherein, the $C_{1-4}$ alkyl, $C_{2-4}$ alkenyl, $C_{2-4}$ alkynyl, $C_{3-6}$ carbocyclyl, 3-to 6-membered heterocyclyl, $C_{6-10}$ aryl, 5- to 10- membered heteroaryl is optionally substituted by one or more substituents; the substituents are selected from hydrogen, halogen, -CN, -$NO_2$, oxo, $C_{1-4}$ alkyl, $C_{2-4}$ alkenyl, $C_{2-4}$ alkynyl, $C_{3-6}$ carbocyclyl, 3- to 6- membered heterocyclyl, $C_{6-10}$ aryl, 5- to 10- membered heteroaryl, -$OR^5$, -$SR^6$, -$NR^7R^8$, -(C=O)$R^9$, -C(=O)$OR^{10}$ or -C(=O)$NR^{11}R^{12}$;

X is selected from -O, -N($R^{1X}$), -S, -C(=O), -C(=O)O, -C(=O)N($R^{2X}$), -S(=O)$_2$, -S(=O)$_2$O, -S(=O)$_2$N($R^{3X}$), -S(=O), -S(=O)O, -S(=O)N($R^{4X}$), -OS(=O)$_2$O,

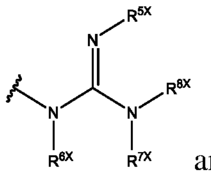 and 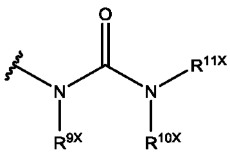 ;

$R^{1X}$, $R^{2X}$, $R^{3X}$, $R^{4X}$, $R^{5X}$, $R^{6X}$, $R^{7X}$, $R^{8X}$, $R^{9X}$, $R^{10X}$, $R^{11X}$ are independently selected from hydrogen, $C_{1-3}$ alkyl, $C_{3-6}$ cycloalkyl or 3- to 6- membered heterocyclyl;

M is selected from -O, -$NR^{1a}$ or -$CR^{1b}R^{1c}$;

$R^{1a}$ is selected from hydrogen, $C_{1-3}$ alkyl, $C_{3-6}$ cycloalkyl, 3- to 6- membered heterocyclyl or absent;

$R^{1b}$, $R^{1c}$ are independently selected from hydrogen, $C_{1-3}$ alkyl, $C_{3-6}$ cycloalkyl, 3-to 6- membered heterocyclyl, -$OR^{1d}$, -$NR^{1e}R^{1f}$ or absent;

$R^{1d}$, $R^{1e}$, $R^{1f}$ are independently selected from hydrogen, $C_{1-3}$ alkyl, $C_{3-6}$ cycloalkyl or 3- to 6- membered heterocyclyl;

$Y_1$ is selected from -$NR^{3a}$ or -$CR^{3b}R^{3c}$;

$R^{3a}$ is selected from hydrogen, $C_{1-3}$ alkyl, $C_{3-6}$ cycloalkyl, 3- to 6- membered heterocyclyl or absent;

$R^{3b}$, $R^{3c}$ are independently selected from hydrogen, $C_{1-3}$ alkyl, $C_{3-6}$ cycloalkyl, 3-to 6- membered heterocyclyl or absent;

$Y_2$ is selected from -$NR^{4a}$ or -$CR^{4b}R^{4c}$;

$R^{4a}$ is selected from hydrogen, $C_{1-3}$ alkyl, $C_{3-6}$ cycloalkyl, 3- to 6- membered heterocyclyl or absent;

$R^{4b}$, $R^{4c}$ are independently selected from hydrogen, $C_{1-3}$ alkyl, $C_{3-6}$ cycloalkyl, 3-to 6- membered heterocyclyl or absent;

The ring D is $C_{6-10}$ aryl, 5- to 10- membered heteroaryl, $C_{3-8}$ carbocyclyl, 3- to 8-membered heterocyclyl or absent; wherein, the $C_{3-8}$ carbocyclyl or 3- to 8- membered heterocyclyl optionally contains double bond;

each $R^4$ is independently selected from hydrogen, halogen, -CN, -$NO_2$, oxo, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, -$OR^5$, -$SR^6$, -$NR^7R^8$, -(C=O)$R^9$, -C(=O)$OR^{10}$, -C(=O)$NR^{11}R^{12}$, -S(=O)$_2R^{13}$, -S(=O)$_2OR^{14}$, -S(=O)$_2NR^{15}R^{16}$, -O$(CH_2)_sOR^{17}$, -O$(CH_2)_sNR^{18}R^{19}$, -$NR^{20}(CH_2)_tNR^{21}R^{22}$, -$NR^{23}(CH_2)_tOR^{24}$; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, 5- to 10- membered heteroaryl is optionally

substituted with one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$;
$R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$ are independently selected from hydrogen, $C_{1-8}$ alkyl, $C_{2-8}$ alkenyl, $C_{2-8}$ alkynyl, $C_{3-8}$ carbon cyclyl, 3- to 10- membered heterocyclyl, $C_{6-10}$ aryl, 5- to 10 membered heteroaryl; wherein, $C_{1-8}$ alkyl, $C_{2-8}$ alkenyl, $C_{2-8}$ alkynyl, $C_{3-8}$ carbocyclyl, 3- to 10-membered heterocyclyl, $C_{6-10}$ membered aryl, 5- to 10- membered heteroaryl are optionally substituted with one or more substituents; the substituents are selected from hydrogen, halogen, -CN, $-NO_2$, oxo, -OH, $-NH_2$, $C_{1-4}$ alkyl, $C_{1-4}$ alkoxy, $C_{2-4}$ alkenyl, $C_{2-4}$ alkynyl, $C_{3-10}$ carbocyclyl, 3- to 10- membered heterocyclyl, $C_{6-10}$ aryl, 5- to 10-membered heteroaryl;
n is 0, 1, 2 or 3;
p is 0, 1, 2, or 3;
q is 0, 1, 2, 3 or 4;
r is 0, 1, 2, 3 or 4;
s is 1, 2, 3 or 4;
t is 1, 2, 3 or 4.

In some embodiments, in the Formula I, ring C is selected from 3- to 10-membered saturated or partially saturated heterocyclyl, $C_{6-10}$ aryl or 5- to 10-membered heteroaryl.

In some embodiments, in the Formula I, the ring D is $C_{6-10}$ aryl, 5- to 10-membered heteroaryl, $C_{3-8}$ carbocyclyl or 3- to 8- membered heterocyclyl; wherein, the $C_{3-8}$ carbocyclyl or 3- to 8- membered heterocyclyl optionally contains double bond.

In some embodiments, in the Formula I, ring A is selected from 6- to 10-membered heteroaryl or a 6- to 10-membered heterocyclyl; the 6- to 10- membered heterocyclyl contains at least one double bond.

In some embodiments, in the Formula I, ring A is selected from a 6- membered heteroaryl or a 6- membered heterocyclyl; the 6- membered heterocyclyl contains at least one double bond.

In some embodiments, in the Formula I, ring A is selected from

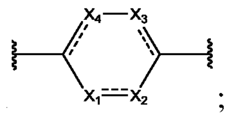
;

wherein, $X_1$, $X_2$, $X_3$, $X_4$ are independently selected from $-NR^1$ or $-CR^1R^1$; - - - - - - is single bond or double bond.

In some embodiments, in the Formula I, ring A is selected from

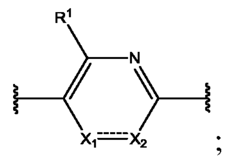
;

wherein, $X_1$, $X_2$ are independently selected from $-NR^1$ or $-CR^1R^1$; - - - - - - is single bond or double bond.

In some embodiments, in the Formula I, ring A is selected from 9- to 10-membered heteroaryl or 9- to 10-membered heterocyclyl; the 9- to 10- membered heterocyclyl contains at least one double bond.

In some embodiments, in the Formula I, ring A is selected from the group consisting of pyridyl, pyrazinyl, pyrimidinone, pyrimidinyl, thienyl, thiazolyl, furanyl, oxazolyl, indolyl, indazolyl , quinolyl and isoquinolyl.

In some embodiments, in the Formula I, ring A is selected from

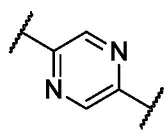 or 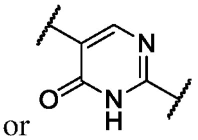.

In some embodiments, in the Formula I, the ring C is selected from $C_{3-10}$ carbocyclyl or 3- to 10- membered

heterocyclyl.

In some embodiments, in the Formula I, the ring C is selected from cyclopropyl, cyclobutyl, cyclopentyl, cyclohexyl, morpholinyl, piperazinyl, or piperidinyl.

In some embodiments, in the Formula I, the ring C is selected from $C_{6-10}$ aryl or 5- to 10- membered heteroaryl.

In some embodiments, in the Formula I, ring C is selected from phenyl, pyrrolyl, furyl, thienyl, oxazolyl, imidazolyl, pyrazolyl, thiazolyl, pyridyl, pyrimidinyl, Pyridazinyl, pyrazinyl, pyridinone, indolyl, benzoxazolyl, indazolyl, benzimidazolyl, benzotriazolyl, pyrrolopyridyl, quinolyl, naphthyl, dihydro Isobenzofuranyl, benzobisoxazolyl, tetrahydroquinolinyl, dihydropyrrolopyridyl, dihydronaphthyridinyl, dihydropyridopyrimidinonyl, isoindolinyl, dihydropyrazole pyrimidyl, cyclohexenyl, tetrahydrobenzazepine, dihydrobenzimidazolone, indolinone, isoindolinone, dihydroquinolinone, dihydroisoquinolinone, benzoxazinone, quinolinone, benzobisoxazolyl, dihydrobenzodioxin, pyridone, indazolyl.

In some embodiments, in the Formula I, the ring C is selected from

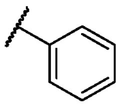,

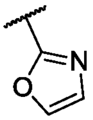, 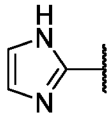, 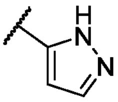, 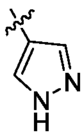, 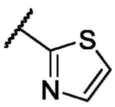, 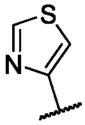, 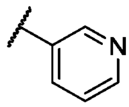,

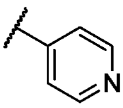, 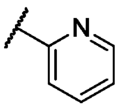, 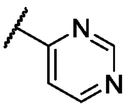, 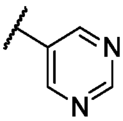, 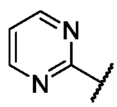, 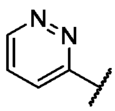, 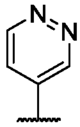, 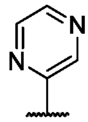,

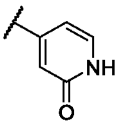, 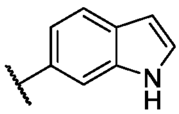, 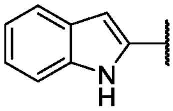, 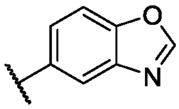, 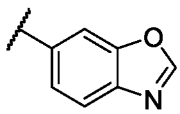,

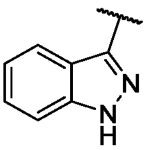, 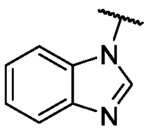, 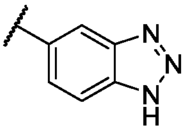, 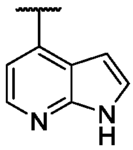, 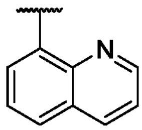, 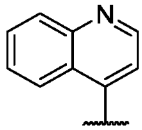,

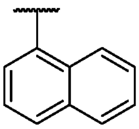, 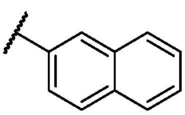, 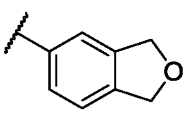, 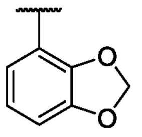, 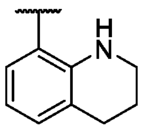, 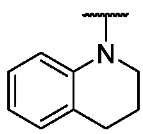,

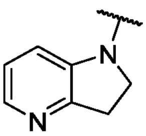, 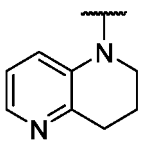, 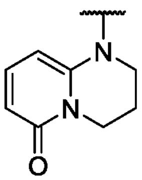, 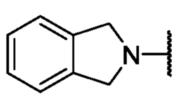, 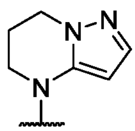, 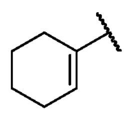,

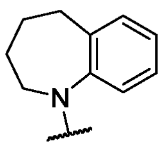, 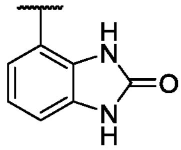, 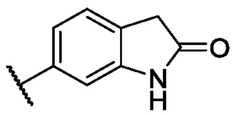, 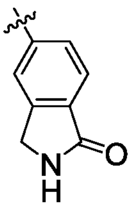, 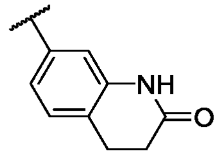,

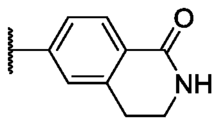, 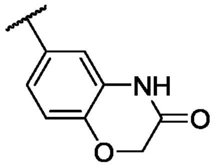, 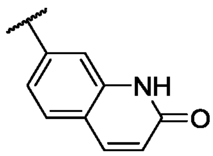, 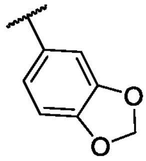, 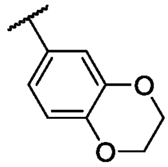,

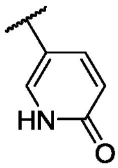, 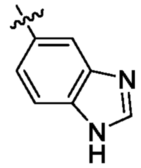.

In some embodiments, in the Formula I, ring C is selected from

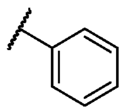,

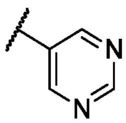, 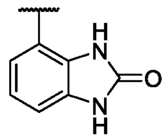, 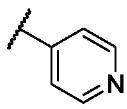, 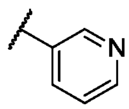, 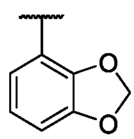, 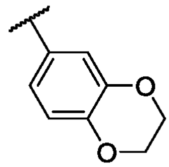,

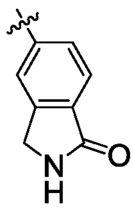, 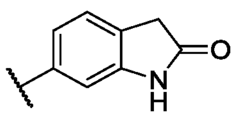 or 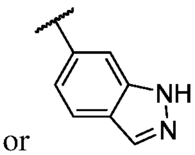.

In some embodiments, in the Formula I, n is 1.

In some embodiments, in the Formula I, n is 2.

In some embodiments, in the Formula I, each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, 5- to 10-membered heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C({=}O)R^{26}$, $-OS({=}O)_2R^{27}$ $-NR^{28}S({=}O)_2R^{29}$, $-OC({=}O)R^{30}$, $-S({=}O)R^{31}$, $-P({=}O)R^{32}R^{33}$.

In some embodiments, in the Formula I, each $R^c$ is independently selected from hydrogen, oxo, $-CH_3$, $-OCH_3$, -OH, $-OCF_3$, -CN, -F, -Cl, $-CF_3$ , $-NH_2$, $-NHCH_3$, $-NH(C{=}O)CH_3$, $-CH_2OH$, $-C({=}O)CH_3$, $-C({=}O)NH_2$, $-C({=}O)NHCH_3$, $-C({=}O)N\ (CH_3)_2$, -COOH, -COOEt, $-S({=}O)_2CH_3$, $-S({=}O)_2NH_2$, $-P({=}O)(CH_3)_2$, $-CH_2OCH_3$, $-CH_2C({=}O)NH_2$, ,

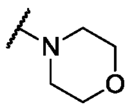, 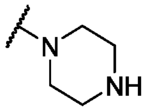, 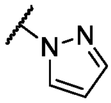, 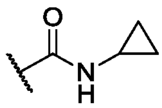, 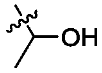,

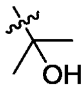, 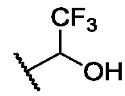, 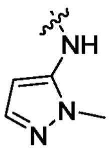 or 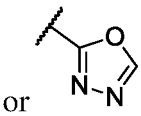.

In some embodiments, in the Formula I, $R^{c1}$, $R^{c2}$ are independently selected from hydrogen, halogen, $C_{1-4}$ alkyl or $-OR^5$.

In some embodiments, in the Formula I, $R^{c1}$, $R^{c2}$ are independently selected from hydrogen, $-CH_3$, -F, -OH.

In some embodiments, in the Formula I, $R^{c1}$, $R^{c2}$ with connected atom form a cyclopropyl.

In some embodiments, in the Formula I, each $R^1$ is independently selected from hydrogen, halogen, oxo, $C_{1-4}$ alkyl, $-NR^7R^8$, $-C(=O)NR^{11}R^{12}$; wherein, the $C_{1-4}$ alkyl is optionally substituted with one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-6}$ carbocyclyl, 3- to 6- membered heterocyclyl, $C_{6-10}$ membered aryl, 5- to 10-membered heteroaryl, $-OR^5$, $-SR^6$, $-NR^7R^8$, $-(C=O)R^9$, $-C(=O)OR^{10}$ or $-C(=O)NR^{11}R^{12}$,

In some embodiments, in the Formula I, each $R^1$ is independently selected from hydrogen, oxo, $-CH_3$, $-NH_2$, $-CH_2OH$, $-C(=O)NH_2$.

In some embodiments, in the Formula I, X is selected from -O-, $-N(R^{1X})-$, -C(=O)- or $-C(=O)N(R^{2X})-$.

In some embodiments, in the Formula I, X is selected from -O-, -NH-, -C(=O)- or -C(=O)NH-.

In some embodiments, in the Formula I, M is selected from -O- or $-CR^{1b}R^{1c}-$.

In some embodiments, in the Formula I, $R^{1b}$ and $R^{1c}$ are independently selected from hydrogen, $C_{1-3}$ alkyl, $C_{3-6}$ cycloalkyl, 3- to 6- membered heterocyclyl, $-OR^{1d}$ or $-NR^{1e}R^{1f}$.

In some embodiments, in the Formula I, $Y_1$ is selected from $-NR^{3a}-$ and $Y_2$ is selected from $-CR^{4b}R^{4c}-$.

In some embodiments, in the Formula I, $Y_1$ is selected from $-CR^{3b}R^{3c}-$, and $Y_2$ is selected from $-NR^{4a}-$.

In some embodiments, in the Formula I, $Y_1$ is selected from $-CR^{3b}R^{3c}-$ and $Y_2$ is selected from $-CR^{4b}R^{4c}-$.

In some embodiments, in the Formula I, the ring D is a $C_{6-10}$ aryl or a 5- to 10-membered heteroaryl.

In some embodiments, in the Formula I, the ring D is

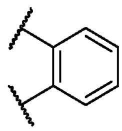, 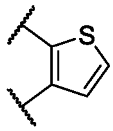,

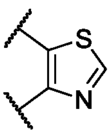, 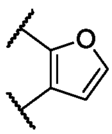, 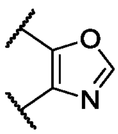, 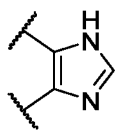, 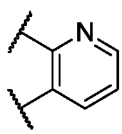 or 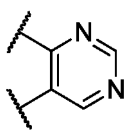.

In some embodiments, in the Formula I, the ring D is

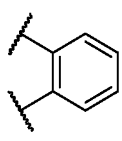

In some embodiments, in the Formula I, each $R^4$ is independently selected from hydrogen, halogen, oxo, $C_{1-8}$ alkyl or $-OR^5$.

In some embodiments, in the Formula I, each $R^4$ is independently selected from hydrogen, oxo, $-CH_3$ or $-OCH_3$.

In some embodiments, in the Formula I, X is selected from -O-.

In some embodiments, in the Formula I, X is selected from $-N(R^{1X})-$,

In some embodiments, in the Formula I, $R^{1X}$ is selected from hydrogen or methyl.

In some embodiments, in the Formula I, p is 0, 1 or 2 or 3.

In some embodiments, in the Formula I, q is 0, 1 or 2.

In some embodiments, in the Formula I, r is 0, 1, or 2.

In some embodiments, the compound of Formula I, or a pharmaceutically acceptable salt, deuterated compound, cis-trans isomer, tautomer, solvate, chelate, non-covalent complex or precursor thereof, selected from Formula II:

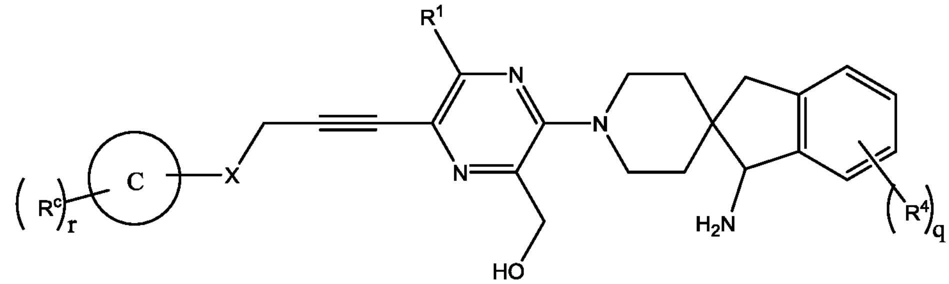

Formula II

ring C is selected from $C_{3-10}$ carbocyclyl, 3- to 10- membered saturated or partially saturated heterocyclyl, $C_{6-10}$ aryl or 5- to 10- membered heteroaryl;

each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C({=}O)R^{26}$, $-OS({=}O)_2R^{27}$, $-NR^{28}S({=}O)_2R^{29}$, $-OC({=}O)R^{30}$, $-S({=}O)R^{31}$, $-P({=}O)R^{32}R^{33}$, $-OC_{2-6}$ alkenyl $C_{1-6}$ alkoxy, $-OC_{2-6}$ alkenyl $C_{6-10}$ aryl,

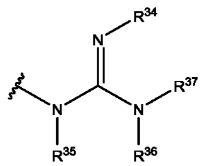 and 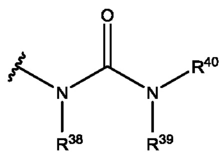 ;

wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10-membered heteroaryl optionally substituted with one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- member heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C({=}O)R^{26}$, $-OS({=}O)_2R^{27}$, $-NR^{28}S({=}O)_2R^{29}$, $-OC({=}O)R^{30}$, $-S({=}O)R^{31}$, $-P({=}O)R^{32}R^{33}$; two $R^c$ with connected atoms $C_{3-8}$ carbocyclyl, 3- to 10- membered heterocyclyl, $C_{6-10}$ aryl, 5- to 10- membered heteroaryl; wherein, the $C_{3-8}$ carbocyclyl, 3- to 10-membered heterocyclyl, $C_{6-10}$ aryl, and 5- to 10- membered heteroaryl are optionally substituted by one or more substituents; the substituents are each independently is selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10-membered heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C({=}O)R^{26}$, $-OS({=}O)_2R^{27}$ $-NR^{28}S({=}O)_2R^{29}$, $-OC({=}O)R^{30}$, $-S({=}O)R^{31}$, $-P({=}O)R^{32}R^{33}$, $-OC_{2-6}$ alkenyl $C_{1-6}$ alkoxy, $-OC_{2-6}$ alkenyl $C_{6-10}$ aryl,

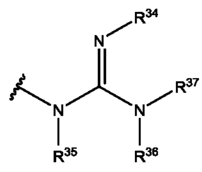 and 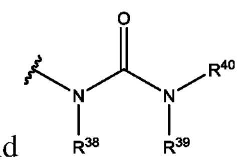 ;

wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5-

to 10- membered heteroaryl optionally substituted with one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- member heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$;

$R^1$ is selected from hydrogen, halogen, -CN, $-NO_2$, oxo, $C_{1-4}$ alkyl, $C_{2-4}$ alkenyl, $C_{2-4}$ alkynyl, $-OR^5$, $-NR^7R^8$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$; wherein, the $C_{1-4}$ alkyl, $C_{2-4}$ alkenyl or $C_{2-4}$ alkynyl is optionally substituted by one or more substituents; the substituent is selected from hydrogen, halogen, -CN, $-NO_2$, oxo, $C_{1-4}$ alkyl, $C_{2-4}$ alkenyl, $C_{2-4}$ alkynyl, $C_{3-6}$ carbocyclyl, 3-6 membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$ or $-C(=O)NR^{11}R^{12}$;

X selected from -O-, $-N(R^{1X})-$, -S-, -C(=O)-, -C(=O)O-, $-C(=O)N(R^{2X})-$, $-S(=O)_2-$, $-S(=O)_2O-$, $-S(=O)_2N(R^{3X})-$, -S(=O)-, -S(=O)O-, $-S(=O)N(R^{4X})-$, $-OS(=O)_2O-$,

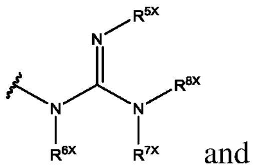
and
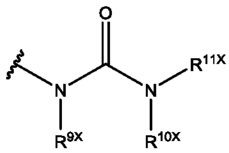
;

$R^{1X}$, $R^{2X}$, $R^{3X}$, $R^{4X}$, $R^{5X}$, $R^{6X}$, $R^{7X}$, $R^{8X}$, $R^{9X}$, $R^{10X}$, $R^{11X}$ are independently selected from hydrogen, $C_{1-3}$ alkyl, $C_{3-6}$ cycloalkyl or 3- to 6- membered heterocyclyl;

each $R^4$ is independently selected from hydrogen, halogen, -CN, $-NO_2$, oxo, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl optionally substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$;

$R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$ independently selected from hydrogen, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- heteroaryl is optionally substituted with one or more substituents; the substituents are selected from hydrogen, halogen,

-CN, $-NO_2$, oxo, -OH, $-NH_2$, $C_{1-4}$ alkyl, $C_{1-4}$ alkoxy, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl;

q is 0, 1, 2, 3 or 4;

r is 0, 1, 2, 3 or 4;

s is 1, 2, 3 or 4;

t is 1, 2, 3 or 4.

In some embodiments, in the Formula II, ring C is selected from 3- to 10-membered saturated or partially saturated heterocyclyl, $C_{6-10}$ aryl or 5- to 10-membered heteroaryl;

In some embodiments, in the Formula II, the ring C is selected from $C_{3-10}$ carbocyclyl or 3- to 10- membered heterocyclyl.

In some embodiments, in the Formula II, the ring C is selected from cyclopropyl, cyclobutyl, cyclopentyl, cyclohexyl, morpholinyl, piperazinyl, or piperidinyl.

In some embodiments, in the Formula II, the ring C is selected from $C_{6-10}$ aryl or 5- to 10- membered heteroaryl.

In some embodiments, in the Formula II, the ring C is selected from phenyl, pyrrolyl, furyl, thienyl, oxazolyl, imidazolyl, pyrazolyl, thiazolyl, pyridyl, pyrimidinyl, pyridazinyl, pyrazinyl, pyridinone, indolyl, benzoxazolyl, indazolyl, benzimidazolyl, benzotriazolyl, pyrrolopyridyl, quinolinyl, naphthyl, dihydro Isobenzofuranyl, benzobisoxazolyl, tetrahydroquinolinyl, dihydropyrrolopyridyl, dihydronaphthyridinyl, dihydropyridopyrimidinonyl, isoindolinyl, dihydropyrazole pyrimidyl, cyclohexenyl, tetrahydrobenzazepine, dihydrobenzimidazolone, indolinone, isoindolinone, dihydroquinolinone, dihydroisoquinolinone, benzoxazinone, quinolinone, benzobisoxazolyl, dihydrobenzodioxin, pyridone, indazolyl.

In some embodiments, in the Formula II, the ring C is selected from

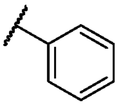,

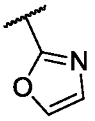, 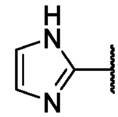, 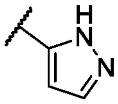, 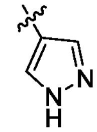, 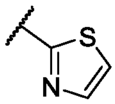, 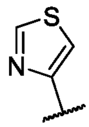, 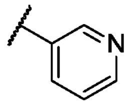,

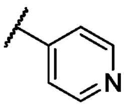 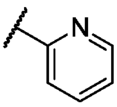, 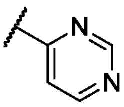, 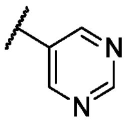, 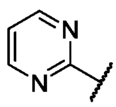, 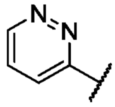, 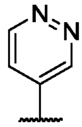, 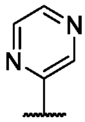,

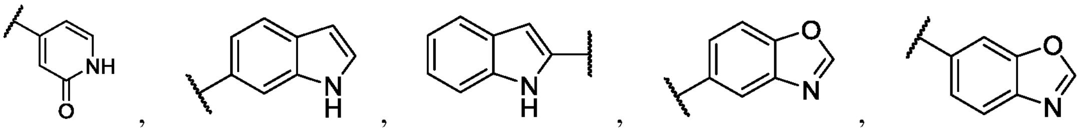
, , , , ,

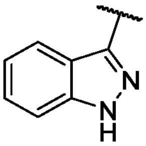, 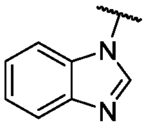, 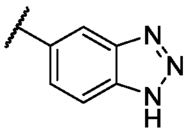, 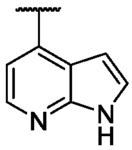, 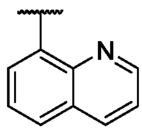, 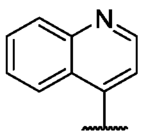,

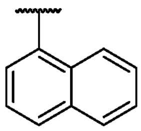, 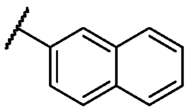, 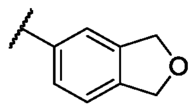, 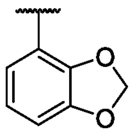, 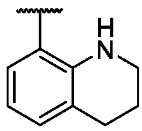, 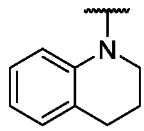,

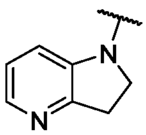, 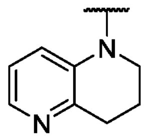, 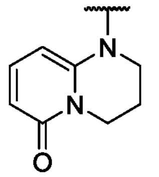, 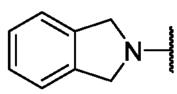, 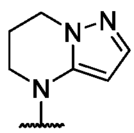, 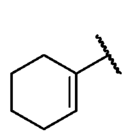,

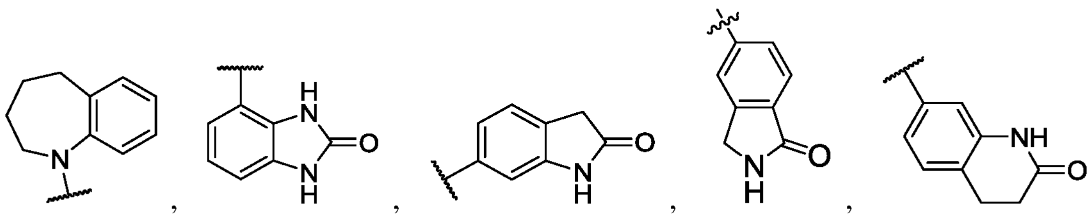
, , , , ,

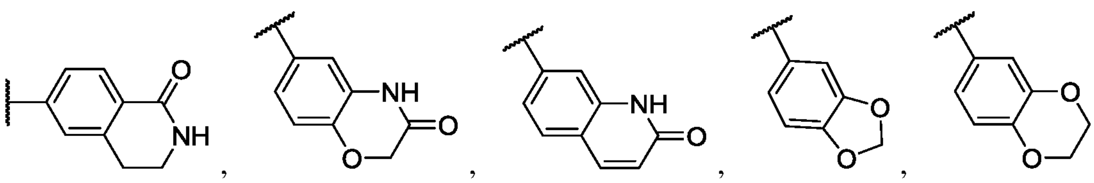
, , , , ,

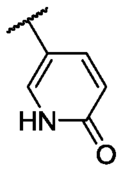, 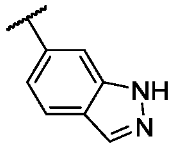 and 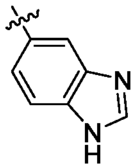 .

In some embodiments, in the Formula II, the ring C is selected from

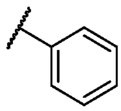 .

In some embodiments, in the Formula II, each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$ $-NR^{28}S(=O)_2R^{29}$, $-OC(=O)R^{30}$, $-S(=O)R^{31}$ 或$-P(=O)R^{32}R^{33}$.

In some embodiments, in the Formula II, each $R^c$ is independently selected from hydrogen, oxo, $-CH_3$, $-OCH_3$, -OH, $-OCF_3$, -CN, -F, -Cl, $-CF_3$, $-NH_2$, $-NHCH_3$, $-NH(C{=}O)CH_3$, $-CH_2OH$, $-C(=O)CH_3$, $-C(=O)NH_2$, $-C(=O)NHCH_3$, $-C(=O)N(CH_3)_2$, -COOH, -COOEt, $-S(=O)_2CH_3$, $-S(=O)_2NH_2$, $-P(=O)(CH_3)_2$, $-CH_2OCH_3$ , $-CH_2C(=O)NH_2$,

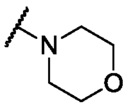, 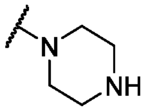, 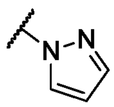, 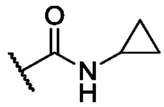, 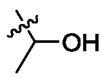, 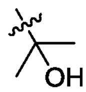,

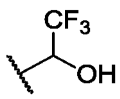, 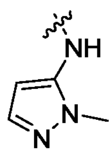 or 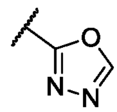 .

In some embodiments, in the Formula II, each $R^c$ is independently selected from hydrogen, $-CH_3$, -F, -Cl, $-NH(C{=}O)CH_3$, $-C(=O)NH_2$ or $-C(=O)NHCH_3$.

In some embodiments, in the Formula II, each $R^c$ is independently selected from hydrogen, $-S(=O)_2NH_2$.

In some embodiments, in the Formula II, $R^1$ is selected from hydrogen, halogen or $C_{1-4}$ alkyl.

In some embodiments, in the Formula II, $R^1$ is selected from hydrogen.

In some embodiments, in the Formula II, each $R^4$ is independently selected from hydrogen, halogen, oxo, $C_{1-8}$ alkyl or $-OR^5$.

In some embodiments, in the Formula II, each $R^4$ is independently selected from hydrogen or $-OCH_3$.

In some embodiments, in the Formula II, X is selected from -O-.

In some embodiments, in the Formula II, X is selected from $-N(R^{1X})-$,

In some embodiments, in the Formula II, $R^{1X}$ is selected from hydrogen or methyl.

In some embodiments, in the Formula II, q is 0 or 1.

In some embodiments, in the Formula II, r is 0, 1, or 2.

In some embodiments, the compound of Formula I, or a pharmaceutically acceptable salt, deuterated compound, cis-trans isomer, tautomer, solvate, chelate, non-covalent complex or precursor, selected from any of the following Formula III-Formula V:

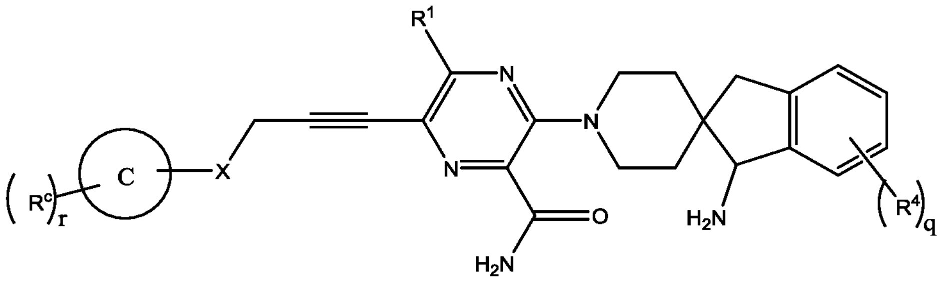

Formula III

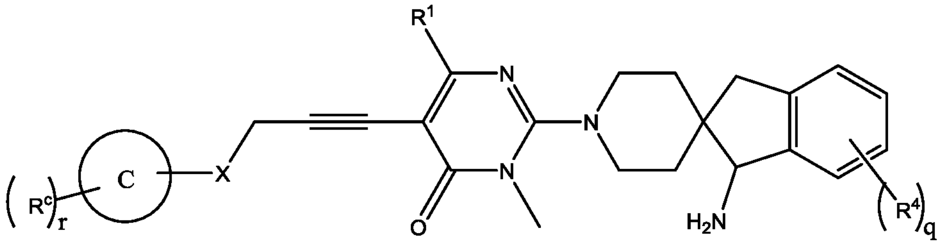

Formula IV

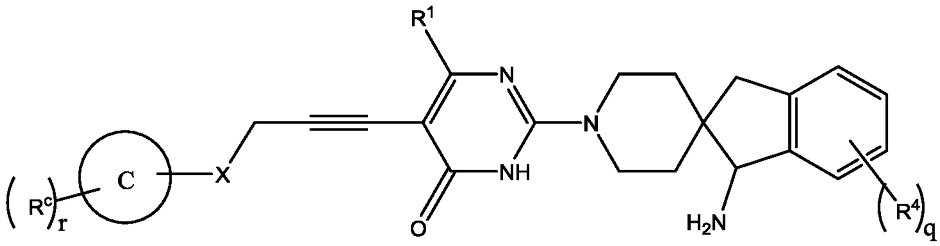

Formula V

In some embodiments, the compound of Formula I is selected from:

(S)-(3-(1-amino-1,3-dihydrospiro[indene-2,4'-piperidin]-1'-yl)-6-(3-phenoxyprop-1-yn-1-yl)pyrazin-2-yl)methanol;
(S)-(3-(1-amino-1,3-dihydrospiro[indene-2,4'-piperidin]-1'-yl)-6-(3-(3-methoxybenzene)oxy)prop-1-yn-1-yl)pyrazin-2-yl)methanol;
(S)-(3-(1-amino-1,3-dihydrospiro[indene-2,4'-piperidin]-1'-yl)-6-(3-(3-aminophenoxy)prop-1-yn-1-yl)pyrazin-2-yl)methanol;
(S)-N-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-(3-(1-amino-1,3-dihydrospiro[indene-2,4'-piperidin]-1'-yl)-6-(3-(3-chloro-2-fluorophenoxy)prop-1-yn-1-yl)pyrazin-2-yl)methanol;
(S)-(3-(1-amino-1,3-dihydrospiro[indene-2,4'-piperidin]-1'-yl)-6-(3-((3-methoxybenzeneyl)thio)prop-1-yn-1-yl)pyrazin-2-yl)methanol;
(S)-(3-(1-amino-1,3-dihydrospiro[indene-2,4'-piperidin]-1'-yl)-6-(3-(phenylamino)prop-1-yn-1-yl)pyrazin-2-yl)methanol;
(S)-N-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-(3-(1-amino-1,3-dihydrospiro[indene-2,4'-piperidin]-1'-yl)-6-(3-(4-methoxybenzene)prop-1-yn-1-yl)pyrazin-2-yl)methanol;
(S)-(3-(1-amino-1,3-dihydrospiro[indene-2,4'-piperidin]-1'-yl)-6-(3-(2-methoxybenzene)prop-1-yn-1-yl)pyrazin-2-yl)methanol;
(S)-(3-(1-amino-1,3-dihydrospiro[indene-2,4'-piperidin]-1'-yl)-6-(3-(3,4-dimethoxyphenoxy)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)benzonitrile;
(S)-(3-(1-amino-1,3-dihydrospiro[indene-2,4'-piperidin]-1'-yl)-6-(3-(3-(trifluoromethyl)phenoxy)prop-1-yn-1-yl)pyrazin-2-yl)methanol;
(S)-(3-(1-amino-1,3-dihydrospiro[indene-2,4'-piperidin]-1'-yl)-6-(3-(4-(trifluoromethyl)phenoxy)prop-1-yn-1-yl)pyrazin-2-yl)methanol;
(S)-(3-(1-amino-1,3-dihydrospiro[indene-2,4'-piperidin]-1'-yl)-6-(3-(3-(trifluoromethyl) phenoxy)prop-1-yn-1-yl)pyrazin-2-yl)methanol;
(S)-1-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)ethane-1-one;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)benzamide;
(S)-(3-(1-amino-1,3-dihydrospiro[indene-2,4'-piperidin]-1'-yl)-6-(3-(pyridin-4-oxyl)prop-1-yn-1-yl)pyrazin-2-yl)methanol;
(S)-(3-(1-amino-1,3-dihydrospiro[indene-2,4'-piperidin]-1'-yl)-6-(3-((2-Aminopyrimidine-5-yl)oxyl)prop-1-yn-1-yl)pyrazin-2-yl)methanol;
(S)-(3-(1-amino-1,3-dihydrospiro[indene-2,4'-piperidin]-1'-yl)-6-(3-(4-molinophenoloxyl)prop-1-yn-1-yl)pyrazin-2-yl)methanol;
(S)-1-(4-(4-((3-(5-(1-amino-1,3-dihydrospiro[indene-2,4'-piperidin]-1'-yl)-6-(hydroxymethyl)pyrazin-2-yl)prop-2-yn-1-yl)oxy)piperazin-1-yl)ethane-1-one;
(S)-7-((3-(5-(1-amino-1,3-dihydrospiro[indene-2,4'-piperidin]-1'-yl)-6-(hydroxymethyl)pyrazin-2-yl)prop-2-yn-1-yl)oxy)-3,4-dihydroquinolin-2(1H)-one;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)-2H-benzo[b][1,4]oxazin-3(4H)-one;
(S)-7-((3-(5-(1-amino-1,3-dihydrospiro[indene-2,4'-piperidin]-1'-yl)-6-(hydroxymethyl)pyrazin-2-yl)prop-2-yn-1-yl)oxy)quinolin-2(1H)-one;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)indol-2-one;
(S)-(3-(1-amino-1,3-dihydrospiro[indene-2,4'-piperidin]-1'-yl)-6-(3-((2-Aminopyridine-4-yl)oxy)prop-1-yn-1-yl)pyrazin-2-yl)methanol;
(S)-3-((3-(5-(1-amino-1,3-dihydrospiro[indene-2,4'-piperidin]-1'-yl)-6-(hydroxymethyl)pyrazin-2-yl)prop-2-yn-1-yl)oxy)benzamide;
(S)-(3-(1-amino-1,3-dihydrospiro[indene-2,4'-piperidin]-1'-yl)-6-(3-(benzo[d][ 1,3]Dioxyol-5-oxy)prop-1-yn-1-yl)pyrazin-2-yl)methanol;
(S)-(3-(1-amino-1,3-dihydrospiro[indene-2,4'-piperidin]-1'-yl)-6-(3-(((2,3-dydrobenzo[b] [1,4]dioxin-6-yl)oxy)prop-1-yn-1-yl)pyrazin-2-yl)methanol;
(S)-5-((3-(5-(1-amino-1,3-dihydrospiro[indene-2,4'-piperidin]-1'-yl)-6-(hydroxymethyl)pyrazin-2-yl)prop-2-yn-1-yl)oxy)-1-methylpyridin-2(1H)-one;
(S)-(6-(3-((1H-Indolazol-6-yl)oxy)prop-1-yn-1-yl)-3-(1-amino-1,3-dihydro spiro[indene-2,4'-piperidin]-1'-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)benzenesulfonamide;
(S)-3-((3-(2-(1-amino-1,3-dihydrospiro[indene-2,4'-piperidin]-1'-yl)-6-oxo-1,6-dihydropyrimidin-5-yl)prop-2-yn-1-yl)oxy)benzamide;
(S)-N-(3-((3-(2-(1-amino-1,3-dihydrospiro[indene-2,4'-piperidin]-1'-yl)-1-methyl)yl-6-oxo-1,6-dihydropyrimidin-5-yl)prop-2-yn-1-yl)oxy)phenyl)acetamide;
(S)-3-((3-(5-(1-amino-1,3-dihydrospiro[indene-2,4'-piperidin]-1'-yl)-6-(hydroxymethyl)pyrazin-2-yl)prop-2-yn-1-yl)oxy)-N-methylbenzamide;
(S)-3-((3-(5-(1-amino-1,3-dihydrospiro[indene-2,4'-piperidin]-1'-yl)-6-(hydroxymethyl)pyrazin-2-yl)prop-2-yn-1-yl)oxy)-N,N-dimethylbenzamide;
(S)-(3-(1-amino-1,3-dihydrospiro[indene-2,4'-piperidin]-1'-yl)-6-(3-(3-(methylthio)acyl)phenoxy)prop-1-yn-1-yl)pyrazin-2-yl)methanol;
(S)-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)dimethylphosphine oxide;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-1-methylpyridin-2(1H)-one;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-N-picolinamide;
(S)-6-(3-(3-acetamidophenoxy)prop-1-yn-1-yl)-3-(1-amino-1,3-dihydrospiro[indene-2,4]'-piperidin]-1'-yl)pyrazine-2-carboxamide;
(S)-3-(1-amino-1,3-dihydrospiro[indene-2,4'-piperidin]-1'-yl)-6-(3-((3-oxoisooctanol)-5-yl)oxy)prop-1-yn-1-yl)pyrazine-2-carboxamide;
(S)-5-(5-(1-amino-1,3-dihydrospiro[indene-2,4'-piperidin]-1'-yl)-6-(hydroxymethyl)pyrazine-2-yl)-1-phenylpent-4-yn-1-one;
(S)-4-(5-(1-amino-1,3-dihydrospiro[indene-2,4'-piperidin]-1'-yl)-6-(hydroxymethyl)pyrazine-2-yl)-N-phenyl-3-alkynamide;
(S)-N-(3-(5-(1-amino-1,3-dihydrospiro[indene-2,4'-piperidin]-1'-yl)-6-(hydroxymethyl)pyrazin-2-yl)prop-2-yn-1-yl)benzamide;
(S)-(3-(1-amino-1,3-dihydrospiro[indene-2,4'-piperidin]-1'-yl)-6-(4-(phenylamino)butyl-1-yn-1-yl)pyrazin-2-yl)methanol;
(S)-(3-(1-amino-1,3-dihydrospiro[indene-2,4'-piperidin]-1'-yl)-6-(3-((3,4-dimethoxyphenyl)amino)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)amino)benzamide;
(S)-(3-(1-amino-1,3-dihydrospiro[indene-2,4'-piperidin]-1'-yl)-6-(3-((2-methoxypyridine-4-yl)oxy)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-2-fluorobenzamide;
(S)-5-((3-(5-(1-amino-1,3-dihydrospiro[indene-2,4'-piperidin]-1'-yl)-6-(hydroxymethyl)pyrazin-2-yl)prop-2-yn-1-yl)oxy)isoindolin-1-one;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)-3,4-dihydroisoquinolin-1(2H)-one;
(S)-3-((3-(5-(1-amino-1,3-dihydrospiro[indene-2,4'-piperidin]-1'-yl)-6-(hydroxymethyl)pyrazin-2-yl)prop-2-yn-1-yl)oxy)benzonitrile;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-2-chlorobenzamide;
(S)-4-((3-(5-(1-amino-1,3-dihydrospiro[indene-2,4'-piperidin]-1'-yl)-6-(hydroxymethyl)pyrazin-2-yl)prop-2-yn-1-yl)(methyl)amino)benzamide;
(S)-5-((3-(5-(1-amino-1,3-dihydrospiro[indene-2,4'-piperidin]-1'-yl)-6-(hydroxymethyl)pyrazin-2-yl)prop-2-yn-1-yl)oxy)pyridineamide;
(S)-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)dimethylphosphine oxide;
(S)-4-((3-(5-(1-amino-6-methoxy-1,3-dihydrospiro[indene-2,4'-piperidin]-1'-yl)-6-(hydroxymethyl)pyrazin-2-yl)prop-2-yn-1-yl)oxy)benzamide;
(S)-2-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-(3-(1-amino-1,3-dihydrospiro[inden-2,4'-piperidin]-1'-yl)-6-(3-(oxazol-2-ylAmino)prop-1-yn-1-yl)pyrazin-2-yl)methanol;
(S)-(3-(1-amino-1,3-dihydrospiro[indene-2,4'-piperidin]-1'-yl)-6-(3-((1-methyl-1H-pyrazol-5-yl)amino)prop-1-yn-1-yl)pyrazin-2-yl)methanol;
(S)-4-((3-(5-(1-amino-1,3-dihydrospiro[inden-2,4'-piperidin]-1'-yl)pyrazin-2-yl)propane-2-yn-1-yl)oxy)-2-chlorobenzamide;
(S)-(3-(1-amino-1,3-dihydrospiro[indene-2,4'-piperidin]-1'-yl)-6-(3-((6-(trifluoromethyl)yl)pyridin-3-yl) amino)prop-1-yn-1-yl)pyrazin-2-yl)methanol;
(S)-7-((3-(5-(1-amino-1,3-dihydrospiro[indene-2,4'-piperidin]-1'-yl)-6-(hydroxymethyl)pyrazin-2-yl)prop-2-yn-1-yl)amino)-3,4-dihydroquinolin-2(1H)-one;
(S)-N-(3-((3-(5-(1-amino-1,3-dihydrospiro[indene-2,4'-piperidin]-1'-yl)-6-(hydroxyl methyl)pyrazin-2-yl)prop-2-yn-1-yl)amino)phenyl)acetamide;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)isoindol-1-one;
(S)-(6-(3-((1H-Indazol-5-yl)oxy)prop-1-yn-1-yl)-3-(1-amino-1,3-dihydrospiro[ indene-2,4'-piperidin]-1'-yl)pyrazin-2-yl)methanol;
(S)-(3-(1-amino-1,3-dihydrospiro[indene-2,4'-piperidin]-1'-yl)-6-(3-((2-(methylAmino)pyrimidin-5-yl)oxy)prop-1-yn-1-yl)pyrazin-2-yl)methanol;
(S)-(3-(1-amino-1,3-dihydrospiro[inden-2,4'-piperidin]-1'-yl)-6-(3-((2-Aminobenzo[d]oxazol-5-yl)oxy)prop-1-yn-1-yl)pyrazin-2-yl)methanol;
(S)-(3-(1-amino-1,3-dihydrospiro[indene-2,4'-piperidin]-1'-yl)-6-(3-((6-(trifluoromethyl)yl)pyridin-3-yl)oxy)prop-1-yn-1-yl)pyrazin-2-yl)methanol;
(S)-2-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-N-methylbenzamide;
(S)-(3-(1-amino-1,3-dihydrospiro[indene-2,4'-piperidin]-1'-yl)-6-(3-((2-methyl-1H-benzo[d]imidazol-5-yl)oxy)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-2-methylbenzamide;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)pyridin-3-ol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-N-cyclopropylbenzamide;
(S)-(3-(1-amino-1,3-dihydrospiro[indene-2,4'-piperidin]-1'-yl)-6-(3-(4-((1-methy1)yl)-1H-pyrazol-5-yl)amino)phenoxy)prop-1-yn-1-yl)pyrazin-2-yl)methanol;
1-(4-((3-(5-((S)-1-amino-1,3-dihydrospiro[indene-2,4'-piperidin]-1'-yl)-6-(hydroxyl)methyl)pyrazin-2-yl)prop-2-yn-1-yl)oxy)phenyl)-2,2,2-trifluoroethane-1-ol;
1-(4-((3-(5-((S)-1-amino-1,3-dihydrospiro[indene-2,4'-piperidin]-1'-yl)-6-(hydroxyl)methyl)pyrazin-2-yl)prop-2-yn-1-yl)oxy)phenyl)ethane-1-ol;
(S)-2-(4-((3-(5-(1-amino-1,3-dihydrospiro[indene-2,4'-piperidin]-1'-yl)-6-(hydroxyl methyl)pyrazin-2-yl)prop-2-yn-1-yl)oxy)phenyl)propan-2-ol;
(S)-3-((3-(5-(1-amino-1,3-dihydrospiro[indene-2,4'-piperidin]-1'-yl)-6-(hydroxymethyl)pyrazin-2-yl)prop-2-yn-1-yl)amino)benzamide;
(S)-2-(4-((3-(5-(1-amino-1,3-dihydrospiro[indene-2,4'-piperidin]-1'-yl)-6-(hydroxymethyl)pyrazin-2-yl)prop-2-yn-1-yl)oxy)-1H-pyrazol-1-yl)acetamide;
(R)-4-((3-(5-(3-amino-3H-spiro[benzofuran-2,4'-piperidin]-1'-yl)-6-(hydroxymethyl)pyrazine-2-yl)prop-2-yn-1-yl)oxy)benzamide;
(S)-4-((3-(5-(5-amino-5,7-dihydrospiro[cyclopenta[b]pyridin-6,4'-piperidin]-1'-yl)-6-(hydroxymethyl)pyrazin-2-yl)prop-2-yn-1-yl)oxy)benzamide;
(S)-4-((3-(5-(4-amino-4,6-dihydrospiro[cyclopenta[d]thiazol-5,4'-piperidin]-1'-yl)-6-(hydroxymethyl)pyrazin-2-yl)prop-2-yn-1-yl)oxy)benzamide;
(S)-4-((3-(5-(4-amino-4,6-dihydrospiro[cyclopenta[d]oxazol-5,4'-piperidin]-1'-yl)-6-(hydroxymethyl)pyrazin-2-yl)prop-2-yn-1-yl)oxy)benzamide;
(S)-4-((3-(5-(4-amino-4,6-dihydro-1H-spiro[cyclopenta[d]imidazol-5,4'-piperidin]-1'-yl)-6-(hydroxymethyl)pyrazin-2-yl)prop-2-yn-1-yl)oxy)benzamide.

The present invention also provides a pharmaceutical composition, which is characterized in that it comprises a therapeutically effective amount of at least one compound represented by Formula I and at least one pharmaceutically acceptable adjuvant.

The present invention further provides a pharmaceutical composition, characterized in that the mass percentage of the therapeutically effective amount of at least one compound represented by Formula I and pharmaceutically acceptable adjuvants is 0.0001:1-10.

The present invention provides the application of the compound represented by the structural Formula I or the pharmaceutical composition in the preparation of medicine.

The present invention further provides the preferred technical solution of the application:
Preferably, the application is for the preparation of a medicament for treating, preventing, delaying or preventing cancer, cancer metastasis, cardiovascular disease, immune disease, fibrosis or eye disease.

Preferably, the application is in the preparation of a medicament for treating diseases mediated by SHP2. Preferably, the disease is cancer.

Preferably, the cancer is selected from Noonan syndrome, leopard spot syndrome, juvenile myelomonocytic leukemia, neuroblastoma, melanoma, head and neck squamous cell carcinoma, acute myeloid leukemia, breast cancer, esophageal tumor , lung cancer, colon cancer, head cancer, stomach cancer, lymphoma, glioblastoma, pancreatic cancer, or a combination thereof.

Preferably, the application is for the preparation of SHP2 inhibitors.

The present invention also provides a method for treating and/or preventing diseases mediated by SHP2, comprising administering to a subject a therapeutically effective amount of at least any compound or pharmaceutical composition represented by structural Formula I.

Preferably, in the above method, the SHP2-mediated disease is cancer.

The present invention also provides a method for treating cancer, comprising administering a therapeutically effective amount of at least any one compound or pharmaceutical composition represented by structural Formula I to a treatment subject.

Preferably, the cancer is selected from Noonan syndrome, leopard spot syndrome, juvenile myelomonocytic leukemia, neuroblastoma, melanoma, head and neck squamous cell carcinoma, acute myeloid leukemia, breast cancer, esophageal tumor , lung cancer, colon cancer, head cancer, stomach cancer, lymphoma, glioblastoma, pancreatic cancer, or a combination thereof.

Preferably, in the above method, the treatment subject is human.

Unless otherwise specified, the terms used in the present invention have the following meanings:

The term "alkyl" includes straight, branched or cyclic saturated alkyl groups. For example, alkyl include, but are not limited to, methyl, ethyl, propyl, isopropyl, cyclopropyl, n-butyl, isobutyl, sec-butyl, tert-butyl, cyclobutyl, n-pentyl, 3-(2-methyl)butyl, 2-pentyl, 2-methylbutyl, neopentyl, cyclopentyl, n-hexyl, 2-hexyl, 2-methylpentyl, cyclohexyl and the like. Similarly, "$C_{1-8}$" in "$C_{1-8}$ alkyl" refers to a linear, branched or cyclic arrangement containing 1, 2, 3, 4, 5, 6, 7 or 8 carbon atoms.

"Alkenyl" and "alkynyl" include straight chain, branched or cyclic alkenyl and alkynyl. Likewise, "$C_{2-8}$ alkenyl" and "$C_{2-8}$ alkynyl" refer to alkenes containing 2, 3, 4, 5, 6, 7 or 8 carbon atoms arranged in straight chain, branched chain or cyclic form radical or alkynyl.

The term "alkoxy" refers to the oxyether forms of the aforementioned linear, branched or cyclic alkyl groups.

The term "aryl" refers to an unsubstituted or substituted monocyclic or polycyclic aromatic group including carbon atoms. Preferably it is a 6- to 10-membered monocyclic or bicyclic aromatic group. Preferred are phenyl and naphthyl. Most preferred is phenyl.

The term "heteroaryl" refers to a monovalent heteroatom group formed by the removal of a hydrogen atom from a carbon atom of a parent heteroaromatic ring system. Heteroaryl groups include: 5- to 7- membered aromatic, monocyclic rings, including at least one heteroatom selected from N, O or S, eg, 1 to 4 heteroatoms, or preferably 1 to 3 heteroatoms, other atoms on the ring are carbon; polyheteroaryl rings include at least one heteroatom selected from N, O or S, for example, 1 to 4 heteroatoms, or preferably 1 to 3 heteroatoms, other atoms on the ring is carbon, and at least one heteroatom is on aromatic ring; it also includes bicyclic or polycyclic rings, but at least one of the bicyclic or polycyclic rings is aromatic. Particularly preferred heteroaryl are $C_{3-10}$ heteroaryl groups including, but not limited to, pyrrolyl, furyl, thienyl, pyridyl, pyranyl, pyrazolyl, pyrimidinyl, pyridazinyl, pyridyl azinyl, imidazolyl, thiazolyl, oxazolyl, isoxazolyl, triazolyl, indolyl, benzofuranyl, benzothiazolyl, benzimidazolyl, benzopyrazolyl, benzoyl Triazolyl, carbazolyl, quinolinyl, isoquinolinyl, purinyl and the like.

However, under no circumstances will heteroaryl and aryl groups intersect or contain each other. Thus, according to the above definition, if at least one all-carbon aromatic ring is fused to a heterocyclyl, a heteroaryl is obtained, not an aryl.

"Carbocyclyl" refers to a cyclyl that is saturated or unsaturated but not aromatic. Depending on their particular level of saturation, the terms "cycloalkyl", "cycloalkenyl" or "cycloalkynyl" are used, respectively. Representative carbocyclyl include, but are not limited to, cyclopropane, cyclobutane, cyclopentane, cyclohexane or cyclohexene and the like.

"Heterocyclyl" means a saturated or unsaturated, but non-aromatic, cyclyl in which one or more carbon atoms (and attached hydrogen atoms) may be replaced by the same or different heterocyclyl respectively atom and the corresponding attached hydrogen atom. Representative heteroatoms substituted for carbon atoms include, but are not limited to, N, P, O, S, and Si. When it is desired to describe a particular degree of saturation, the terms "heterocycloalkyl" or "heterocycloalkenyl", respectively, are used. Representative heterocyclyl include, but are not limited to, epoxy, imidazolidine, morpholine, piperazine, piperidine, pyrazolidine, pyrrolidine, quinuclidine, tetrahydrofuran or tetrahydropyran and the like. Substituent-containing heterocyclyl also include ring systems substituted with at least one oxygen-containing (=O) or oxide (-O-) substituent, such as: piperidine-nitrogen-oxide, morpholinyl-nitrogen-oxide, 1-oxo-1-thiomorpholinyl and 1 -dioxo-1 -thiomorpholinyl.

However, under no circumstances will heterocycloalkyl and carbocyclyl cross or contain each other. Thus, according to the above definition, if at least one percarbocycle is fused to a heterocycloalkyl to form a bi-, poly- or spiro-ring, it will still be defined as a heterocycloalkyl.

Additionally, if a heteroaryl group is fused with a heterocyclyl to form a bi-, poly- or spiro-ring, it will be defined as heterocyclyl rather than heteroaryl.

"Halogen" means fluorine (F), chlorine (Cl), bromine (Br) or iodine (I). Preferred halogens are fluorine, chlorine and bromine.

"Halo" means a fluoro, chloro, bromo or iodo. Preferred halo refer to fluoro and chloro.

"Substituted" means that one or more hydrogen atoms in a group are replaced by the same or different substituents, respectively. Representative substituents include, but are not limited to, halogen, amino, hydroxyl, oxo, carbonyl, cyano, $-C(O)NH_2$, alkyl, alkoxy, aryl, cycloalkyl, heterocyclyl, heteroaryl. In some embodiments, substituents include, but are not limited to, halogen, amino, hydroxy, cyano, methyl, $-CH_2OH$, $-C(O)NH_2$, $-OCH_3$, trifluoromethyl.

Whenever the term "alkyl" or "aryl" or its prefix root appears in the name of a substituent (eg, aralkyl, or

dialkylAmino), the term "alkyl" and "aryl" should be "Definition defines a limited interpretation of the substituents. A specified number of carbon atoms (eg, $C_{1-6}$) will independently represent the number of carbon atoms in an alkyl moiety or in the alkyl moiety of a larger substituent (where alkyl is the prefix root).

"Compounds" as used herein include compounds of Formula I, and all pharmaceutically acceptable forms thereof. These pharmaceutically acceptable forms include salts, solvates, non-covalent complexes, chelates, stereoisomers (including diastereomers, enantiomers and racemates), cis-trans Isomers, isotopically labeled compounds, tautomers, prodrugs, or any mixture of all of the above.

The "cis-trans isomer" is a stereoisomerism that exists in certain double bond compounds or cyclic compounds. The free rotation of these molecules is hindered due to the presence of double bonds or rings, resulting in two isomers that are not identical in physical or chemical properties, called cis (cis) and trans (trans) isomers, respectively.

Unless otherwise stated, the compounds of the present invention are intended to include all such possible cis-trans isomers, including cis-configured compounds\trans-configured compounds and mixtures of cis-trans isomers in any ratio. Cis-trans isomers can be separated using conventional techniques (eg, on a Prep-HPLC column).

The "pharmaceutically acceptable" refers to those known to be used in animal, especially in human.

The term "composition" in the present invention includes a product containing the specified components in the specified amounts, as well as any product obtained directly or indirectly from the specified amounts of the specified components. Accordingly, pharmaceutical compositions comprising the compounds of the present invention as active ingredients and methods of preparing the compounds are the subject of the present invention.

A "therapeutically effective amount" refers to a compound that when administered to a subject is sufficient to treat and prevent and/or inhibit at least one clinical symptom of a disease, condition, symptom, indication and/or disorder. The dose that produces an effect in the treatment of symptoms, indications, or discomfort. The specific "therapeutically effective dose" may vary depending on the compound, the route of administration, the age of the patient, the weight of the patient, the type, symptoms and severity of the disease or disorder being treated, and the like. Wherever possible, an appropriate dose will be apparent to those skilled in the art or may be determined by routine experimentation.

The compounds provided herein may exist in the form of "pharmaceutically acceptable salts". In terms of pharmaceutical applications, the salts of the compounds provided by the present invention refer to non-toxic pharmaceutically acceptable salts. Pharmaceutically acceptable salt forms include pharmaceutically acceptable acid/anion or base/cation salts. Pharmaceutically acceptable acid/anion salts generally exist in the protonated form of the basic nitrogen with an inorganic or organic acid. Typical organic or inorganic acids include hydrochloric, hydrobromic, hydroiodic, perchloric, sulfuric, nitric, phosphoric, acetic, propionic, glycolic, lactic, succinic, maleic, fumaric, malic , tartaric acid, citric acid, $\alpha$-ketoglutaric acid, hippuric acid, benzoic acid, mandelic acid, methanesulfonic acid, isethionic acid, benzenesulfonic acid, oxalic acid, pamoic acid, 2-naphthalenesulfonic acid, p-toluenesulfonic acid acid, cyclohexylamine sulfonic acid, salicylic acid, saccharinic acid or trifluoroacetic acid. Pharmaceutically acceptable base/cation salts including, but not limited to, aluminum, calcium, chloroprocaine, choline, diethanolamine, ethylenediamine, lithium, magnesium, potassium, sodium salt and zinc salts.

Prodrugs of the compounds of the present invention are included within the scope of the present invention. Typically, the prodrugs are functional derivatives that are readily converted in vivo to the desired compound. Accordingly, the term "administration" in reference to the methods of treatment provided by the present invention includes the administration of a compound disclosed herein, or, although not expressly disclosed, but capable of being converted in vivo to a compound disclosed herein after administration to a subject to treat the various described disease. Conventional procedures for the selection and preparation of suitable prodrug derivatives are described in, for example, Design of Prodrugs (Design of Prodrugs, ed. H. Bundgaard, Elsevier, 1985).

Obviously, the definition of any substituent or position-specific variable in one molecule is independent of the definition of any substituent or position-specific variable in other molecules. It is easy to understand that the compounds in the present invention can select suitable substituents or substitution forms according to the prior art in this subject, so as to provide chemical stability and be easily prepared and synthesized by the methods described in the prior art in this subject or in the present invention.

When the compounds of Formula I and pharmaceutically acceptable salts thereof are in the form of solvates or polymorphs, the present invention includes any possible solvates and polymorphs. The type of solvent that forms the solvate is not particularly limited as long as the solvent is pharmacologically acceptable. For example, water, ethanol, propanol, acetone and similar solvents can be used.

The term "pharmaceutically acceptable salts" refers to salts prepared from pharmaceutically acceptable non-toxic bases or acids. When the compounds provided herein are acids, their corresponding salts can be prepared from pharmaceutically acceptable non-toxic base, including inorganic and organic base. Salts derived from inorganic base include aluminum, ammonium, calcium, copper (ic and ous), iron, ferrous, lithium, magnesium, manganese (ic and ous), potassium, sodium, zinc and the like. In particular, ammonium, calcium, magnesium, potassium and sodium salts are preferred. Nontoxic organic bases that can be derivatized into pharmaceutically acceptable salts include primary, secondary, and tertiary amines, as well as cyclic amines and substituted amines, such as naturally occurring and synthetic

substituted amines. Other pharmaceutically acceptable nontoxic organic bases capable of forming salts, including ion exchange resins and arginine, betaine, caffeine, choline, N',N'-dibenzylethylenediamine, diethylamine, 2-DiethylAminoethanol, 2-DimethylAminoethanol, Ethanolamine, Ethylenediamine, N-Ethylmorpholine, N-Ethylpiperidine, Reduced Glucosamine, Glucosamine, Histidine, Haramine, Isopropylamine , lysine, methylglucamine, morpholine, piperazine, piperidine, polyamine resin, procaine, purine, theobromine, triethylamine, trimethylamine, tripropylamine, tromethamine, etc.

When the compounds provided by the present invention are base, their corresponding salts can be prepared from pharmaceutically acceptable non-toxic acids, including inorganic and organic acids. Such acids include, for example, acetic acid, benzenesulfonic acid, benzoic acid, camphorsulfonic acid, citric acid, ethanesulfonic acid, isethionic acid, formic acid, fumaric acid, gluconic acid, glutamic acid, hydrobromic acid, hydrochloric acid, lactic acid, maleic acid, malic acid, mandelic acid, alpha-ketoglutaric acid, hippuric acid, methanesulfonic acid, mucic acid, nitric acid, pamoic acid, pantothenic acid, phosphoric acid, succinic acid, sulfuric acid, tartaric acid, p-toluenesulfonic acid acid etc. Preferably, malic acid, citric acid, hydrobromic acid, hydrochloric acid, methanesulfonic acid, maleic acid, phosphoric acid, sulfuric acid and tartaric acid. More preferably, phosphoric acid, hydrochloric acid and malic acid. Since the compound of Formula I will be used as a pharmaceutical, it is preferred to use a substantially pure form, for example, at least 60% pure, more suitably at least 75% pure, particularly suitably at least 98% pure (% is by weight).

The pharmaceutical composition provided by the present invention includes the compound represented by Formula I (or a pharmaceutically acceptable salt thereof) as an active component, a pharmaceutically acceptable excipient and other optional therapeutic components or adjuvants. Although the most suitable mode of administration of the active ingredient in any given situation will depend on the particular subject being administered, the nature of the subject and the severity of the condition, the pharmaceutical compositions of the present invention include oral, rectal, topical and pharmaceutical compositions for parenteral (including subcutaneous, intramuscular, intravenous) administration. The pharmaceutical compositions of the present invention may conveniently be presented in unit dosage form and prepared by any of the methods of preparation well known in the art of pharmacy.

In fact, according to the conventional drug mixing technology, the compound represented by the Formula I of the present invention, or the prodrug, or the metabolite, or the pharmaceutically acceptable salt, can be combined with the drug as the active component, and mixed with the drug carrier to form a drug combination thing. The pharmaceutical carrier can take a wide variety of forms, depending on the intended mode of administration, eg, oral or injection (including intravenous). Accordingly, the pharmaceutical compositions of the present invention may take the form of discrete units suitable for oral administration, such as capsules, cachets or tablets containing a predetermined dose of the active ingredient. Further, the pharmaceutical compositions of the present invention may take the form of powders, granules, solutions, aqueous suspensions, non-aqueous liquids, oil-in-water emulsions, or water-in-oil emulsions. In addition, in addition to the common dosage forms mentioned above, the compound of Formula I or a pharmaceutically acceptable salt thereof can also be administered by means of controlled release and/or delivery device. The pharmaceutical composition of the present invention can be prepared by any pharmaceutical method. In general, such methods include the step of bringing into association the active ingredient with the carrier which constitutes one or more essential ingredients. In general, the pharmaceutical compositions are prepared by homogeneous intimate admixture of the active ingredient with liquid carriers or finely divided solid carriers, or a mixture of both. In addition, the product can be easily prepared to the desired appearance.

Therefore, the pharmaceutical composition of the present invention comprises a pharmaceutically acceptable carrier and a compound represented by Formula I, or a pharmaceutically acceptable salt thereof. The compound of Formula I, or a pharmaceutically acceptable salt thereof, and one or more other compounds having therapeutic activity in combination are also included in the pharmaceutical composition of the present invention.

The pharmaceutical carrier employed in the present invention can be, for example, solid carrier, liquid carrier or gaseous carrier. Examples of solid carriers include, lactose, terra alba, sucrose, talc, gelatin, agar, pectin, acacia, magnesium stearate, stearic acid, mannitol, sorbitol, microcrystalline cellulose, inorganic salts, starch, pregelatinized starch, powdered sugar, dextrin, etc. Examples of liquid carriers include syrup, peanut oil, olive oil and water. Examples of gaseous carriers include carbon dioxide and nitrogen. In the preparation of pharmaceutical oral Formulations, any convenient pharmaceutical medium may be employed. For example, water, glycols, oils, alcohols, flavor enhancers, preservatives, colorants, etc. can be used in oral liquid preparations such as suspensions, elixirs and solutions; and carriers such as starches, sugars, Microcrystalline cellulose, diluents, granulating agents, lubricants, binders, disintegrating agents, etc. can be used for oral solid preparations such as powders, capsules and tablets. For ease of administration, tablets and capsules are preferred for oral Formulations. Alternatively, the tablet coating can use standard aqueous or non-aqueous Formulation techniques.

Tablets containing the compounds or pharmaceutical compositions of the present invention may be prepared by mixing, compressing or molding, optionally with one or more accessory ingredients or adjuvants. Compressed tablets may be prepared by compressing the active ingredient in a free-flowing form such as powder or granules with a lubricant, inert diluent, surface-active or dispersing agent, by compression in a suitable machine. Molded tablets may be made by

wetting the powdered compound or pharmaceutical composition with an inert liquid diluent and molding in a suitable machine. Preferably, each tablet contains about 0.01 mg to 5 g of active ingredient and each cachet or capsule contains about 0.1 mg to 0.5 g of active ingredient. For example, a dosage form intended for oral administration to humans contains from about 0.1 mg to about 0.5 g of the active ingredient in admixture with suitable and conveniently metered adjunct materials ranging from about 5% to 99.99% of the total pharmaceutical composition . Unit dosage forms generally contain from about 0.1 mg to about 0.5 g of the active ingredient, typically 0.1 mg, 0.2 mg, 0.5 mg, 1 mg, 2 mg, 2.5 mg, 5 mg, 10 mg, 25 mg, 50 mg, 100 mg mg, 200 mg, 300 mg, 400 mg or 500 mg.

The pharmaceutical composition suitable for parenteral administration provided by the present invention can be prepared as an aqueous solution or suspension by adding the active ingredient to water. Suitable surfactants such as sodium lauryl sulfate, polysorbate-80 (Tween-80), polyoxyethylene hydrogenated castor oil, poloxamers may be included. Dispersions can also be prepared in glycerol, liquid polyethylene glycols, and mixtures thereof in oils. Further, preservatives may also be included in the pharmaceutical compositions of the present invention to prevent the growth of harmful microorganisms.

The present invention provides pharmaceutical compositions suitable for injectable use, including sterile aqueous solutions or dispersions. Further, the above-mentioned pharmaceutical compositions can be prepared in the form of sterile powders that can be used for the extemporaneous preparation of sterile injectable solutions. In any event, the final injectable form must be sterile and, for ease of injection, must be readily flowable. Furthermore, the pharmaceutical compositions must be stable during manufacture and storage. Therefore, preservation against contamination by microorganisms such as bacteria and fungi is preferred. The carrier can be a solvent or dispersion medium, for example, water, ethanol, polyol (eg, glycerol, propylene glycol, liquid polyethylene glycol), vegetable oils, and suitable mixtures thereof.

The pharmaceutical compositions provided by the present invention may be in a form suitable for topical administration, for example, aerosol, cream, ointment, lotion, dusting powder, or other similar dosage forms. Further, the pharmaceutical composition provided by the present invention can be in a form suitable for use in a transdermal drug delivery device. These formulations can be prepared by conventional processing methods using the compound represented by Formula I of the present invention, or a pharmaceutically acceptable salt thereof. As an example, a cream or ointment is prepared by adding a hydrophilic material and water to the above compound (the total amount of both being about 5 wt % to 50 wt % of the compound) to produce a cream or ointment having the desired consistency.

The pharmaceutical composition provided by the present invention can be prepared in form suitable for rectal administration with solid as carrier. Suppositories of admixture to form unit doses are the most preferred dosage forms. Suitable excipients include cocoa butter and other materials commonly used in the art. Suppositories can be conveniently prepared by first mixing the pharmaceutical composition with softened or melted excipients, then cooling and moulding.

In addition to the above-mentioned carrier components, the above-mentioned pharmaceutical preparations may also include, as appropriate, one or more additional adjuvant components, such as diluents, buffers, flavoring agents, binders, surfactants, enhancers Thickeners, lubricants, preservatives (including antioxidants), etc. Further, other adjuvants may also include osmotic enhancers that adjust the isotonic pressure of the drug and blood. The pharmaceutical composition containing the compound represented by Formula I, or a pharmaceutically acceptable salt thereof, can also be prepared in the form of a powder or a concentrated solution.

## Specific Embodiments

In order to make the above content clearer and clearer, the present invention will further illustrate the technical solutions of the present invention with the following examples. The following examples are only used to illustrate the specific embodiments of the present invention, so that those skilled in the art can understand the present invention, but are not intended to limit the protection scope of the present invention. In the specific embodiments of the present invention, the technical means or methods that are not specifically described are conventional technical means or methods in the field.

All parts and percentages herein are by weight and all temperatures are in degrees Celsius unless otherwise indicated.

The following abbreviations are used in the examples:

DCM: dichloromethane;
DIEA: N,N-diisopropylethylamine;
DIBAL-H: diisobutylaluminum hydride;
DME: ethylene glycol dimethyl ether
DMF: N,N-dimethylformamide;
DMSO: dimethyl sulfoxide;
EtOAc: ethyl acetate;

h, hr or hrs: hours;
LC-MS or LC-MS: liquid chromatography-mass spectrometry;
MeCN: acetonitrile;
MeOH: methanol;
min or mins: minutes;
$NEt_3$: triethylamine;
Prep-HPLC: Preparative high performance liquid chromatography;
$PdCl_2(PPh_3)_2$: bistriphenylphosphonium palladium dichloride;
$Pd(PPh_3)_4$: tetrakis (triphenylphosphine) palladium;
PE: petroleum ether;
rt, r.t. or RT: room temperature;
TFA: trifluoroacetic acid;
THF: tetrahydrofuran;
$Ti(OEt)_4$: tetraethyl titanate;
TLC: Thin Layer Chromatography.

Preparation of intermediate compound M1:

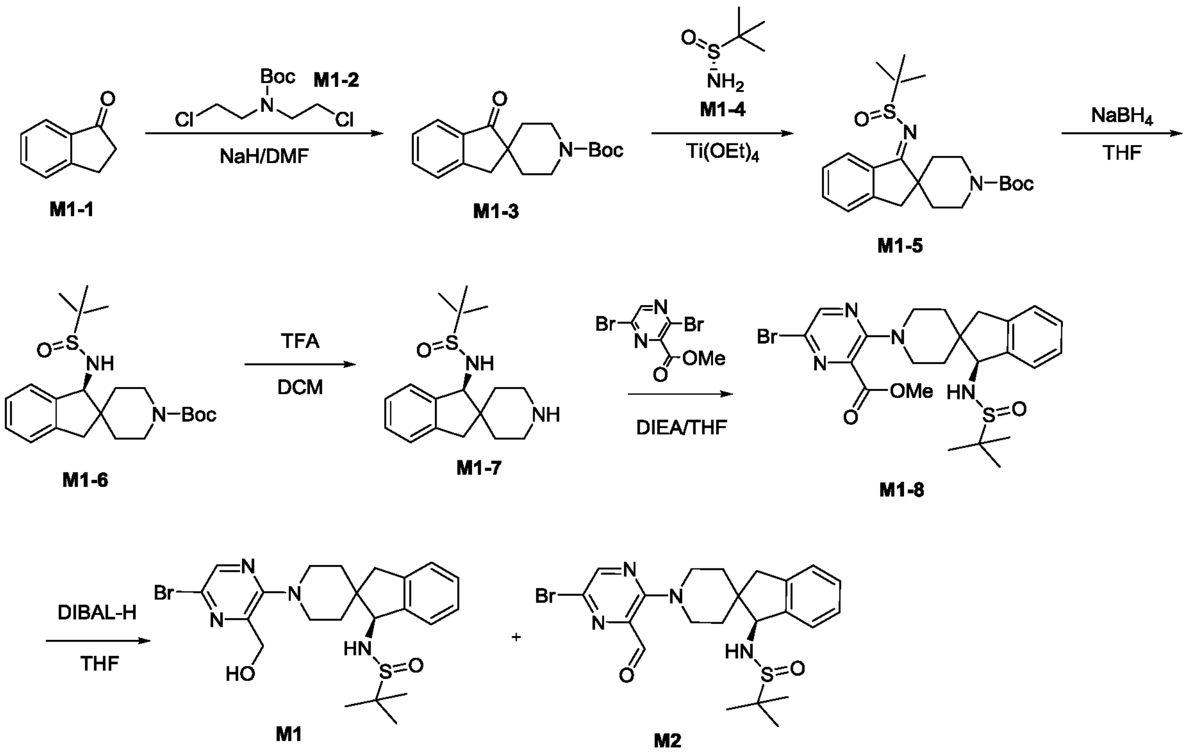

M1-1, M1-2, M1-3, M1-4, M1-5, M1-6, M1-7, M1-8, M1 + M2

Step 1: Preparation of Compound M1-3

Under nitrogen protection, 25.00 g of compound M1-1 was dissolved in 200 mL of DMF, cooled to 0 °C, 22.70 g of NaH was added in batches, kept at 0 °C for 1 hr, and then 54.96 g of compound M6-2 was slowly added dropwise to the reaction solution, after dripping, react at 0 °C for 1 hr, and heat up to 60 °C and continue to react for 1 hr. The reaction solution was cooled to 0 °C, quenched with 500 mL of ice water, extracted with EtOAc (500 mL×3), the organic phases were combined, washed with saturated brine, dried over anhydrous sodium sulfate, concentrated under reduced pressure, and the residue was filtered through a column Chromatographic purification yielded 29.00 g of brown oil M1-3.

Step 2: Preparation of Compounds M1-5

29.00g of compound M1-3 was dissolved in 50 mL of $Ti(OEt)_4$, 34.99g of compound M1-4 was added, and then heated to 90 °C for 12 hrs. TCL detected the completion of the reaction, poured the reaction solution into 500 mL of ice

water, added 300 mL of EtOAc, stirred for 1 hr, extracted with EtOAc (300 mL×3), combined the organic phases, and washed the organic phase with saturated brine (100 mL×4), dried over anhydrous sodium sulfate, and concentrated under reduced pressure to obtain 39.00 g of crude compound M1-5 as a brown oil.

Step 3: Preparation of Compounds M1-6

Under nitrogen protection, 48.00g of compound M1-5 was dissolved in 500 mL of anhydrous THF, cooled to -20 °C, 6.73g of $NaHB_4$ was slowly added, and then the temperature was naturally raised to RT and stirred for 2 hrs. After the reaction was completed, the reaction solution was cooled to 0 °C, quenched with 300 mL of water, extracted with EtOAc (300 mL×3), the organic phases were combined, the organic phases were washed with saturated brine, dried over anhydrous sodium sulfate, and concentrated under reduced pressure. Column chromatography gave 25.40 g of compound M1-6 as brown oil.

Step 4: Preparation of Compounds M1-7

10.00 g of compound M1-6 was dissolved in 100 mL of DCM solution, 28.04 g of TFA solution was added dropwise, and then reacted at RT for 1 hr. The reaction solution was cooled to 0 °C, quenched with 100 mL of saturated $NaHCO_3$ aqueous solution, extracted with EtOAc:THF=3:1 (100 mL×3), the organic phases were combined, washed with saturated brine, dried over anhydrous sodium sulfate, and reduced pressure. Concentrated to obtain 7.64 g of brown solid, the crude product of compound M1-7, which was directly used in the next reaction.

1H NMR (500 MHz, DMSO-d6) δ 7.26-7.21 (m, 4H), 5.80 (d, J = 10.5 Hz, 1H), 4.43 (d, J = 10.5 Hz, 1H), 3.17-3.15 (m, 2H), 3.08 (d, J = 15.5 Hz, 1H), 2.98-2.88 (m, 2H), 2.69 (d, J = 15.5 Hz, 1H), 2.04-1.99 (m, 1H), 1.80-1.75 (m , 1H), 1.62-1.59 (m, 1H), 1.35(m, 1H), 1.22(s, 9H).

Step 5: Preparation of Compounds M1-8

1.29 g of methyl 3,6-dibromopyrazine-2-carboxylate and 1.60 g of compound M1-7 were dissolved in 20 mL of tetrahydrofuran, 1.12 g of DIEA was added dropwise, and the reaction was stirred at 60 °C for 12 hours. TLC detected that the reaction was complete, concentrated under reduced pressure, added 50 mL of water, and extracted with EtOAc (50 mL×3).

Step 6: Preparation of Compounds M1 and M2

Under nitrogen protection, 1.00 g of compound M1-8 was dissolved in 30 mL of anhydrous dichloromethane, the temperature was lowered to -78 °C, and a solution of DIBAL-H (1 M, 9.6 mL) in n-hexane was added dropwise. The reaction was stirred at -78 °C for 1 hour. The temperature was slowly raised to -40 °C, and the reaction was continued for 2 hours. TLC detected that the reaction of the raw materials was complete. At 0 °C, 0.4 mL of water was slowly added dropwise, then an aqueous sodium hydroxide solution (15%, 0.4 mL) was added dropwise, and 1 mL of water was added. Warm to room temperature and stir for 15 minutes. It was dried by adding sodium sulfate, stirred for ten minutes and filtered. The filtrate was concentrated and purified by column chromatography to obtain 0.40 g of compound M1 and 0.11 g of compound M2.

The intermediates listed in Table 1 were prepared in analogy to the aforementioned intermediates M1-7 via different reaction starting materials and appropriate reagents.

Table 1

| Intermediate Number | Chemical Structure | Intermediate Number | Chemical Structure |
|---|---|---|---|
| M12 | 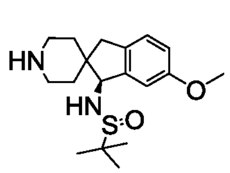 | M6 | 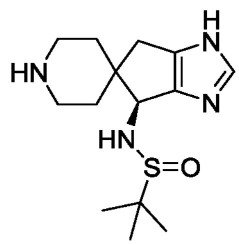 |

(continued)

| Intermediate Number | Chemical Structure | Intermediate Number | Chemical Structure |
|---|---|---|---|
| M3 | 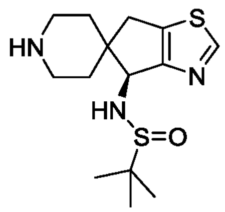 | M7 | 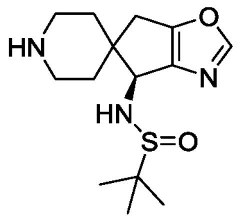 |
| M4 | 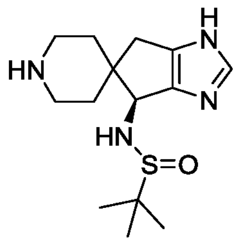 | | |

Preparation of intermediate compound M14:

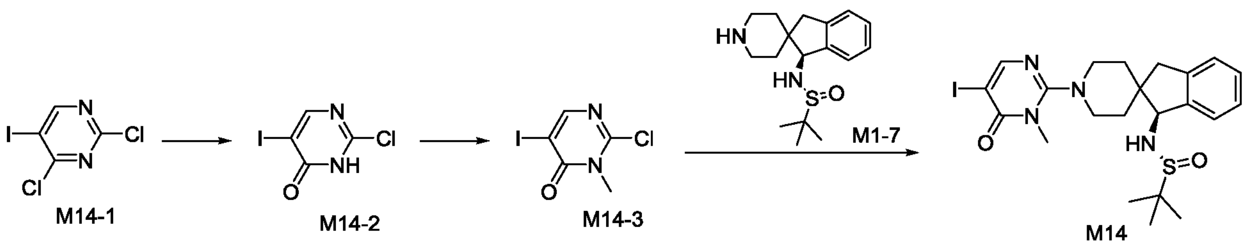


Step 1: Preparation of Compound M14-2

2.00 g of compound M14-1 was dissolved in 40 mL of THF, NaOH (30 mL, 1N) was added, and then RT was reacted for 2 hrs. TLC detected that the reaction was complete, the reaction solution was poured into 100 mL of water, adjusted to pH 4-5 with 6N HCl, extracted with EtOAc (100 mL×4), the organic phases were combined, washed with 50 mL of saturated NaCl, anhydrous sodium sulfate. It was dried and concentrated under reduced pressure, and the residue was slurried with n-hexane to obtain 1.67 g of pale yellow solid M14-2.

Step 2: Preparation of Compound M14-3

1.28g of compound M14-2 was dissolved in 13 mL of DMF, the temperature was lowered to 0 °C, 3.26g of $Cs_2CO_3$ was added, and then 2.13g of $CH_3I$ was added dropwise. After dropping, the temperature was raised to 10 °C and reacted for 1hr. TLC detected that the reaction was complete, the reaction solution was cooled to 0 °C, 20 mL of EtOAc and 20 mL of water were added, the organic phase was separated, the aqueous phase was continuously extracted with 20 mL of EtOAc, the organic phases were combined, washed with saturated NaCl (5×4 mL), and anhydrous. It was dried over sodium sulfate, concentrated under reduced pressure, and the residue was purified by column chromatography to obtain 635 mg of compound M14-3.

Step 3: Preparation of Compound M14

359 mg of compound M14-3 and 448 mg of compound M1-7 were dissolved in 6 mL of DMAc, 860 mg of DIPEA was added, nitrogen was replaced three times, and the reaction was microwaved at 120 °C for 1 hr. TLC detected that the reaction was complete, the reaction solution was cooled to room temperature, 20 mL of water was added, and EtOAc (20 mL×3) was added for extraction. The organic phases were combined, washed with saturated NaCl (5 mL×4), dried

over anhydrous sodium sulfate, and concentrated under reduced pressure, the residue was purified by column chromatography to obtain 290 mg of compound M14.

Preparation of intermediate compound M15:

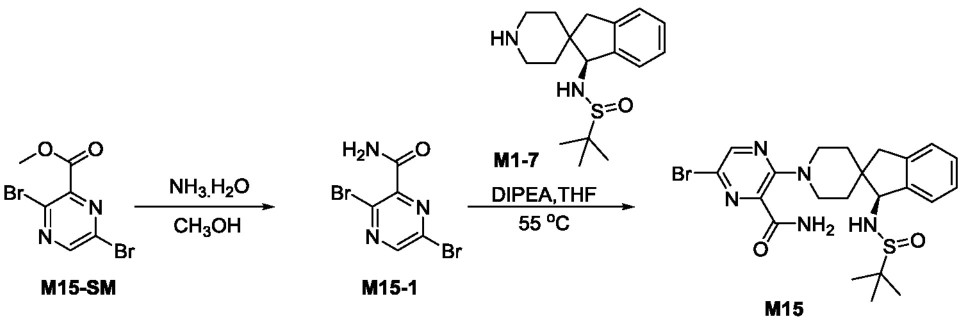


Step 1: Preparation of Compound M15-1

5.0 g of compound M15-SM was dissolved in 50 mL of methanol, ammonia water (60 mL, 25%) was added, and then RT was reacted for 2.5 hours. TLC detected that the reaction was complete, concentrated under reduced pressure (spin off most of the methanol), then added MeOH (10 mL), stirred for 5 minutes, filtered, washed the filter cake with MeOH (3 mL), and dried the filter cake to obtain 4.75g of solid, the compound M15-1.

Step 2: Preparation of Compound M15

3.5 g of compound M15-1, 3.82 g of compound M1-7, 5.04 g of DIPEA were dissolved in 40 mL of THF, and the reaction was stirred at 55 °C for 12 hrs. TLC detected that the reaction was complete, the reaction solution was filtered, the filter cake was washed with DCM (40 mL), the filtrate was concentrated under reduced pressure, and the residue was purified by column chromatography to obtain 5.66 g of compound M15 as a white solid.

Preparation of intermediate compound M16

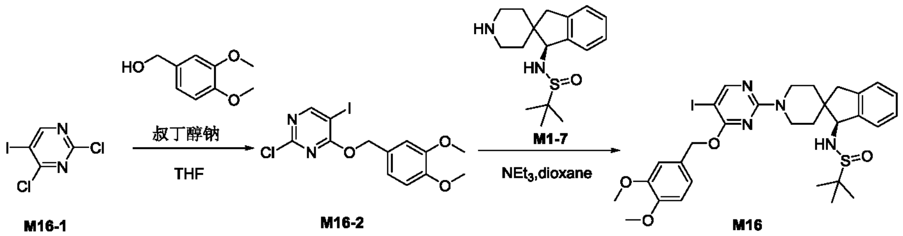


Step 1: Preparation of Compound M16-2

Dissolve 673 mg of 3,4-dimethoxybenzyl alcohol in 30 mL of anhydrous THF, add 385 mg of sodium tert-butoxide, cool the reaction solution to -10 °C, add 1.0g of compound M16-1, and heat the reaction at -10 °C for 30 minute. At low temperature, water (50 mL) was added to quench the reaction, extracted with EA (50 mL), washed with saturated NaCl (10 mL*2), the organic phase was dried over anhydrous sodium sulfate, and purified by column chromatography to obtain 695 mg of compound M16-2 .

Step 2: Preparation of Compound M16

200 mg of compound M16-2 and 196 mg of compound M1-7 were dissolved in 5 mL of dioxane, 149 mg of triethylamine was added, and the reaction was heated at 75 °C for 2 hours. TLC detected that the reaction was complete,

the reaction solution was spun dry, and purified by column chromatography to obtain 186 mg of compound M16.

Preparation of intermediate compound M30:

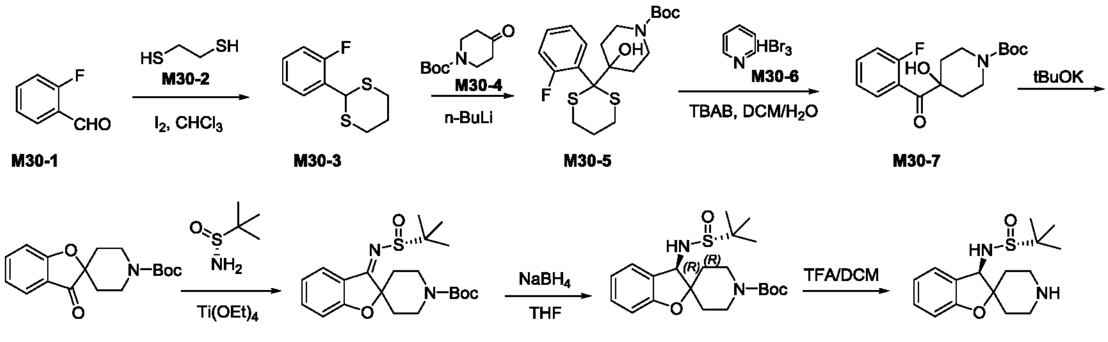


M30-1 M30-3 M30-5 M30-7 M30-8 M30-9 M30-10 M30

Step 1: Preparation of Compound M30-3

10.00 g of compound M30-1 and 7.59 g of compound M30-2 were dissolved in 170 mL of chloroform, 350 mg of iodine was added, and the reaction was stirred at room temperature for 24 hours. TLC detected that the reaction was complete, the compound was poured into aqueous sodium thiosulfate solution (100 mL, 0.4 mol/L), then aqueous sodium hydroxide solution (70 mL, 40%) was added, and then extracted with chloroform (150 mL×2). The organic layers were combined, dried with sodium sulfate, and desolvated, and recrystallized from dichloromethane/n-hexane to obtain 8.77 g of compound M30-3.

Step 2: Preparation of Compound M30-5

8.77 g of compound M30-3 was dissolved in 300 mL of anhydrous tetrahydrofuran, cooled to -30 °C, n-butyllithium (2.5 M, 16 mL) in n-hexane was added dropwise, and the mixture was stirred for 1 hour. Subsequently, 8.15 g of compound M30-4 was dissolved in 30 mL of tetrahydrofuran and slowly added dropwise to the reaction solution. After the dropwise addition, the temperature was raised to -10 °C and the reaction was continued for 2.5 hours. The reaction solution was quenched with 200 mL of saturated brine, extracted with EtOAc (300 mL×2), the organic phases were combined, dried over anhydrous sodium sulfate, concentrated under reduced pressure, and purified by column chromatography to obtain 11.06 g of compound M30-5.

Step 3: Preparation of Compound M30-7

11.06 g of compound M30-5 was dissolved in a mixed solution of water (40 mL) and dichloromethane (200 mL), followed by 3.17 g of pyridine, 12.83 g of compound M30-6 and 0.86 g of tetrabutylammonium bromide in this order. Stir at room temperature for 24 hours. The reaction solution was quenched with 100 mL of saturated brine, extracted with DCM (100 mL×3), the organic phases were combined, dried over anhydrous sodium sulfate, concentrated under reduced pressure, and purified by column chromatography to obtain 5.46 g of compound M30-7.

Step 4: Preparation of Compound M30-8

5.46 g of compound M30-7 and 2.84 g of potassium tert-butoxide were dissolved in 85 mL of anhydrous tetrahydrofuran, and after 3 times of nitrogen replacement, the reaction was microwaved at 70 °C for 5 minutes. The solvent was removed under reduced pressure, 100 mL of saturated brine was added, and extracted with EtOAc (100 mL×2). The organic phases were combined, dried over anhydrous sodium sulfate, concentrated under reduced pressure, and purified by column chromatography to obtain 2.91 g of compound M30-8.

Step 5: Preparation of Compound M30-9

Dissolve 1.30 g of compound M30-8 and 1.56 g of (R)-(+)-tert-butylsulfinamide in a mixed solution of tetraethyl titanate (10 mL) and anhydrous tetrahydrofuran (2 mL), and replace with nitrogen. After 3 times, react at 90 °C for 8

hours. Heating was stopped, the reaction solution was poured into 200 mL of ice water, 150 mL of ethyl acetate was added, and the mixture was stirred for 1 hour. The filter residue was removed by filtration. The filtrate was extracted with ethyl acetate (100 mL×2), the organic phases were combined, dried over anhydrous sodium sulfate, and concentrated under reduced pressure to obtain 1.90 g of compound M30-9.

Step 6: Preparation of Compound M30-10

1.30 g of compound M30-9 was dissolved in 25 mL of anhydrous tetrahydrofuran, 344 mg of sodium borohydride was added in batches at -20 °C, slowly warmed to room temperature, and stirred at room temperature for 3 hours. At 0 °C, 100 mL of water was added, followed by extraction with EtOAc (80 mL×3). The organic phases were combined, dried over anhydrous sodium sulfate, concentrated under reduced pressure, and purified by column chromatography to obtain 0.94 g of compound M30-10.

Step 7: Preparation of Compound M30

0.94 g of compound M30-10 was dissolved in 10 mL of dichloromethane, 2.62 g of trifluoroacetic acid was added dropwise, and the mixture was stirred at room temperature for an hour. The reaction solution was lowered to 0 °C, and then a saturated aqueous sodium bicarbonate solution was added to adjust the pH to weakly alkaline. Extracted with a mixed solution of EtOAc:THF=1:1 (60 mL×4), combined the organic phases, dried over anhydrous sodium sulfate, and concentrated under reduced pressure. 692 mg of compound M30 were obtained.

Example 1 Preparation of Compound A01 (S)-(3-(1-amino-1,3-dihydrospiro [indene-2,4'-piperidine]-1'-yl)-6-(3-phenoxyprop-1-yn-1-yl)pyrazin-2-yl)methanol

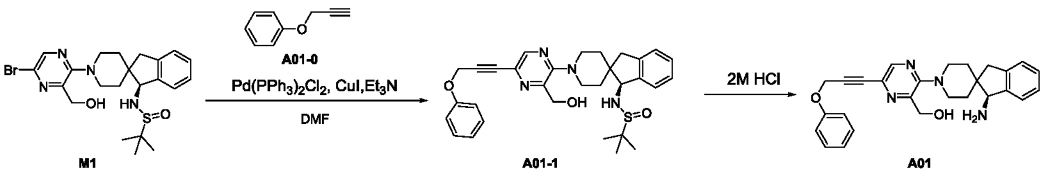


M1 A01-1 A01

Step 1: Preparation of Compound A01-1

100 mg of compound M1, 40 mg of compound A01-1 (phenyl propargyl ether), 14 mg of $PdCl_2(PPh_3)_2$, 2 mg of CuI were dissolved in DMF (3 mL) and $NEt_3$ (0.5 mL), after nitrogen replacement, 60 °C reaction 2.5 hours. LC-MS and TLC detected that the reaction was complete, 10 mL of ethyl acetate was added, the insolubles were filtered off, the filtrate was dispersed in 20 mL of water, the organic phase was washed with saturated brine, dried over anhydrous sodium sulfate, and concentrated in vacuo. The crude product was purified by preparative plate to obtain 40 mg gray solid compound A01-1.

Step 2: Preparation of Compound A01

40 mg of compound A01-1 was dissolved in 2.5 mL of dioxane and 0.5 mL of MeOH, 2N HCl (0.1 mL, methanol solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 19 mg of off-white solid compound A01.
[M+H+]=441.4.

A01: 1H NMR (500 MHz, DMSO-d6) δ 8.22 (s, 1H), 7.35-7.30 (m, 3H), 7.19-7.15 (m, 3H), 7.05-7.04 (m, 2H), 7.00-6.97 (m, 1H), 5.44 (t, J = 6.0 Hz, 1H), 5.08 (s, 2H), 4.46 (d, J = 6.0 Hz, 2H), 3.87 (s, 1H), 3.84-3.76 (m, 2H), 3.16 -3.10 (m, 2H), 3.04 (d, J = 15.6 Hz, 1H), 2.62 (d, J = 15.6 Hz, 1H), 1.92-1.85 (m, 1H), 1.88-1.73 (m , 1H), 1.78-1.72 (m, 1H), 1.14-1.12 (m, 1H).

Example 2 Preparation of Compound A02 (S)-(3-(1-amino-1,3-dihydrospiro [indene-2,4'-piperidine]-1'-yl)-6-(3-(3-methoxyphenoxy)prop-1-yn-1-yl) pyrazin-2-yl) methanol

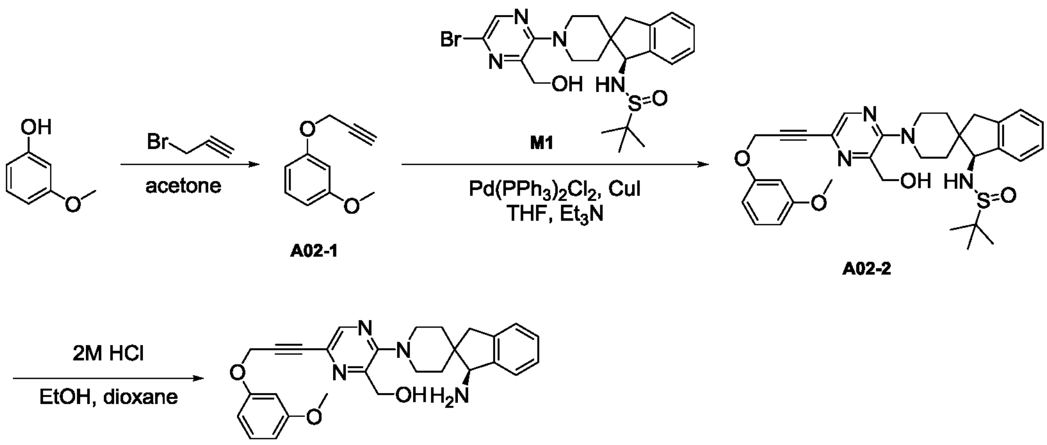


A02-1

A02-2

A02

Step 1: Preparation of Compound A02-1

Dissolve 0.18 g m-methoxyphenol and 0.80 g potassium carbonate in acetone (5 mL), heat at 60 °C for 0.5 h, add 0.23 g bromopropyne, and continue stirring for 2 h. TLC detected that the reaction was complete, filtered, concentrated the filtrate, and purified the crude product by column chromatography to obtain 164 mg of light yellow transparent oil, namely compound A02-1.

Step 2: Preparation of Compound A02-2

100 mg of compound M1, 99 mg of compound A02-1, 7.1 mg of $PdCl_2(PPh_3)_2$, and 1.9 mg of CuI were dissolved in THF (2 mL) and $NEt_3$ (0.2 mL), and after nitrogen replacement, the reaction was carried out at 60 °C for 3 hours. LC-MS and TLC detected that the reaction was complete, 10 mL of ethyl acetate was added, 20 mL of water was added to disperse, the organic phase was washed with saturated brine, dried over anhydrous sodium sulfate, and concentrated in vacuo. The crude product was purified by column chromatography to obtain 103 mg of pale yellow solid. Namely compound A02-2.

Step 3: Preparation of Compound A02

103 mg of compound A02-2 was dissolved in 5 mL of dioxane and 1 mL of EtOH, 2N HCl (134 $\mu$L, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 25.4 mg of off-white solid compound A02.

$[M+H^+]$=471.3

A02: $^1$H NMR (500 MHz, DMSO-$d_6$) δ 8.21 (s, 1H), 7.30 (d, *J* = 6.7 Hz, 1H), 7.22 (t, *J* = 8.2 Hz, 1H), 7.19-7.11 (m, 3H), 6.65-6.54 (m, 3H), 5.42 (t, *J* = 5.9 Hz, 1H), 5.06 (s, 2H), 4.47 (d, *J* = 5.9 Hz, 2H), 3.84 (s, 1H), 3.81-3.75 (m, 2H), 3.74 (s, 3H), 3.19-3.06 (m, 2H), 3.03 (d, *J* = 15.5 Hz, 1H), 2.57 (d, *J* = 15.5 Hz, 1H), 1.91-1.81 (m, 1H), 1.81-1.71 (m, 1H), 1.55-1.48 (m, 1H), 1.14-1.08 (m, 1H).

Example 3 Preparation of Compound A03 (S)-(3-(1-amino-1,3-dihydrospiro [indene-2,4'-piperidin]-1'-yl)-6-(3-(3-Aminophenoxy))prop-1-yn-1-yl)pyrazin-2-yl)m ethanol

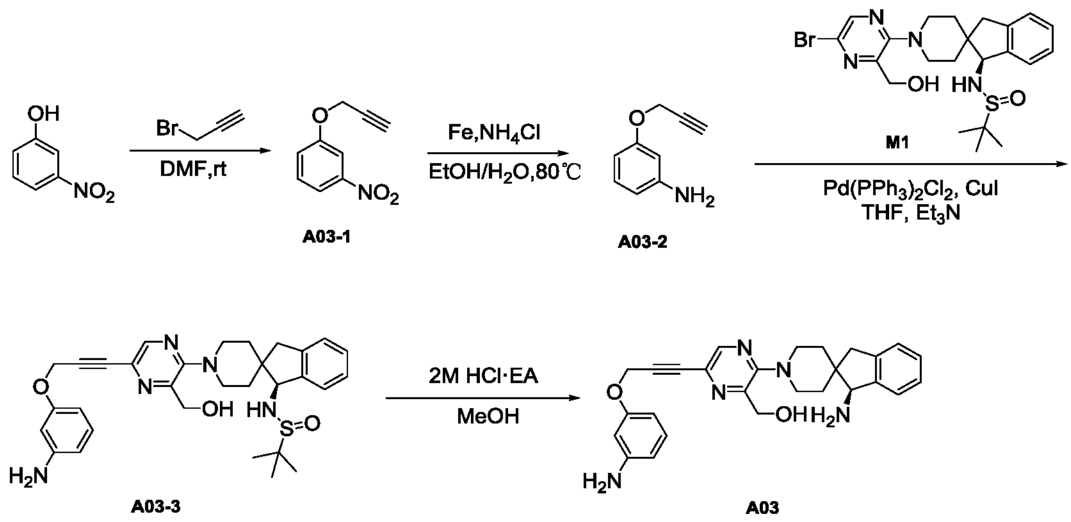


Step 1: Preparation of Compound A03-1

1.00 g of m-nitrophenol and 1.49 g of potassium carbonate were dissolved in DMF (10 mL), reacted at room temperature for 0.5 hour, 1.03 g of bromopropyne was added, and stirring was continued for 2 hours. TLC detected the completion of the reaction, added water (20 mL), extracted with ethyl acetate (3 × 15 mL), combined the organic phases, washed with saturated brine successively, dried over anhydrous sodium sulfate, filtered, concentrated the filtrate, and purified the crude product by column chromatography 1.10 g of brownish-yellow liquid was obtained, namely compound A03-1.

Step 2: Preparation of Compound A03-2

Dissolve 1.10g A03-1, 1.30g iron powder, and 2.08g ammonium chloride in ethanol/water (8 mL/8 mL), and react at 80 °C for 1 hour. TLC detected that the reaction was complete, the reaction solution was cooled to room temperature, filtered, the ethanol in the filtrate was removed by spin, the residue was extracted with ethyl acetate (3 × 10 mL), the combined organic phases were dried over anhydrous sodium sulfate, filtered, the filtrate was concentrated, and the crude product was Purified by column chromatography to obtain 900 mg of brown-red liquid, namely compound A03-2.

Step 3: Preparation of Compound A03-3

100 mg of compound M1, 45 mg of compound A03-2, 7.1 mg of $PdC_{12}(PPh_3)_2$, 1.9 mg of CuI were dissolved in DMF (2 mL) and $NEt_3$ (0.2 mL), and after nitrogen replacement, the reaction was performed at 60 °C for 18 hours. LC-MS and TLC detected the completion of the reaction, added 10 mL of ethyl acetate, dispersed in 10 mL of water, washed the organic phase with saturated brine, dried over anhydrous sodium sulfate, and concentrated in vacuo. The crude product was purified by column chromatography to obtain 76 mg of pale yellow solid, namely compound A03-3.

Step 4: Preparation of Compound A03

76 mg of compound A03-3 was dissolved in 2 mL of methanol, 2N HCl (200 μL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 17 mg of pale yellow solid, compound A03.

[M+H+]=456.3.

1H NMR (500 MHz, DMSO-d6) δ 8.22 (s, 1H), 7.30 (d, J = 6.5 Hz, 1H), 7.18-7.15 (m, 3H), 6.93 (t, J = 8.0 Hz, 1H) , 6.22-6.17 (m, 3H), 5.43 (t, J = 6.0 Hz, 1H), 5.10 (s, 2H), 4.94 (s, 2H) , 4.47 (d, J = 6.0 Hz, 2H), 3.86- 3.76 (m, 3H), 3.16-3.09 (m, 2H), 3.03 (d, J = 15.5 Hz, 1H), 2.60 (d, J = 15.5 Hz, 1H), 1.89-1.84 (m, 1H), 1.79 -1.73 (m, 1H), 1.53-1.50 (m, 1H), 1.12-1.10 (m, 1H).

Example 4 Preparation of Compound A04 (S)-N-(3-((3-(5-(1-amino-1,3-dihydrospiro[indene-2,4'-piperidin]-1'-yl)-6-(hydroxym ethyl)pyrazin-2-yl)prop-2-yn-1-yl)oxy)phenyl)acetamide

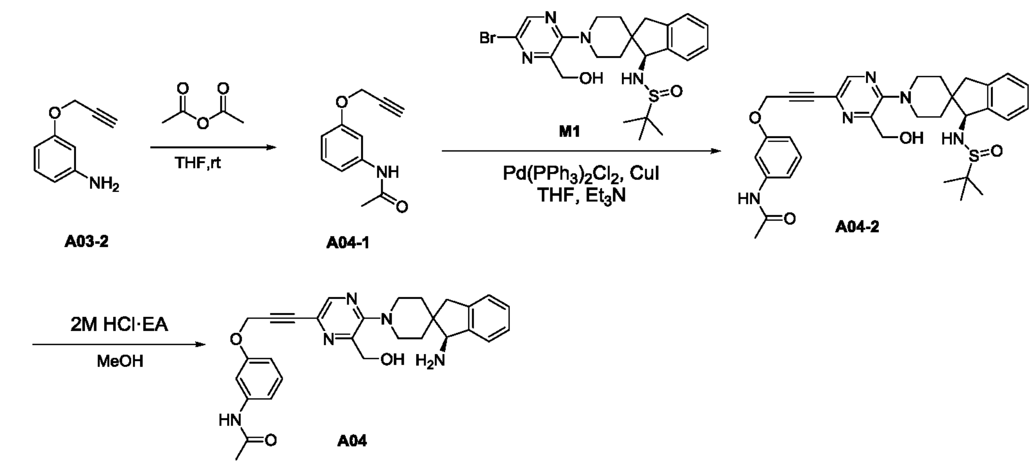


Step 1: Preparation of Compound A04-1

200 mg of A03-2 and 159 mg of acetic anhydride were dissolved in THF (5 mL) and reacted at room temperature for 0.5 h. TLC detected the completion of the reaction, added water (10 mL), extracted with ethyl acetate (3 × 10 mL), combined the organic phases, washed with saturated brine successively, dried over anhydrous sodium sulfate, filtered, concentrated the filtrate, and purified the crude product by column chromatography 200 mg of brownish-yellow solid were obtained, namely compound A04-1.

Step 3: Preparation of Compound A04-2

260 mg of compound M1, 150 mg of compound A04-2, 18.60 mg of $PdCl_2(PPh_3)_2$, 5.0 mg of CuI were dissolved in DMF (2 mL) and $NEt_3$ (0.4 mL), and after nitrogen replacement, the reaction was carried out at 60 °C for 18 hours. LC-MS and TLC detected that the reaction was complete, 20 mL of ethyl acetate was added, dispersed in 15 mL of water, the organic phase was washed with saturated brine, dried over anhydrous sodium sulfate, and concentrated in vacuo. The crude product was purified by column chromatography to obtain 150 mg of pale yellow solid. Namely compound A04-2.

Step 4: Preparation of Compound A04

44 mg of compound A04-2 was dissolved in 2 mL of methanol, 2N HCl (200 μL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 14.8 mg of pale yellow solid compound A04.

[M+H+]=498.3.

1H NMR (500 MHz, DMSO-d6) δ9.95 (s, 1H), 8.23 (s, 1H), 7.38-7.37 (m, 1H), 7.30 (d, J = 6.5 Hz, 1H), 7.23 (t , J = 8.0 Hz, 1H), 7.18-7.14 (m, 4H), 6.74-6.72 (m, 1H), 5.42 (t, J = 6.0 Hz, 1H), 5.03 (s, 2H), 4.47 (d, J = 6.0 Hz, 2H), 3.84-3.77 (m, 3H), 3.14-3.07 (m, 2H), 3.03 (d, J = 15.5 Hz, 1H), 2.60 (d, J = 15.5 Hz, 1H), 2.03 (s, 3H), 1.89-1.83 (m, 1H), 1.79-1.74 (m, 1H), 1.53-1.50 (m, 1H), 1.15-1.10 (m, 1H).

Example 6 Preparation of Compound A06 (S)-(3-(1-amino-1,3-dihydrospiro[indene-2,4'-piperidin]-1'-yl)-6-(3-((3-methoxyphen yl)thio)prop-1-yn-1-yl)pyrazin-2-yl)methanol

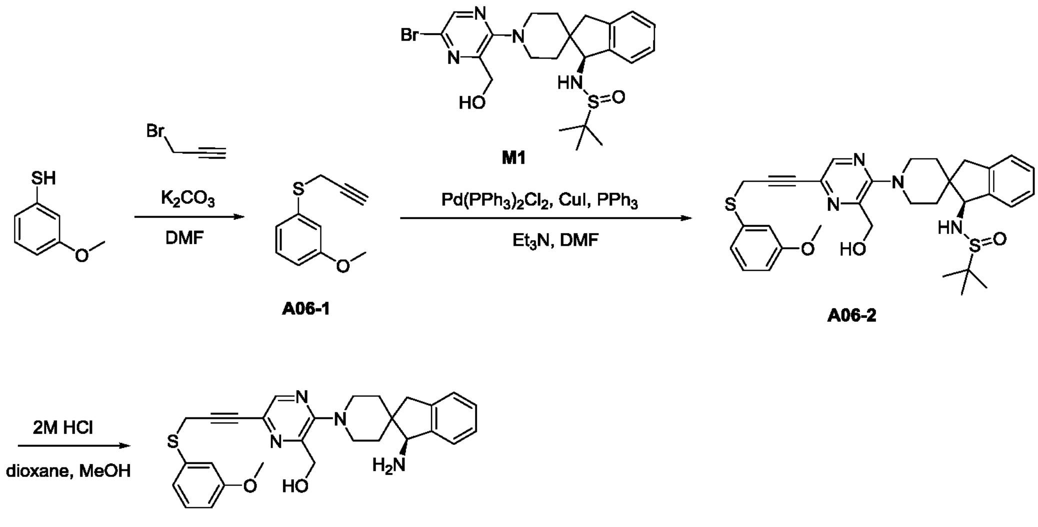


M1

A06-1

A06-2

A06

Step 1: Preparation of Compound A06-1

Under nitrogen protection, 1.00 g of m-methoxythiophenol, 1.02 g of bromopropyne and 1.48 g of potassium carbonate were dissolved in DMF (10 mL) and reacted at room temperature for 2 hours. TLC detected that the reaction was complete, filtered, concentrated the filtrate, and purified the crude product by column chromatography to obtain 840 mg of colorless and transparent oil A06-1.

Step 2: Preparation of Compound A06-2

150 mg of compound M1, 49 mg of compound A06-1, 6.4 mg of $PdCl_2(PPh_3)_2$, 3.5 mg of CuI, and 4.8 mg of $PPh_3$ were dissolved in DMF (2 mL) and $NEt_3$ (0.5 mL), and the reaction was carried out at 60 °C after nitrogen replacement 2.5 hours. LC-MS and TLC detected that the reaction was complete, 10 mL of ethyl acetate was added, insolubles were filtered off, the filtrate was dispersed in 20 mL of water, the organic phase was washed with saturated brine, dried over anhydrous sodium sulfate, and concentrated in vacuo. The crude product was purified by column chromatography to obtain 35 mg of pale yellow solid, namely compound A06-2.

Step 3: Preparation of Compound A06

35 mg of compound A06-2 was dissolved in 2 mL of dioxane and 0.5 mL of MeOH, 2 M HCl (50 μL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 5.1 mg of yellow solid compound A06.

[M+H+]=487.31.

1H NMR (500 MHz, DMSO-d6) δ 8.11 (s, 1H), 7.32 (d, J = 6.4 Hz, 1H), 7.28 (t, J = 7.9 Hz, 1H), 7.19-7.14 (m, 3H) , 7.06-6.98 (m, 2H), 6.83-6.80 (m, 1H), 4.45 (s, 2H), 4.17 (s, 2H), 3.89 (s, 1H), 3.79-3.73 (m, 5H), 3.13 -3.02 (m, 3H), 2.63 (d, J = 15.6 Hz, 1H), 1.89-1.85 (m, 1H), 1.79-17.3 (m, 1H), 1.52-1.49 (m, 1H), 1.17-1.13 (m, 1H).

Example 7 Preparation of Compound A07 (S)-(3-(1-amino-1,3-dihydrospiro [indene-2,4'-piperidine]-1'-yl)-6-(3-(phenylamino)prop-1-yn-1-yl)pyrazin-2-yl) methanol

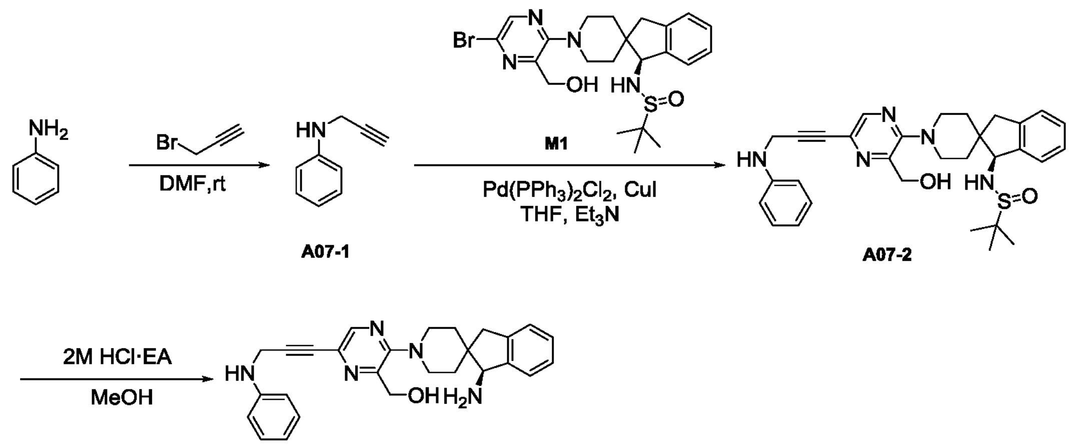


A07-1 A07-2 A07

Step 1: Preparation of Compound A07-1

Dissolve 0.50 g of aniline and 0.74 g of potassium carbonate in DMF (5 mL), react at room temperature for 0.5 hour, add 0.70 g of bromopropyne, and continue stirring for 2 hours. TLC detected the completion of the reaction, added water (15 mL), extracted with ethyl acetate (3 × 10 mL), combined the organic phases, washed with saturated brine successively, dried over anhydrous sodium sulfate, filtered, the filtrate was concentrated, and the crude product was purified by column chromatography 300 mg of pale yellow transparent liquid was obtained, namely compound A07-1.

Step 2: Preparation of Compound A07-2

100 mg of compound M1, 35 mg of compound A07-1, 7.1 mg of $PdCl_2(PPh_3)_2$, 1.9 mg of CuI were dissolved in DMF (2 mL) and $NEt_3$ (0.2 mL), and after nitrogen replacement, the reaction was performed at 60 °C for 3 hours. LC-MS and TLC detected that the reaction was complete, 10 mL of ethyl acetate was added, 10 mL of water dispersed, the organic phase was washed with saturated brine, dried over anhydrous sodium sulfate, and concentrated in vacuo. The crude product was purified by column chromatography to obtain 70 mg of pale yellow solid. Namely compound A07-2.

Step 3: Preparation of Compound A07

70 mg of compound A07-2 was dissolved in 2 mL of methanol, 2N HCl (200 μL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 15.3 mg of pale yellow solid, compound A07.

[M+H+]=440.3.

1H NMR (500 MHz, DMSO-d6) δ 8.14 (s, 1H), 7.30 (d, J = 6.5 Hz, 1H), 7.18-7.11 (m, 5H), 6.69 (d, J = 8.0 Hz, 2H) , 6.61 (t, J = 7.5 Hz, 1H), 6.09 (t, J = 6.0 Hz, 1H), 5.40 (t, J = 6.0 Hz, 1H), 4.44 (d, J = 6.0 Hz, 2H), 4.15 (d, J = 6.5 Hz, 2H), 3.84 (s, 1H), 3.77-3.70 (m, 2H), 3.13-3.06 (m, 2H), 3.02 (d, J = 15.5 Hz, 1H), 2.59 (d, J = 15.5 Hz, 1H), 1.89-1.83 (m, 1H), 1.79-1.73 (m, 1H), 1.52-1.48 (m, 1H), 1.12-1.09 (m, 1H).

Example 8 Preparation of Compound A08 (S)-N-(4-((3-(5-(1-amino-1,3-dihydrospiro [indene-2,4'-piperidine]-1'-yl) -6-(hydroxymethyl) pyrazin-2-yl) prop-2-yn-1-yl) oxy) phenyl) acetamide

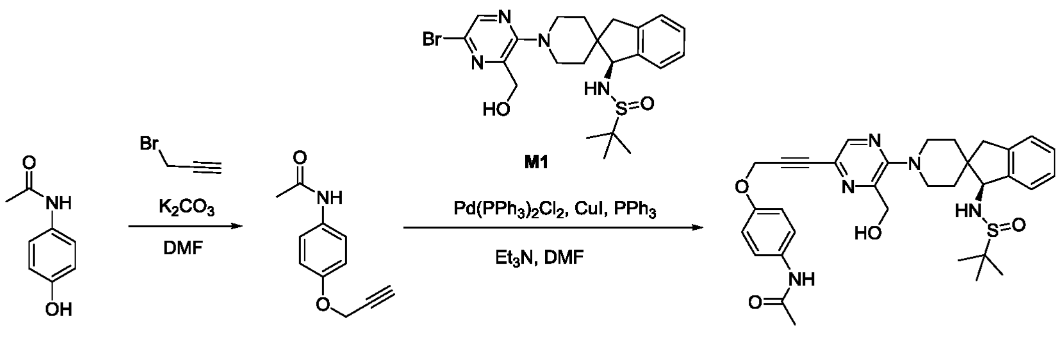


M1

A08-1

A08-2

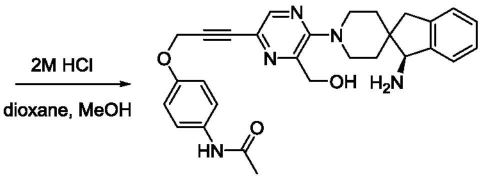


A08

Step 1: Preparation of Compound A08-1

Under nitrogen protection, 1.00 g of acetAminophen, 1.2 g of bromopropyne and 1.37 g of potassium carbonate were dissolved in DMF (10 mL) and reacted at room temperature for 2 hours. TLC detected that the reaction was complete, filtered, concentrated the filtrate, and purified the crude product by column chromatography to obtain 935 mg of white solid A08-1.

Step 2: Preparation of Compound A08-2

150 mg of compound M1, 69 mg of compound A08-1, 6.4 mg of $PdCl_2(PPh_3)_2$, 3.5 mg of CuI, and 4.8 mg of $PPh_3$ were dissolved in DMF (2 mL) and $NEt_3$ (0.5 mL), and the reaction was carried out at 60 °C after nitrogen replacement 8 hours. LC-MS and TLC detected that the reaction was complete, 10 mL of ethyl acetate was added, insolubles were filtered off, the filtrate was dispersed in 20 mL of water, the organic phase was washed with saturated brine, dried over anhydrous sodium sulfate, and concentrated in vacuo. The crude product was purified by column chromatography to obtain 166 mg of pale yellow solid, namely compound A08-2.

Step 3: Preparation of Compound A08

166 mg of compound A08-2 was dissolved in 2 mL of dioxane and 0.5 mL of MeOH, 2 M HCl (0.3 mL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 4.6 mg of white solid compound A08.

[M+H+]=498.34

1H NMR (500 MHz, DMSO-d6) δ 9.81 (s, 1H), 8.21 (s, 1H), 7.50 (d, J = 8.9 Hz, 2H), 7.30 (d, J = 6.8 Hz, 1H), 7.22 -7.11 (m, 3H), 6.97 (d, J = 8.8 Hz, 2H), 5.42 (t, J = 6.0 Hz, 1H), 5.03 (s, 2H), 4.46 (d, J = 6.0 Hz, 2H) , 3.84-3.76 (m, 3H), 3.17-3.01 (m, 3H), 2.60 (d, J = 16.0 Hz, 1H), 1.89-1.83 (m, 1H), 1.78-1.73 (m, 1H), 1.53 -1.49 (m, 1H), 1.15-1.12 (m, 1H).

Example 9 Preparation of Compound A09 (S)-(3-(1-amino-1,3-dihydrospiro [indene-2,4'-piperidine]-1'-yl)-6-(3-(4-methoxyphenoxy)prop-1-yn-1-yl) pyrazin-2-yl) methanol

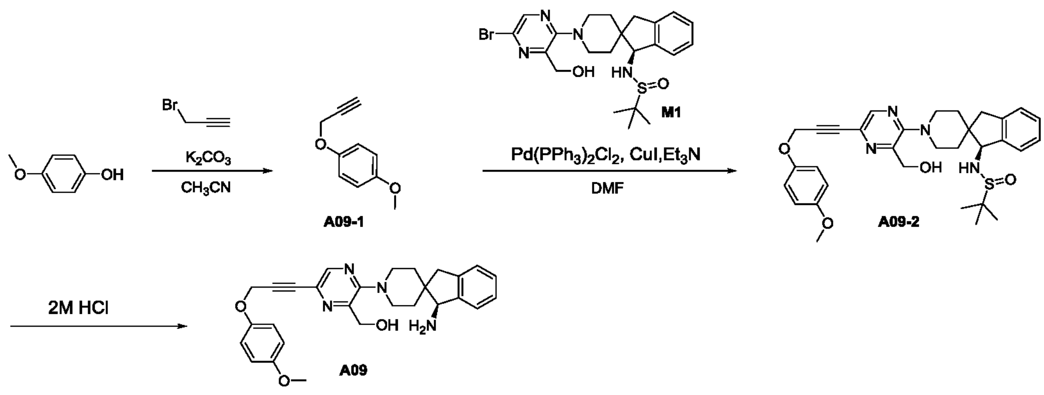


Step 1: Preparation of Compound A09-1

Under nitrogen protection, 1.00 g of p-methoxyphenol, 1.15 g of bromopropyne and 4.45 g of potassium carbonate were dissolved in $CH_3CN$ (10 mL) and heated at 60 °C for 2 hours. TLC detected that the reaction was complete, filtered, concentrated the filtrate, and purified the crude product by column chromatography to obtain 900 mg of colorless and transparent oil A09-1.

Step 2: Preparation of Compound A09-2

100 mg of compound M1, 49 mg of compound M1, 14 mg of $PdCl_2(PPh_3)_2$, and 2 mg of CuI were dissolved in DMF (3 mL) and $NEt_3$ (0.5 mL). After nitrogen replacement, the reaction was performed at 60 °C for 2.5 hours. LC-MS and TLC detected that the reaction was complete, 10 mL of ethyl acetate was added, the insolubles were filtered off, the filtrate was dispersed in 20 mL of water, the organic phase was washed with saturated brine, dried over anhydrous sodium sulfate, and concentrated in vacuo. The crude product was purified by preparative plate to obtain 40 mg pale yellow solid, namely compound A09-2.

Step 3: Preparation of Compound A09

40 mg of compound A09-2 was dissolved in 2.5 mL of dioxane and 0.5 mL of MeOH, 2N HCl (0.1 mL, methanol solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 16 mg of off-white solid compound A09.

[M+H+]=471.4.

1H NMR (500 MHz, DMSO-d6) δ 8.21 (s, 1H), 7.31-7.30 (m, 1H), 7.19-7.15 (m, 3H), 6.99-6.97 (m, 2H), 6.90-6.88 (m , 2H), 5.42 (t, J = 6.0 Hz, 1H), 5.00 (s, 2H), 4.47 (d, J = 6.0 Hz, 2H), 3.86 (s, 1H), 3.83- 3.76 (m, 2H), 3.71 (s, 3H), 3.16 -3.10 (m, 2H), 3.04 (d, J = 15.6 Hz, 1H), 2.62 (d, J = 15.6 Hz, 1H), 1.92 -1.85 (m, 1H), 1.88 - 1.83 (m, 1H), 1.78-1.73 (m, 1H), 1.14-1.12 (m, 1H).

Example 13 Preparation of Compound A13 (S)-(3-(1-amino-1,3-dihydrospiro [indene-2,4'-piperidine]-1'-yl)-6-(3-(trifluoromethyl)phenoxy)prop-1-yn-1-yl) pyrazin-2-yl) methanol

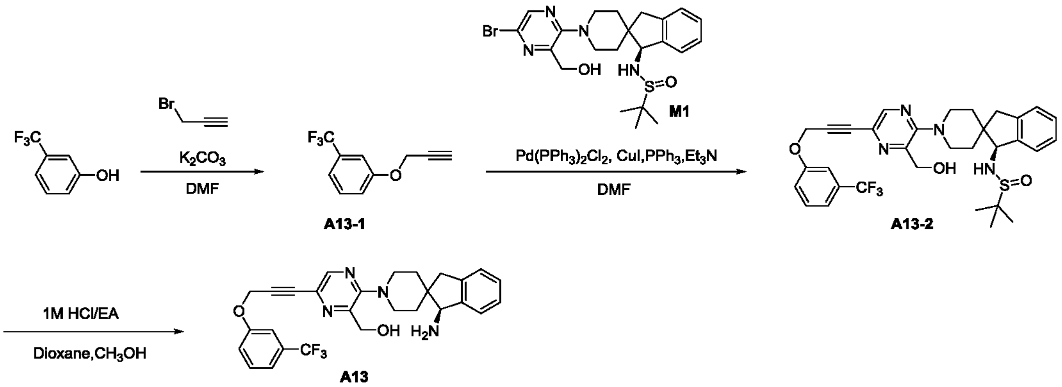


M1

A13-1

A13-2

A13

Step 1: Preparation of Compound A13-1

Under nitrogen protection, 1.00 g of m-trifluoromethylphenol, 1.10 g of bromopropyne and 1.71 g of potassium carbonate were dissolved in DMF (10 mL) and reacted at room temperature for 4 h. TLC detected that the reaction was complete, filtered, concentrated the filtrate, and purified the crude product by column chromatography to obtain 1.10 g of colorless and transparent oil A13-1.

Step 2: Preparation of Compound A13-2

100 mg of compound M1, 60.9 mg of compound A13-1, 4.3 mg of $PdCl_2(PPh_3)_2$, 3.2 mg of $PPh_3$, 2.4 mg of CuI were dissolved in DMF (2 mL) and $NEt_3$ (0.5 mL), after nitrogen replacement, 60 °C reaction for 14h. LC-MS and TLC detected that the reaction was complete, 10 mL of ethyl acetate was added, insolubles were filtered off, the filtrate was dispersed in 20 mL of water, the organic phase was washed with saturated brine, dried over anhydrous sodium sulfate, and concentrated in vacuo. The crude product was purified by preparative plate to obtain 90 mg pale yellow solid, namely compound A13-2.

Step 3: Preparation of Compound A13

90 mg of compound A13-2 was dissolved in 4.0 mL of dioxane and 1.0 mL of MeOH, 1N HCl (0.3 mL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with EA (3×3 mL), the organic phase was dried and concentrated in vacuo, and purified by preparative plate to obtain 28 mg of off-white solid compound A13.

[M+H+]=509.32.

1H NMR (500 MHz, DMSO) δ 8.23 (s, 1H), 7.71 (d, J = 8.4 Hz, 2H), 7.30 (d, J = 6.4 Hz, 1H), 7.24 (d, J = 8.4 Hz, 2H) ), 7.19-7.13 (m, 3H), 5.42 (t, J = 6.0 Hz, 1H), 5.20 (s, 2H), 4.47 (d, J = 5.0 Hz, 2H), 3.88-3.73 (m, 3H) , 3.16-3.11 (m, 2H), 3.03 (d, J = 15.7 Hz, 1H), 2.61 (d, J = 15.6 Hz, 1H), 1.89-1.83 (m, 1H), 1.79-1.72 (m, 1H) ), 1.52-1.49 (m, 1H), 1.13-1.11 (m, 1H).

Example 17 Preparation of Compound A17 (S)-4-((3-(5-(1-amino-1,3-dihydrospiro[indene-2,4'-piperidine]-1'-yl)-6-(hydroxymeth yl)pyrazin-2-yl)prop-2-yn-1-yl) oxy) benzamide

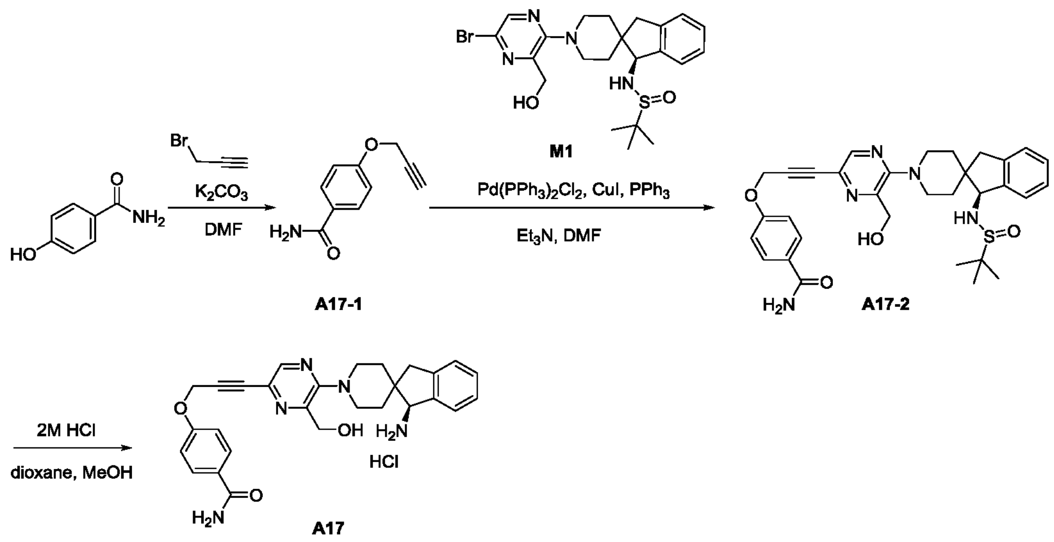


Step 1: Preparation of Compound A17-1

Under nitrogen protection, 1.00 g of p-hydroxybenzamide, 1.04 g of bromopropyne and 1.51 g of potassium carbonate were dissolved in DMF (10 mL) and reacted at room temperature for 2 hours. TLC detected that the reaction was complete, filtered, concentrated the filtrate, and purified the crude product by column chromatography to obtain 1.08 g of white solid A17-1.

Step 2: Preparation of Compound A17-2

150 mg of compound M1, 64 mg of compound A17-1, 6.4 mg of $PdCl_2(PPh_3)_2$, 3.5 mg of CuI, and 4.8 mg of $PPh_3$ were dissolved in DMF (2 mL) and $NEt_3$ (0.5 mL), and the reaction was carried out at 60 °C after nitrogen replacement. 8 hours. LC-MS and TLC detected that the reaction was complete, 10 mL of ethyl acetate was added, insolubles were filtered off, the filtrate was dispersed in 20 mL of water, the organic phase was washed with saturated brine, dried over anhydrous sodium sulfate, and concentrated in vacuo. The crude product was purified by column chromatography to obtain 127 mg of pale yellow solid, namely compound A17-2.

Step 3: Preparation of Compound A17

127 mg of compound A17-2 was dissolved in 2 mL of dioxane and 0.5 mL of MeOH, 2 M HCl (0.3 mL, ethyl acetate solution) was added, and the reaction was stirred at RT for 1 hour. LC-MS and TLC detected that the reaction was complete, the reaction solution was concentrated under reduced pressure, slurried with dichloromethane and methyl tert-butyl ether in sequence, and filtered to obtain 109.5 mg of a yellow solid, the hydrochloride of compound A17.

[M+H+]=484.36

1H NMR (500 MHz, DMSO-d6) δ 8.25 (s, 1H), 7.88 (d, J = 8.8 Hz, 3H), 7.57 (d, J = 7.4 Hz, 1H), 7.37-7.27 (m, 3H) , 7.12-7.04 (m, 2H), 5.16 (s, 2H), 4.48 (s, 2H), 4.36 (d, J = 5.4 Hz, 1H), 3.92-3.88 (m, 2H), 3.21-3.11 (m , 3H), 2.97 (d, J = 16.2 Hz, 1H), 1.89-1.81 (m, 2H), 1.58-1.55 (m, 1H), 1.52-1.49 (m, 1H).

Example 18 Preparation of Compound A18 (S) - (3 - (1-amino-1,3-dihydrospiro [indene-2,4'-piperidine]-1'-yl)-6-(3-(pyridin-4-oxy)prop-1-yn-1-yl)pyrazin-2-yl) methanol

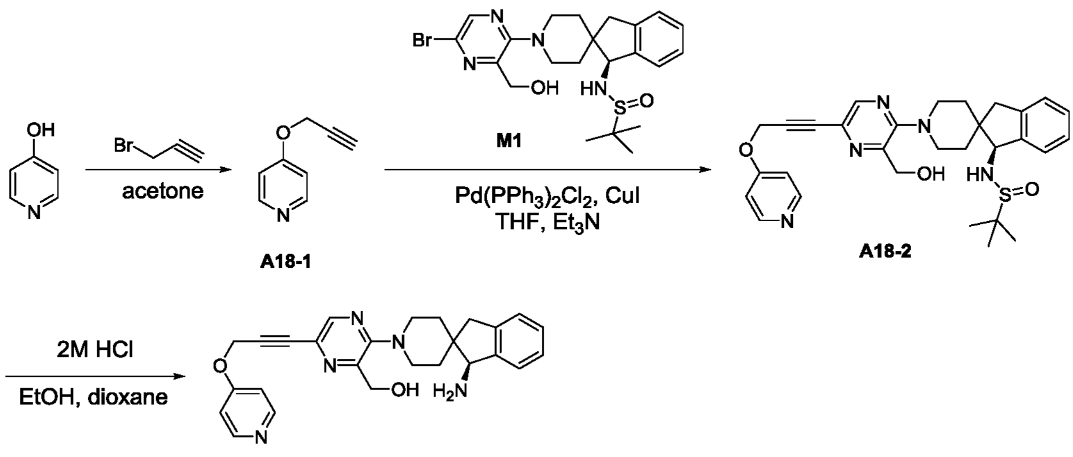


A18

Step 1: Preparation of Compound A18-1

Dissolve 0.5 g of 4-hydroxypyridine and 1.45 g of potassium carbonate in acetone (5 mL), heat at 50 °C for 0.5 hour reaction, add 1.25 g of bromopropyne, and continue stirring for 2 hours. TLC detected that the reaction was complete, filtered, concentrated the filtrate, and purified the crude product by column chromatography to obtain 384 mg of brown solid, namely compound A18-1.

Step 2: Preparation of Compound A18-2

100 mg of compound M1, 48.5 mg of compound A18-1, 7.1 mg of $PdCl_2(PPh_3)_2$, 1.9 mg of CuI were dissolved in THF (2 mL) and $NEt_3$ (0.2 mL), and after nitrogen replacement, the reaction was carried out at 60°C for 3 hours. LC-MS and TLC detected that the reaction was complete, 10 mL of dichloromethane and 20 mL of water were added for extraction, the organic phase was washed with saturated brine, dried over anhydrous sodium sulfate, and concentrated in vacuo. The crude product was purified by preparative plate to obtain 101 mg of yellow solid, namely Compound A18-2.

Step 3: Preparation of Compound A18

101 mg of compound A18-2 was dissolved in 3 mL of dioxane and 0.8 mL of EtOH, 2N HCl (138 μL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 22.7 mg of yellow solid, compound A18.

[M+H+]=442.3.

1H NMR (500 MHz, DMSO-d6) δ 8.24 (s, 1H), 7.75 (d, J = 7.5 Hz, 2H), 7.30 (d, J = 6.8 Hz, 1H), 7.18-7.12 (m, 3H) , 6.14 (d, J = 7.5 Hz, 2H), 5.43 (t, J = 5.5 Hz, 1H), 5.10 (s, 2H), 4.47 (d, J = 5.5 Hz, 2H), 3.88-3.76 (m, 3H), 3.19-3.07 (m, 2H), 3.03 (d, J = 15.6 Hz, 1H), 2.60 (d, J = 15.6 Hz, 1H), 1.90-1.82 (m, 1H), 1.80-1.71 (m , 1H), 1.55-1.48 (m, 1H), 1.13-1.07 (m, 1H).

Example 21 Preparation of Compound A21 (S)-1-(4-(4-(3-(5-(1-amino-1,3-dihydrospiro [indene-2,4 '-piperidine]-1'-yl) -6-(hydroxymethyl) pyrazin-2-yl) prop-2-yn-1-yl)oxy)piperazin-1-yl)ethane-1-one

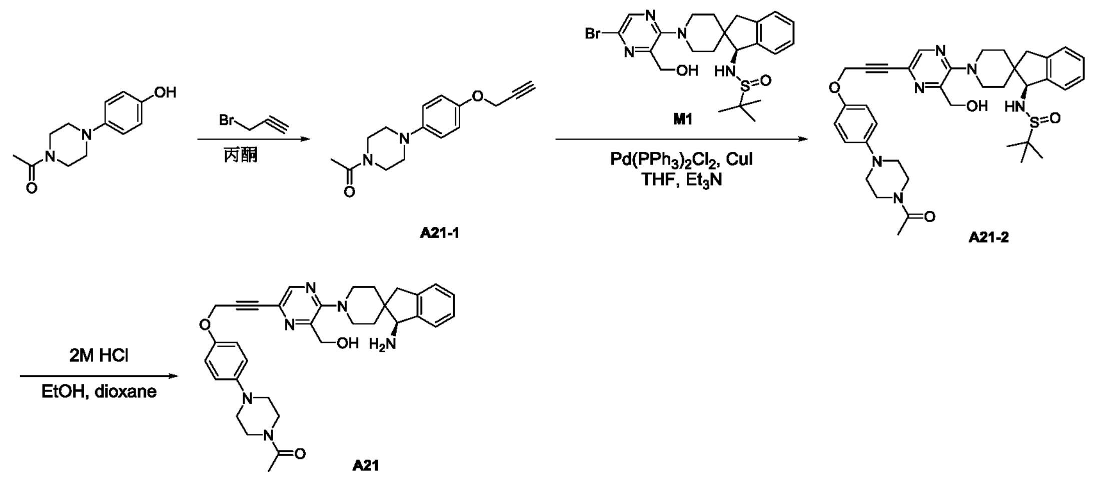


M1 A21-1 A21-2 A21

Step 1: Preparation of Compound A21-1

Dissolve 1.0 g of 1-acetyl-4-(4-hydroxyphenyl)piperazine and 2.51 g of potassium carbonate in acetone (8 mL), heat at 50 °C for 0.5 hours, add 0.73 g of bromopropyne, and continue stirring for 2 hours . TLC detected that the reaction was complete, filtered, concentrated the filtrate, and purified the crude product by column chromatography to obtain 1.09 g of an off-white solid, namely compound A21-1.

Step 2: Preparation of Compound A21-2

100 mg of compound M1, 47.1 mg of compound A21-1, 7.1 mg of $PdCl_2(PPh_3)_2$, 1.9 mg of CuI were dissolved in THF (2 mL) and $NEt_3$ (0.2 mL), and after nitrogen replacement, the reaction was carried out at 60 °C for 3 hours. LC-MS and TLC detected that the reaction was complete, added 10 mL of dichloromethane, dispersed in 20 mL of water, washed the organic phase with saturated brine, dried over anhydrous sodium sulfate, and concentrated in vacuo. A21-2.

Step 3: Preparation of Compound A21

76 mg of compound A21-2 was dissolved in 3 mL of dioxane and 0.7 mL of EtOH, 2N HCl (85 μL, ethyl acetate solution) was added, and the reaction was stirred at RT for 2 hours. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 20.7 mg of yellow solid compound A21.

[M+H+]=567.4.

1H NMR (500 MHz, DMSO-d6) δ 8.21 (s, 1H), 7.30 (d, J = 6.8 Hz, 1H), 7.19-7.12 (m, 3H), 6.96-6.92 (m, 4H), 5.43 (t, J = 5.9 Hz, 1H), 4.99 (s, 2H), 4.46 (d, J = 5.9 Hz, 2H), 3.86-3.74 (m, 3H), 3.57-3.54 (m, 4H), 3.20-3.10 (m, 3H), 3.05-3.01 (m, 2H), 2.98-2.95 (m, 2H), 2.60 (d, J = 15.6 Hz, 1H), 2.03 (s, 3H), 1.91-1.82 (m, 1H) ), 1.80-1.72 (m, 1H), 1.56-1.47 (m, 1H), 1.14-1.07 (m, 1H).

Example 22 Preparation of Compound A22 (S)-7-(3-(5-(1-amino-1,3-dihydrospiro[indene-2,4'-piperidine]-1'-yl)-6-(hydroxymeth yl)pyrazin-2-yl)prop-2-yn-1-yl) oxy)-3,4-dihydroquinoline-2 (1H)-one

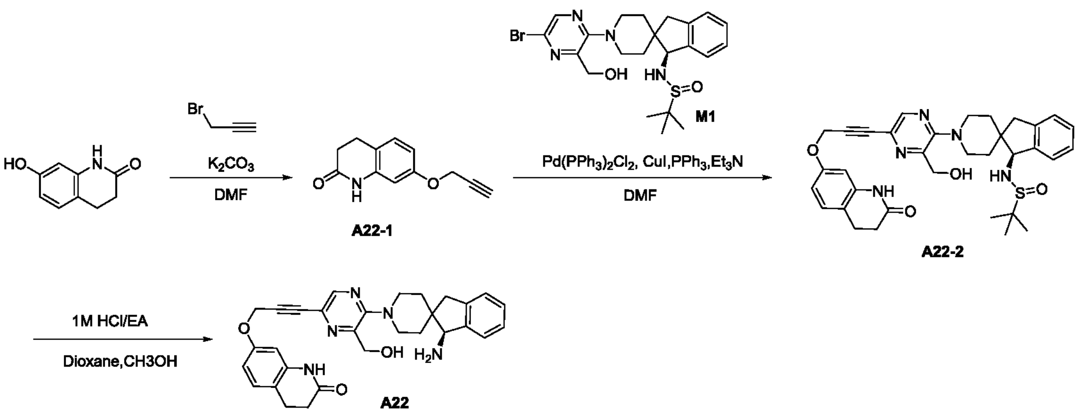


Step 1: Preparation of Compound A22-1

Under nitrogen protection, 1.00 g of 7-hydroxy-3,4-dihydroquinolone, 1.09 g of bromopropyne and 1.69 g of potassium carbonate were dissolved in DMF (10 mL) and reacted at room temperature for 24 h. TLC detected that the reaction was complete, filtered, concentrated the filtrate, and beat the crude product with (PE:EA=3:1) to obtain 0.90 g of pale yellow solid A22-1.

Step 2: Preparation of Compound A22-2

200 mg of compound M1, 89.7 mg of compound A22-1, 14 mg of $PdCl_2(PPh_3)_2$, and 4.0 mg of CuI were dissolved in DMF (4 mL) and $Et_3N$ (0.4 mL), and the reaction was carried out at 60 °C for 10 h after nitrogen replacement. LC-MS and TLC detected that the reaction was complete, the reaction solution was spun off with triethylamine, then cooled to room temperature, 20 mL of water was added, a solid was precipitated, filtered, the filter cake was washed with water, the solid was dissolved in DCM, dried, and the crude product was purified by a preparative plate to obtain 135 mg of pale yellow solid, namely compound A22-2.

Step 3: Preparation of Compound A22

200 mg of compound A22-2 was dissolved in 4.0 mL of dioxane and 1.0 mL of MeOH, 1N HCl (0.65 mL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, a solid was precipitated, filtered, and the filter cake was dissolved in DCM, dried and concentrated in vacuo, and purified by HPLC to obtain 16.8 mg of off-white solid compound A22.

[M+H+]=510.36.

Example 26 Preparation of Compound A26 (S)-(3-(1-amino-1,3-dihydrospiro [indene-2,4'-piperidine]-1'-yl)-6-(3-((2-Aminopyridin-4-yl)oxy)prop-1-yn-1-yl) pyrazin-2-yl) methanol

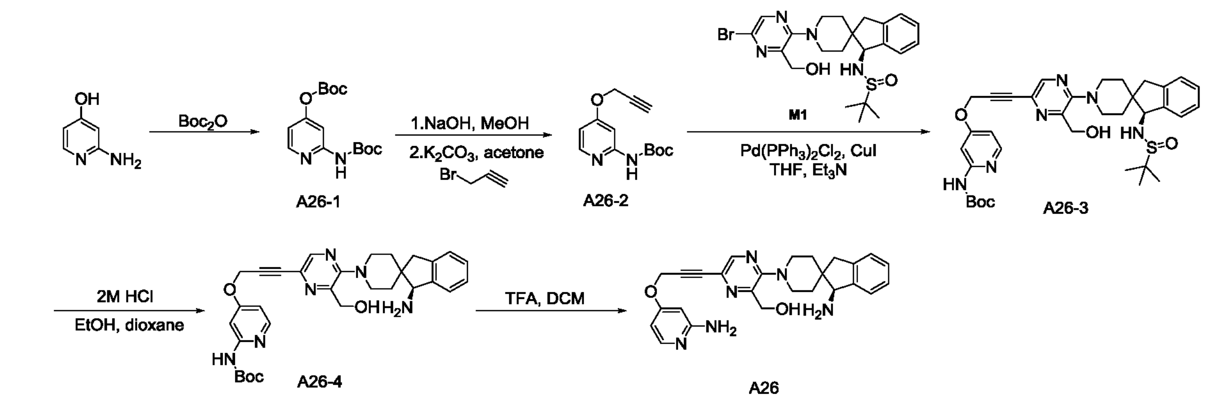


Step 1: Preparation of Compound A26-1

1.00 g of 2-amino-4-hydroxypyridine and 3.96 g of di-tert-butyl dicarbonate were dissolved in n-butanol (10 mL) and heated at 50 °C for 5 hours. TLC detected that the reaction no longer proceeded, 10 mL of ethyl acetate was added, dispersed in 20 mL of water, the organic phase was washed with saturated brine, dried over anhydrous sodium sulfate, and concentrated in vacuo. The crude product was purified by column chromatography to obtain 1.02 g of a white solid, namely compound A26 -1.

Step 2: Preparation of Compound A26-2

350 mg of compound A26-1 and 135 mg of sodium hydroxide were dissolved in methanol (5 mL) and reacted at 65 °C for 1.5 hours. LC-MS and TLC detected that the reaction was complete, filtered, and the filtrate was concentrated. The crude product, 268 mg of bromopropyne, and 312 mg of potassium carbonate were dissolved in acetone (5 mL), and the reaction was carried out at 60 °C for 2 hours. LC-MS and TLC detected that the reaction was complete, filtered, and concentrated the filtrate. The crude product was purified by preparative plate to obtain 310 mg of white solid, namely compound A26-2.

Step 3: Preparation of Compound A26-3

Dissolve 200 mg of compound M1, 100.6 mg of compound A26-2, 14.2 mg of $PdCl_2(PPh_3)_2$, 3.9 mg of CuI in tetrahydrofuran (4 mL) and triethylamine (1 mL), after nitrogen replacement, react at 60 °C for 4 hours, An additional 100.6 mg of compound A26-2 was added, and the reaction was carried out at 60 °C for 12 hours. LC-MS and TLC detection reaction no longer proceeded, 10 mL of dichloromethane was added, 20 mL of water dispersed, the organic phase was washed with saturated brine, dried over anhydrous sodium sulfate, and concentrated in vacuo. The crude product was purified by preparative plate to obtain 158 mg of yellow solid, Namely compound A26-3.

Step 4: Preparation of Compound A26-4

158 mg of compound A26-3 was dissolved in dioxane (4 mL) and ethanol (1 mL), 2N HCl (179 µL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 65 mg of yellow solid, namely compound A26-4 .

Step 5: Preparation of Compound A26

65 mg of compound A26-4 was dissolved in dichloromethane (1.5 mL) and trifluoroacetic acid (1.5 mL), and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with

dichloromethane (3×3 mL), the organic phase was dried and concentrated in vacuo, and purified by preparative plate to obtain 27.5 mg of white solid compound A26.

[M+H+]=457.3.

1H NMR (500 MHz, DMSO-d6) δ 8.23 (s, 1H), 7.78 - 7.74 (m, 1H), 7.32 - 7.27 (m, 1H), 7.20 - 7.12 (m, 3H), 6.21 (dd, J = 5.8, 2.3 Hz, 1H), 6.05 (d, J = 2.3 Hz, 1H), 5.88 (s, 2H), 5.44 (t, J = 6.0 Hz, 1H), 5.04 (s, 2H), 4.47 (d , J = 6.0 Hz, 2H), 3.84 (s, 1H), 3.82 - 3.77 (m, 2H), 3.20 - 3.07 (m, 2H), 3.03 (d, J = 15.6 Hz, 1H), 2.60 (d, J = 15.6 Hz, 1H), 1.93 - 1.82 (m, 1H), 1.81 - 1.71 (m, 1H), 1.56 - 1.46 (m, 1H), 1.14 - 1.08 (m, 1H).

Example 27 Preparation of Compound A27 (S)-3-((3-(5-(1-amino-1,3-dihydrospiro[indene-2,4'-piperidine]-1'-yl)-6-(hydroxymethyl) pyrazin-2-yl) prop-2-yn-1-yl)oxy)benzamide

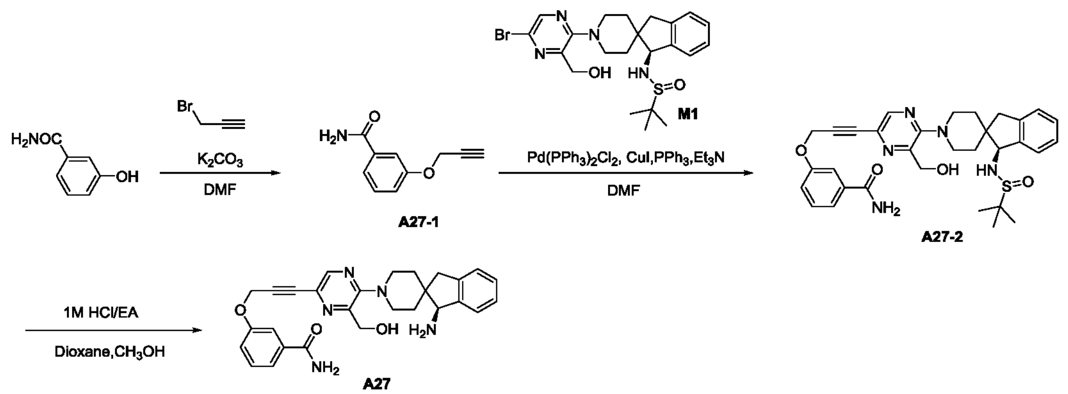


Step 1: Preparation of Compound A27-1

Under nitrogen protection, 1.00 g m-acetamidophenol, 0.87 g bromopropyne and 1.51 g potassium carbonate were dissolved in DMF (10 mL) and reacted at room temperature for 8 h. TLC detected that the reaction was complete, filtered, concentrated the filtrate, and purified the crude product by column chromatography to obtain 0.70 g of colorless and transparent oil A27-1.

Step 2: Preparation of Compound A27-2

200 mg of compound M1, 107.0 mg of compound A27-1, 8.5 mg of $PdCl_2(PPh_3)_2$, 6.4 mg of $PPh_3$, 4.8 mg of CuI were dissolved in DMF (4 mL) and $Et_3N$ (1 mL), and the reaction was performed at 60 °C after nitrogen replacement 14h. LC-MS and TLC detected that the reaction was complete, 10 mL of ethyl acetate was added, insolubles were filtered off, the filtrate was dispersed in 20 mL of water, the organic phase was washed with saturated brine, dried over anhydrous sodium sulfate, and concentrated in vacuo. The crude product was purified by preparative plate to obtain 170 mg pale yellow solid, namely compound A27-2.

Step 3: Preparation of Compound A27

160 mg of compound A27-2 was dissolved in 6.0 mL of dioxane and 1.5 mL of MeOH, 1N HCl (0.43 mL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with EA (5×3 mL), the organic phase was dried and concentrated in vacuo, and purified by preparative plate to obtain 69.4 mg of off-white solid compound A27.
[M+H+]=484.36.
1H NMR (500 MHz, DMSO) δ 8.21 (s, 1H), 7.97 (br, 1H), 7.54-7.49 (m, 2H), 7.42-7.35 (m, 2H), 7.30 (d, J = 6.5 Hz, 1H), 7.20-7.12 (m, 4H), 5.43 (t, J = 5.8 Hz, 1H), 5.13 (s, 2H), 4.46 (d, J = 5.4 Hz, 2H), 3.88-3.75 (m, 3H) ), 3.18-3.09 (m, 2H), 3.03 (d, J = 15.6 Hz, 1H), 2.60 (d, J = 15.6 Hz, 1H), 1.89-1.83 (m, 1H), 1.79-1.72 (m, 1H), 1.52-1.49 (m, 1H), 1.12-1.11 (m, 1H).

Example 32 Preparation of Compound A32 (S)-4-((3-(5-(1-amino-1,3-dihydrospiro[indene-2,4'-piperidin]-1'-yl)-6-(hydroxymethy l)pyrazin-2-yl)prop-2-yn-1-yl)oxy)benzenesulfonamide

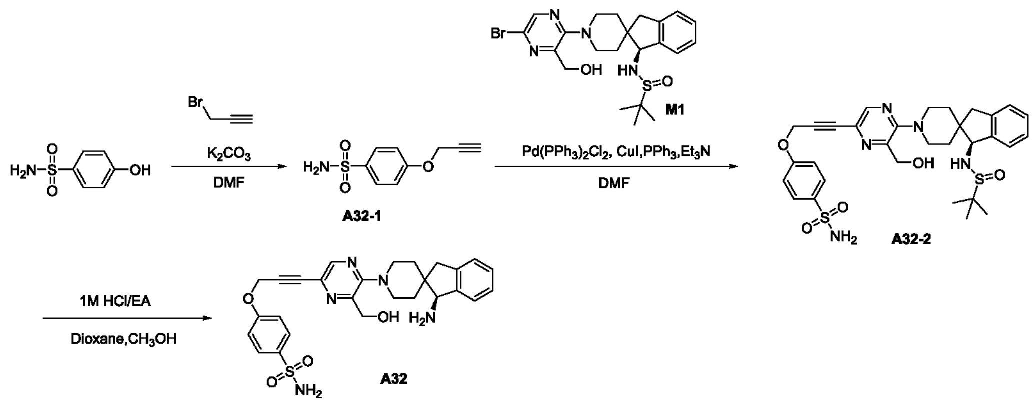


Step 1: Preparation of Compound A32-1

Under nitrogen protection, 1.00 g of p-benzenesulfonamide Aminophenol, 0.68 g of bromopropyne and 0.96 g of potassium carbonate were dissolved in DMF (10 mL) and reacted at room temperature for 4 h. TLC detected that the reaction was complete, filtered, concentrated the filtrate, and purified the crude product by column chromatography to obtain 0.80 g of colorless and transparent oil A32-1.

Step 2: Preparation of Compound A32-2

200 mg of compound M1, 171.0 mg of compound A32-1, 8.5 mg of $PdCl_2(PPh_3)_2$, 6.4 mg of $PPh_3$, 4.8 mg of CuI were dissolved in DMF (4 mL) and $Et_3N$ (1 mL), and the reaction was carried out at 60 °C after nitrogen replacement 14h. LC-MS and TLC detected that the reaction was complete, 10 mL of ethyl acetate was added, insolubles were filtered off, the filtrate was dispersed in 20 mL of water, the organic phase was washed with saturated brine, dried over anhydrous sodium sulfate, and concentrated in vacuo. The crude product was purified by preparative plate to obtain 120 mg pale yellow solid, namely compound A32-2.

Step 3: Preparation of Compound A32

120 mg of compound A32-2 was dissolved in 4.0 mL of dioxane and 1.0 mL of MeOH, 1N HCl (0.38 mL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with EA (5×3 mL), the organic phase was dried and concentrated in vacuo, and purified by preparative plate to obtain 44.3 mg of off-white solid compound A32.

[M+H+]=520.32.

1H NMR (500 MHz, DMSO) δ 8.22 (s, 1H), 7.79 (d, J = 8.8 Hz, 2H), 7.30 (d, J = 6.5 Hz, 1H), 7.24-7.14 (m, 7H), 5.42 (t, J = 5.9 Hz, 1H), 5.19 (s, 2H), 4.46 (d, J = 5.8 Hz, 2H), 3.84-3.76 (m, 3H), 3.17-3.11 (m, 2H), 3.03 (d, J = 15.6 Hz, 1H), 2.62 (d, J = 15.5 Hz, 1H), 1.89-1.85 (m, 1H), 1.78-1.72 (m, 1H), 1.52-1.49 (m, 1H), 1.12 -1.10 (m, 1H).

Example 33 Preparation of Compound A33 (S)-3-((3-(2-(1-amino-1,3-dihydrospiro[indene-2,4'-piperidine]-1'-yl)-6-oxo-1,6-dihyd ropyrimidin-5-yl)prop-2-yn-1-yl) oxy)benzamide)

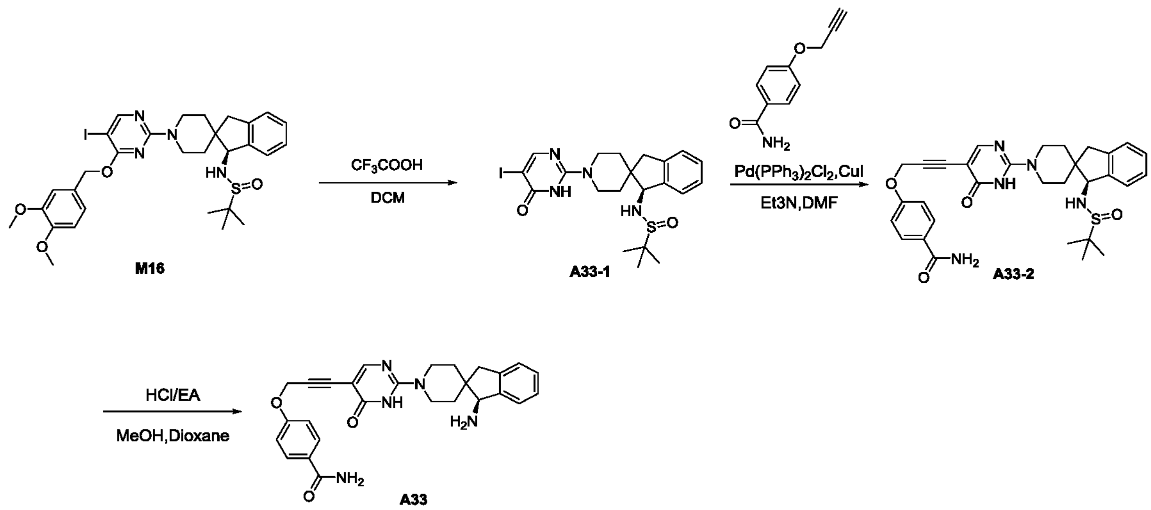


Step 1: Preparation of Compound A33-1

400 mg of compound M16 was dissolved in DCM (4.0 mL), after nitrogen replacement, 675 mg of $CF_3COOH$ was added, and the reaction was stirred at room temperature for 3 h. LC-MS and TLC detected the reaction was complete, and 10 mL of saturated sodium bicarbonate was added to adjust the pH to 8 ~9, extracted with ethyl acetate (3×20 mL), washed with saturated brine, dried over anhydrous sodium sulfate, concentrated in vacuo, and the crude product was purified by column chromatography to obtain 270 mg of white solid compound A33-1.

Step 2: Preparation of Compound A33-2

200 mg of compound A33-1, 99.8 mg of compound 4-(prop-2-yn-1-yloxo)benzamide, 8.0 mg of $PdCl_2(PPh_3)_2$, 4.4 mg of CuI, 6.0 mg of $PPh_3$ were dissolved in DMF (4.0 mL) and $Et_3N$ (1.0 mL), after nitrogen replacement, the reaction was stirred at room temperature for 10 min, and stirred at 50 °C for 15 h under nitrogen protection. LC-MS and TLC detected that the reaction was complete, quenched by adding 20 mL of water, ethyl acetate (3×20 mL) extraction, washed with saturated brine, dried over anhydrous sodium sulfate, concentrated in vacuo, and the crude product was purified by preparative plate to obtain 12 mg of compound A33-2 as a pale yellow solid.

Step 3: Preparation of Compound A33

12 mg of compound A33-2 was dissolved in 2.0 mL of dioxane, 0.5 mL of MeOH was added, 0.02 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 4.5 mg of light yellow solid compound A33 .
[M+H+]=470.19.

Example 41 Preparation of Compound A41 (S)-6-(3-(3-acetylaminophenoxy) prop-1-yn-1-yl)-3-(1-amino-1,3-dihydrospiro[indene-2,4]'-piperidin]-1'-yl)pyrazine-2-formamide

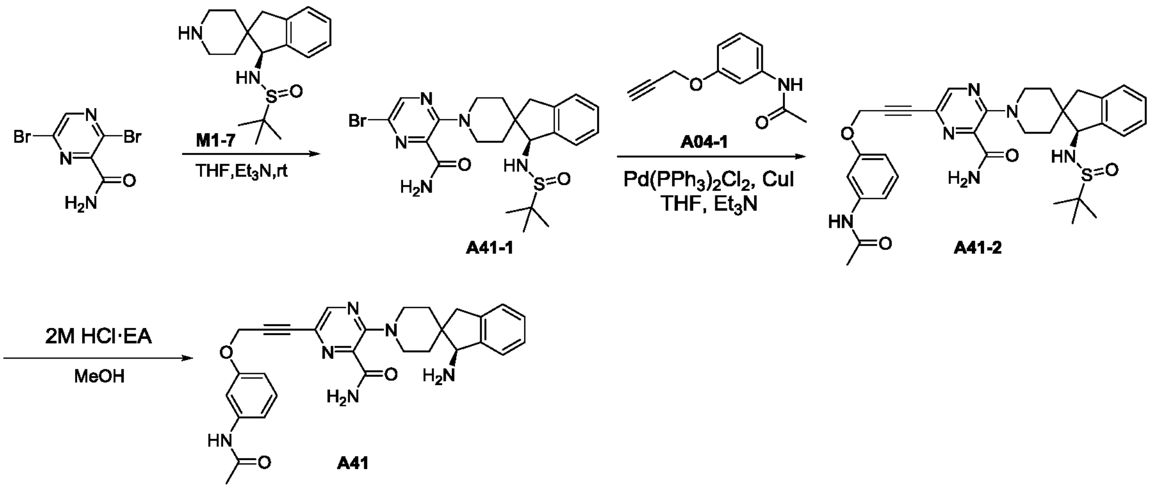


Step 1: Preparation of Compound A41-1

200 mg of 3,6-dibromopyrazine-2-carboxamide, 248 mg of M1-7 and 110 mg of triethylamine were dissolved in THF (5 mL) and reacted at room temperature for 3 hours. TLC detected the completion of the reaction, added water (10 mL), extracted with ethyl acetate (3 × 10 mL), combined the organic phases, washed successively with saturated brine, dried over anhydrous sodium sulfate, filtered, concentrated the filtrate, and purified the crude product by column chromatography 220 mg of yellow solid were obtained, namely compound A41-1.

Step 2: Preparation of Compound A41-2

223 mg of compound A41-1, 100 mg of compound A04-1, 15.5 mg of $PdCl_2(PPh_3)_2$, 4.2 mg of CuI were dissolved in DMF (2 mL) and $NEt_3$ (0.4 mL), and the reaction was performed at 60 °C for 18 hours after nitrogen replacement. LC-MS and TLC detected the completion of the reaction, added 20 mL of ethyl acetate, dispersed in 15 mL of water, washed the organic phase with saturated brine, dried over anhydrous sodium sulfate, and concentrated in vacuo. The crude product was purified by column chromatography to obtain 150 mg of pale yellow solid. Namely compound A41-2.

Step 3: Preparation of Compound A41

150 mg of compound A41-2 was dissolved in 2 mL of methanol, 2N HCl (200 μL, ethyl acetate solution) was added, and the reaction was stirred at RT for 0.5 h. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 80.0 mg of pale yellow solid compound A41.
[M+H+]=511.3.
1H NMR (500 MHz, DMSO-d6) δ 9.95 (s, 1H), 8.26 (s, 1H), 8.04 (s, 1H), 7.61 (s, 1H), 7.38-7.37 (m, 1H), 7.30 ( d, J = 6.5 Hz, 1H), 7.24-7.21 (m, 1H), 7.16 - 7.14 (m, 4H), 6.73-6.71 (m, 1H), 5.76 (s, 1H), 5.02 (s, 2H) , 3.98 - 3.93 (m, 2H), 3.81 (s, 1H), 3.25 - 3.17 (m, 2H), 3.04 (d, J = 15.5 Hz, 1H), 2.61 (d, J = 15.5 Hz, 1H), 2.03 (s, 3H), 1.78-1.73 (m, 1H), 1.69-1.64 (m, 1H), 1.49 - 1.47 (m, 1H), 1.10 - 1.07 (m, 1H).

Example 45 Preparation of Compound A45 (S)-N-(3-(5-(1-amino-1,3-dihydrospiro[indene-2,4'-piperidin]-1'-yl)-6-(hydroxymethy 1))pyrazin-2-yl)prop-2-yn-1-yl)benzamide

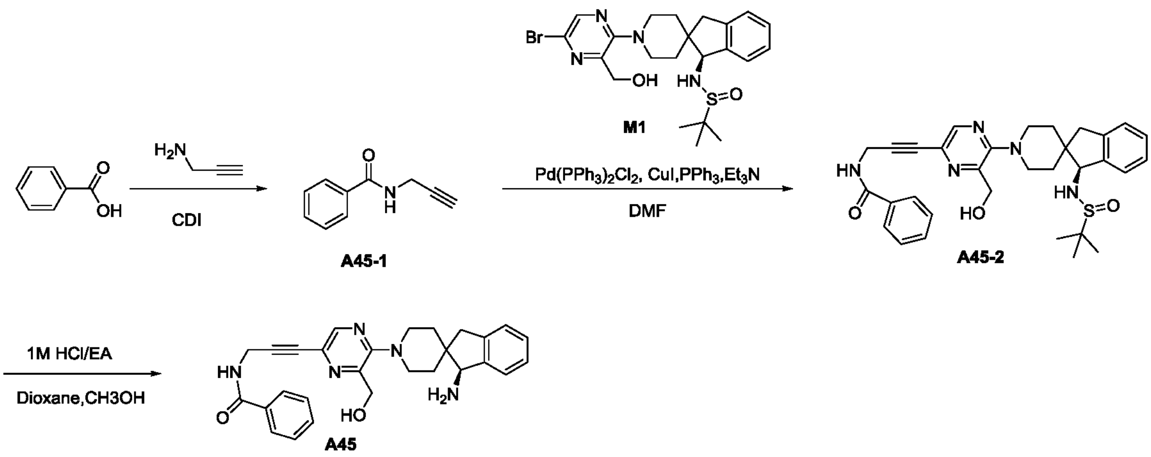


Step 1: Preparation of Compound A45-1

Under nitrogen protection, 1.00 g of benzoic acid was dissolved in 15 mL of DCM, 1.46 g of carbonyldiimidazole was added, and the reaction was carried out at RT for 30 minutes, and then 496 mg of propargylamine was added, and the reaction was carried out at room temperature overnight. TLC detected that the reaction was complete, water was added, extracted with DCM, the organic phase was washed with 10% citric acid, then washed with 10% sodium bicarbonate, dried and filtered over anhydrous sodium sulfate, the filtrate was concentrated, and the crude product was slurried with n-hexane to obtain 960 mg of white solid, namely Compound A45-1.

Step 2: Preparation of Compound A45-2

150 mg of compound M1, 72 mg of compound A45-1, 10 mg of $PdCl_2(PPh_3)_2$, 2.9 mg of CuI were dissolved in DMF (3 mL) and $Et_3N$ (0.5 mL), and after nitrogen replacement, the reaction was carried out at 60 °C for 4 h. LC-MS and TLC detected that the reaction was complete, the reaction solution was spun to remove triethylamine, cooled to room temperature, added 20 mL of water to precipitate a solid, filtered, the filter cake was washed with water, the filter cake was dissolved in DCM, dried over anhydrous sodium sulfate, and directly applied to the column. Flash preparation and purification gave 120 mg of pale yellow solid, namely compound A45-2.

Step 3: Preparation of Compound A45

120 mg of compound A45-2 was dissolved in 4.0 mL of dioxane and 2.0 mL of MeOH, 2N HCl (0.32 mL, ethyl acetate solution) was added, and the reaction was stirred at RT for 0.5 h. LC-MS and TLC detected that the reaction was complete, the reaction solution was neutralized by adding ammonia water, and then concentrated under reduced pressure. The residue was purified by preparative plate to give 8.3 mg of off-white solid compound A45.

[M+H+]=468.37.

1H NMR (500 MHz, DMSO-d6) δ 9.08 (t, J = 5.6 Hz, 1H), 8.20 (s, 1H), 7.92-7.86 (m, 2H), 7.57-7.54 (m, 1H), 7.50- 7.47 (m, 2H), 7.36-7.30 (m, 1H), 7.21-7.15 (m, 3H), 4.47 (s, 2H), 4.35 (d, J = 5.5 Hz, 2H), 3.92 (s, 1H) , 3.81-3.71 (m, 2H), 3.17-3.09 (m, 2H), 3.03 (d, J = 16.0 Hz, 1H), 2.67 (d, J = 15.5 Hz, 1H), 1.89-1.83 (m, 1H) ), 1.79-1.74 (m, 1H), 1.52-1.49 (m, 1H), 1.18-1.16 (m, 1H).

Example 46 Preparation of Compound A46 (S)-(3-(1-amino-1,3-dihydros piro[indene-2,4'-piperidin]-1'-yl)-6-(4-(phenylamino)butyl-1-yn-1-yl)pyrazin-2-yl)met hanol

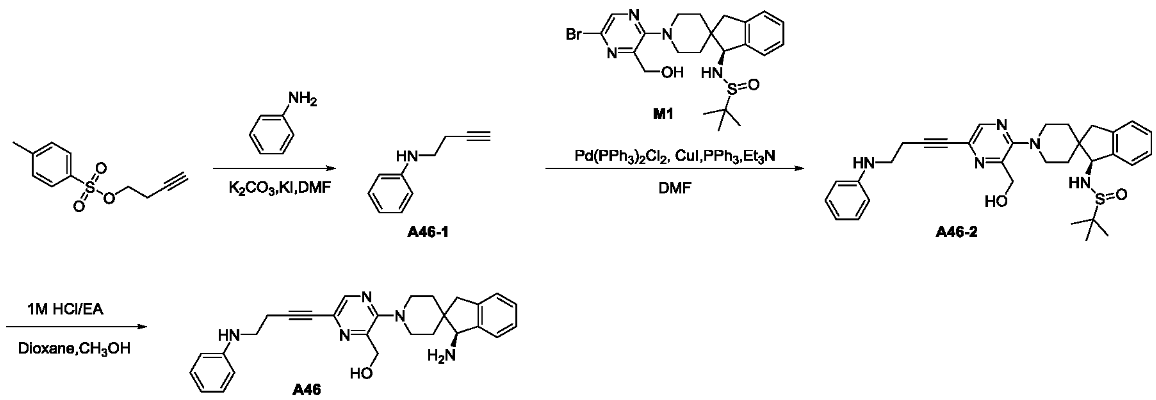


Step 1: Preparation of Compound A46-1

Under nitrogen protection, 1.00 g of 3-butynyl p-toluenesulfonate and 0.62 g of aniline were dissolved in 10 mL of DMF, 1.38 g of potassium carbonate and 0.74 g of potassium iodide were added, and the reaction was heated at 90 degrees for 4 hours. TLC detected the completion of the reaction, cooled to room temperature, quenched the reaction with ammonium chloride, extracted with EA (30 mL*3), combined the organic phases, washed with saturated brine (10 mL*4), dried over anhydrous sodium sulfate and filtered, and the filtrate was concentrated, the crude product was purified by column chromatography to obtain 285 mg of oily substance, namely compound A46-1.

Step 2: Preparation of Compound A46-2

150 mg of compound M1, 66 mg of compound A46-1, 10 mg of $PdCl_2(PPh_3)_2$, 2.9 mg of CuI were dissolved in DMF (4 mL) and $Et_3N$ (0.5 mL), and after nitrogen replacement, the reaction was performed at 60 °C overnight. LC-MS and TLC detected that the reaction was complete, 10 mL of ethyl acetate was added, the insolubles were filtered off, the filtrate was dispersed in 20 mL of water, the organic phase was washed with saturated brine, dried over anhydrous sodium sulfate, and concentrated in vacuo. The crude product was purified by preparative plate to obtain 32 mg pale yellow solid, namely compound A46-2.

Step 3: Preparation of Compound A46

32 mg of compound A46-2 was dissolved in 2.0 mL of dioxane and 1.0 mL of MeOH, 2N HCl (0.05 mL, ethyl acetate solution) was added, and the reaction was stirred at RT for 0.5 h. LC-MS and TLC detected that the reaction was complete, the reaction solution was neutralized by adding ammonia water, and then concentrated under reduced pressure. The residue was purified by preparative plate to give 8.3 mg of off-white solid compound A46.

[M+H+]=484.38.

Example 47 Preparation of Compound A47 (S)-(3-(1-amino-1,3-dihydrospiro [indene-2,4'-piperidin]-1'-yl)-6-(3-((3,4-dimethoxyphenyl)amino)prop-1-yn-1-yl)pyra zin-2-yl)

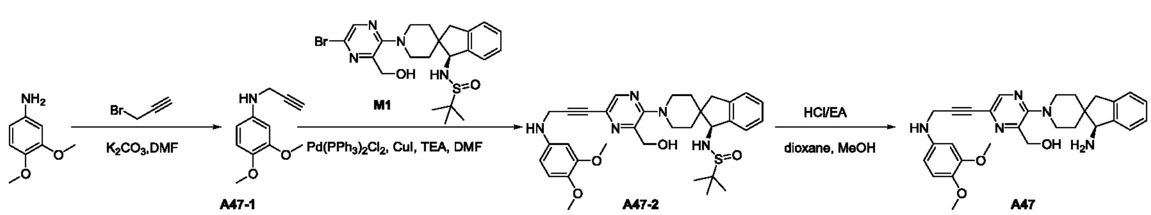


Step 1: Preparation of Compound A47-1

1.00 g of 3,4-dimethoxyaniline was dissolved in DMF (10.0 mL), 1.35 g of $K_2CO_3$ was added and stirred at room temperature for 10 min, 0.76 mL of bromopropyne was added dropwise, and after nitrogen replacement, the reaction was stirred at room temperature for 1 h, LC-MS and TLC detected the completion of the reaction, added 40 mL

of water, extracted with ethyl acetate (3×40 mL), washed with saturated brine, dried over anhydrous sodium sulfate, concentrated in vacuo, the crude product was purified by column chromatography to obtain 430 mg of white solid, Namely compound A47-1.

Step 2: Preparation of Compound A47-2

150 mg of compound M1, 21 mg of $PdCl_2(PPh_3)_2$, 3 mg of CuI, and 87 mg of compound A47-1 were dissolved in DMF (20.0 mL) and $Et_3N$ (0.75 mL). After nitrogen replacement, the reaction was carried out at 60 °C for 3 hours. LC-MS and TLC detected that the reaction was complete, 20 mL of ethyl acetate was added, the filter residue was filtered off, the filtrate was dispersed in 20 mL of water, the organic phase was washed with saturated brine, dried over anhydrous sodium sulfate, and concentrated in vacuo. The crude product was purified by column chromatography to obtain 148 mg pale yellow solid, namely compound A47-2.

Step 3: Preparation of Compound A47

90 mg of compound A47-2 was dissolved in 6.0 mL of dioxane, 1.5 mL of MeOH was added, 0.2 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 6.8 mg of yellow solid compound A47.
[M+H+]=500.35.
1H NMR (500 MHz, DMSO) δ 8.14 (d, J = 2.0 Hz, 1H), 7.31 (s, 1H), 7.17 (d, J = 5.6 Hz, 3H), 6.77 (d, J = 6.4 Hz, 1H) ), 6.40 (s, 1H), 6.23-6.13 (m, 1H), 5.71 (s, 1H), 5.39 (s, 1H), 4.47-4.43 (m, 2H), 4.14-4.09 (m, 2H), 3.88-3.85 (m, 1H), 3.79-3.73 (m, 1H), 3.72-3.68 (m, 3H), 3.65-3.62 (m, 3H), 3.19-3.06 (m, 2H), 3.03 (d, J = 15.6 Hz, 1H), 2.60 (d, J = 15.6 Hz, 1H), 1.89-1.82 (m, 1H), 1.79-1.72 (m, 1H), 1.54-1.48 (m, 1H), 1.13-1.07 (m, 1H).

Example 48 Preparation of Compound A48 (S)-4-((3-(5-(1-amino-1,3-dihydrospiro[indene-2,4'-piperidin]-1'-yl)-6-(hydroxymethy l)pyrazin-2-yl)prop-2-yn-1-yl)amino)benzamide

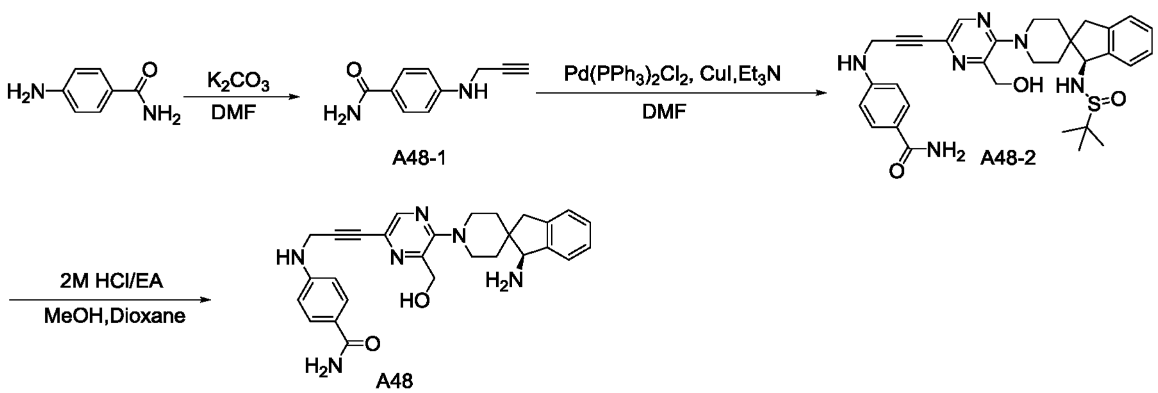


Step 1: Preparation of Compound A48-1

1.00 g of p-Aminobenzamide, 0.87 g of bromopropyne and 1.20 g of potassium carbonate were dissolved in DMF (10 mL) and reacted at room temperature for 12 hours. TLC detected that the reaction no longer proceeded, 50 mL of water was added, 50 mL of ethyl acetate was added for extraction, the organic phase was washed with saturated brine, dried over anhydrous sodium sulfate, and concentrated in vacuo. The crude product was purified by column chromatography to obtain 0.31 g of an off-white solid, which is the compound A48-1.

Step 2: Preparation of Compound A48-2

200 mg of compound M1, 150 mg of A48-1, 9.5 mg of $PdCl_2(PPh_3)_2$ and 3.6 mg of CuI were dissolved in DMF (5 mL) and triethylamine (1 mL), and then reacted at 60°C for 2 hours after nitrogen replacement. LC-MS and TLC detected that the reaction was complete, quenched by adding 10 mL of water, extracted with ethyl acetate (3×20 mL),

washed with saturated brine, dried over anhydrous sodium sulfate, and concentrated in vacuo. The crude product was purified by preparative plate to obtain 60 mg of yellow solid , namely compound A48-2.

Step 3: Preparation of Compound A48

60 mg of compound A48-2 was dissolved in dioxane (4 mL) and methanol (1 mL), 2N HCl (0.3 mL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 43 mg of pale yellow solid, namely Compound A48.

[M+H+]=483.37.

1H NMR (500 MHz, DMSO-d6) δ 8.15 (s, 1H), 7.71 - 7.67 (m, 2H), 7.59 (s, 1H), 7.31-7.29 (m, 1H), 7.19 -7.11 (m, 3H) ), 6.91 (s, 1H), 6.69 - 6.63 (m, 3H), 5.40 (t, J = 6.0 Hz, 1H), 4.44 (d, J = 6.0 Hz, 2H), 4.22 (d, J = 6.0 Hz, 2H), 3.85 (s, 1H), 3.78-3.71 (m, 2H), 3.14-3.05 (m, 1H), 3.02 (d, J = 15.5 Hz, 1H), 2.61 (d, J = 15.5 Hz, 1H), 1.89-1.82 (m, 1H), 1.79- 1.71 (m, 1H), 1.51-1.49 (m, 1H), 1.12-1.10 (m, 1H).

Example 49 Preparation of Compound A49 (S)-(3-(1-amino-1,3-dihydrospiro[indene-2,4'-piperidin]-1'-yl)-6-(3-((2-methoxypyridi ne-4-yl)oxy)prop-1-yn-1-yl)pyrazin-2-yl)methanol

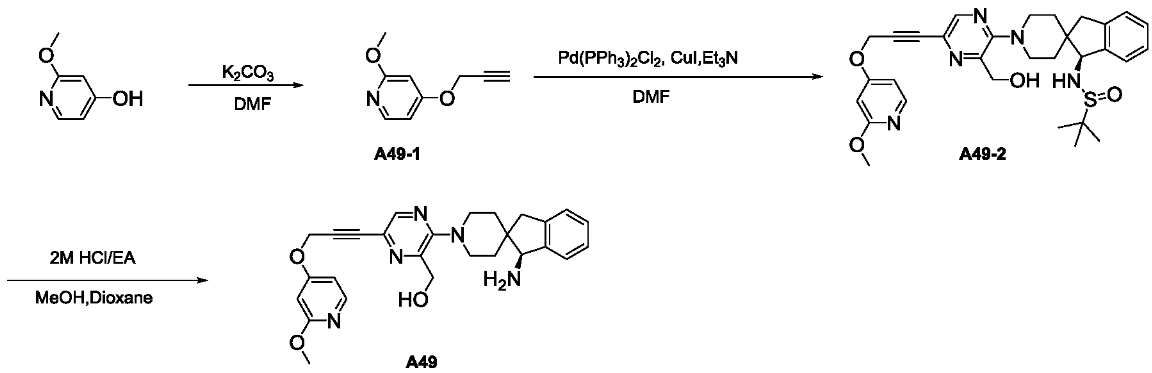


Step 1: Preparation of Compound A49-1

1.00 g of 2-methoxy-4-pyridinol, 0.95 g of bromopropyne and 1.1 g of potassium carbonate were dissolved in DMF (10 mL) and reacted at room temperature for 12 hours. TLC detected the completion of the reaction, added 50 mL of ethyl acetate, dispersed in 50 mL of water, washed the organic phase with saturated brine, dried over anhydrous sodium sulfate, and concentrated in vacuo. The crude product was purified by column chromatography to obtain 0.78 g of an off-white solid, namely compound A49- 1.

Step 2: Preparation of Compound A49-2

200 mg of compound M1, 150 mg of A49-1, 9.5 mg of $PdCl_2(PPh_3)_2$ and 3.6 mg of CuI were dissolved in DMF (5 mL) and 1 mL of triethylamine, and after nitrogen replacement, the reaction was performed at 60°C for 2 hours. LC-MS and TLC detected that the reaction was complete, quenched by adding 10 mL of water, extracted with ethyl acetate (3×20 mL), washed with saturated brine, dried over anhydrous sodium sulfate, and concentrated in vacuo. The crude product was purified by preparative plate to obtain 88 mg of yellow Solid compound A49-2.

Step 3: Preparation of Compound A49

88 mg of compound A49-2 was dissolved in dioxane (4 mL) and methanol (1 mL), 2N HCl (0.3 mL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3 × 3 mL), the organic phase was dried and concentrated in vacuo, and purified by preparative plate to obtain 43 mg of pale yellow solid as

compound A49.

[M+H+]=472.30.

1H NMR (500 MHz, DMSO-d6) δ8.22 (s, 1H), 7.96 (d, J = 3.0 Hz, 1H), 7.50 (dd, J = 9.0, 3.0 Hz, 1H), 7.31 (d, J = 6.5 Hz, 1H), 7.21-7.13 (m, 3H), 6.81 (d, J = 9.0 Hz, 1H), 5.43 (t, J = 6.0 Hz, 1H), 5.09 (s, 2H), 4.47 (d , J = 6.0 Hz, 2H), 3.88 - 3.75 (m, 6H), 3.17 - 2.99 (m, 2H), 3.03 (d, J = 15.5 Hz, 1H), 2.62 (d, J = 15.5 Hz, 1H) , 1.89-1.82 (m, 1H), 1.78-1.74 (m, 1H), 1.53-1.50 (m, 1H), 1.15-1.12 (m, 1H).

Example 50 Preparation of Compound A50 (S)-4-((3-(5-(1-amino-1,3-dihydrospiro[indene-2,4'-piperidin]-1'-yl)-6-(hydroxymethy l)pyrazin-2-yl)prop-2-yn-1-yl)oxy)-2-fluorobenzamide

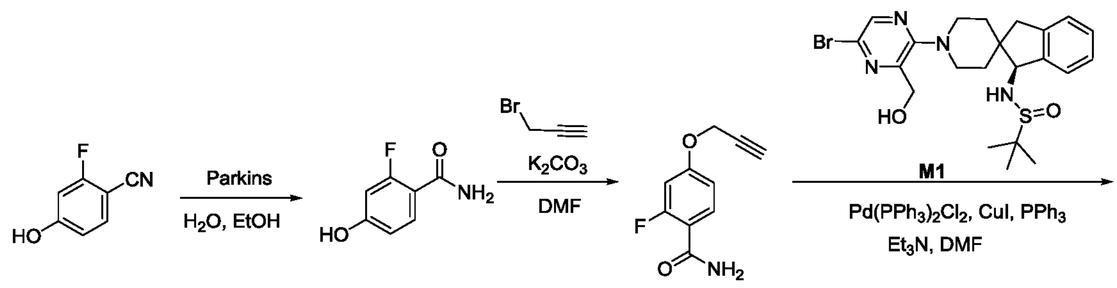


A50-1 A50-2

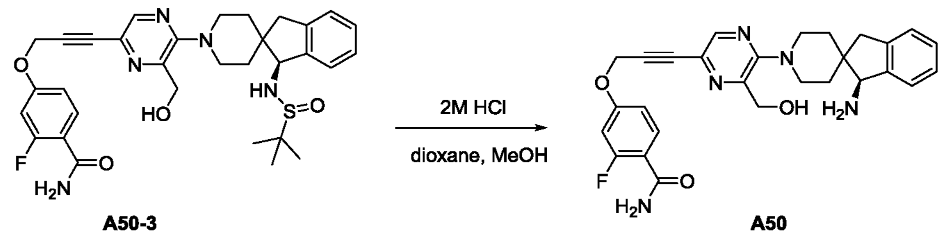


A50-3 A50

Step 1: Preparation of Compound A50-1

1.00 g of raw material 2-fluoro-4-hydroxybenzonitrile and 23 mg of Parkin's reagent were dissolved in 6 mL of ethanol and 2 mL of water, and reacted at 80 °C for 12 h. After TLC (PE:EA=1:1) monitoring showed that the reaction was complete, it was concentrated under reduced pressure and purified by column chromatography (DCM:MeOH=12:1) to obtain 512 mg of white powder A50-1.

Step 2: Preparation of Compound A50-2

512 mg of compound A50-1, 432 mg of 3-bromo-1-propyl, and 684 mg of potassium carbonate were dissolved in 10 mL of DMF, and reacted at room temperature for 2 h. After TLC (PE:EA=1:1) monitoring showed that the reaction was complete, 10 mL of water was added, extracted with 10 mL of EA, the organic phase was washed with 3×10 mL of saturated sodium chloride solution, the organic phase was separated, and dried over anhydrous sodium sulfate, concentrated under reduced pressure to obtain 488 mg of white powder of compound A50-2.

Step 3: Preparation of Compound A50-3

117 mg of compound A50-2, 200 mg of M1, 8.5 mg of $Pd(PPh_3)_2Cl_2$, and 4.6 mg of CuI were dissolved in 3 mL of DMF and 0.5 mL of triethylamine, replaced by N2, and reacted at 60 °C for 12 h. After TLC (DCM:MeOH=10:1) showed that the reaction of the raw materials was complete, 10 mL of ethyl acetate and 10 mL of water were added for extraction, and the organic phase was separated. It was dried, separated and purified by column chromatography (DCM:MeOH=24:1) to obtain 166 mg of pale yellow powder, namely compound A50-3.

Step 4: Preparation of Compound A50

166 mg of compound A50-3 was dissolved in 4 mL of dioxane and 1 mL of methanol solution, replaced with

nitrogen, and 0.27 mL of HCl in EA solution (2 M) was added dropwise with stirring, and stirred at room temperature for 30 min. LC-MS detected that the reaction was complete, the reaction solution was concentrated under reduced pressure, the residue was dissolved in 5 mL of water, and saturated sodium bicarbonate solution was added dropwise to pH 8~9, the precipitated solid was collected, and then separated and purified by a thick preparation plate to obtain 35.9 mg of light yellow powder, namely Compound A50.

[M+H+]=502.33.

1H NMR (500 MHz, DMSO-d6) δ 8.23 (s, 1H), 7.69 (t, J = 8.7 Hz, 1H), 7.50 (s, 2H), 7.34-7.27 (m, 1H), 7.19-7.15 ( m, 4.6 Hz, 3H), 7.00 (dd, J = 12.8, 2.5 Hz, 1H), 6.95 (dd, J = 8.7, 2.5 Hz, 1H), 5.43 (t, J = 5.9 Hz, 1H), 5.18 ( s, 2H), 4.47 (d, J = 5.3 Hz, 2H), 3.86 (s, 1H), 3.85-3.76 (m, 2H), 3.18-3.07 (m, 2H), 3.04 (d, J = 15.6 Hz , 1H), 2.62 (d, J = 15.8 Hz, 1H), 1.90-1.81 (m, 1H), 1.80-1.71 (m, 1H), 1.54-1.46 (m, 1H), 1.19-1.12 (m, 2H)).

Example 51 Preparation of Compound A51 (S)-5-((3-(5-(1-amino-1,3-dihydrospiro[indene-2,4'-piperidin]-1'-yl)-6-(hydroxymethy l)pyrazin-2-yl)prop-2-yn-1-yl)oxy)isoindolin-1-one

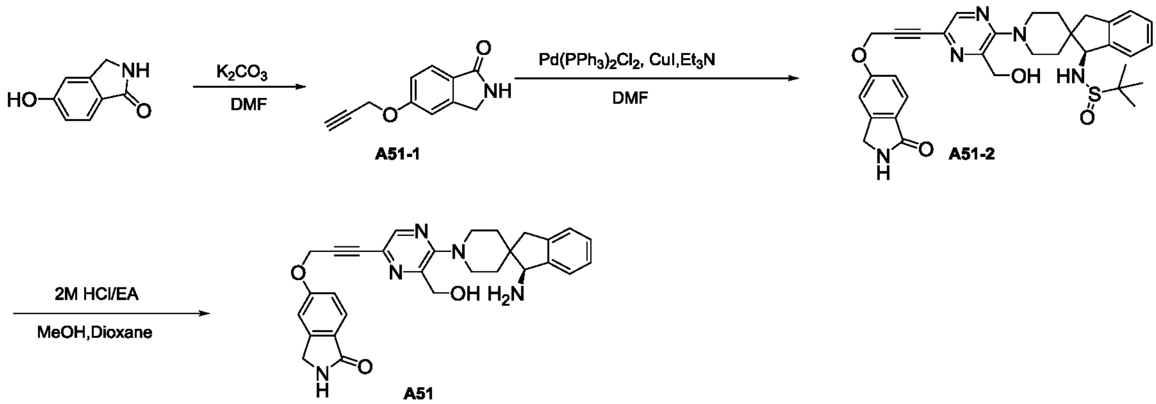


Step 1: Preparation of Compound A51-1

1.00 g of 5-hydroxy-isoindolin-1-one, 0.95 g of bromopropyne and 1.1 g of potassium carbonate were dissolved in DMF (10 mL) and reacted at room temperature for 12 hours. TLC detected that the reaction was complete, 50 mL of water was added, and 50 mL of ethyl acetate was added for extraction. The organic phase was washed with saturated brine, dried over anhydrous sodium sulfate, and concentrated in vacuo. The crude product was purified by column chromatography to obtain 0.50 g of an off-white solid, namely compound A51 -1.

Step 2: Preparation of Compound A51-2

300 mg of compound M1, 160 mg of A51-1, 9.5 mg of $PdCl_2(PPh_3)_2$ and 3.6 mg of CuI were dissolved in DMF (5 mL) and triethylamine (1 mL), and after nitrogen replacement, the reaction was carried out at 60 °C for 2 hours. LC-MS and TLC detected that the reaction was complete, quenched by adding 10 mL of water, extracted with ethyl acetate (3×20 mL), washed with saturated brine, dried over anhydrous sodium sulfate, and concentrated in vacuo. The crude product was purified by preparative plate to obtain 180 mg of yellow Solid, namely compound A51-2.

Step 3: Preparation of Compound A51

180 mg of compound A51-2 was dissolved in dioxane (4 mL) and methanol (1 mL), 2N HCl (0.3 mL, ethyl acetate solution) was added, and the reaction was stirred at RT for 1 hour. LC-MS and TLC detected the completion of the reaction, the reaction solution was concentrated under reduced pressure, the residue was dissolved in $H_2O$ (1.0 mL), the solution was adjusted to pH=8 with saturated $NaHCO_3$, extracted with dichloromethane (3×3 mL), the organic phase was dried and concentrated in vacuo, purified by preparative plate to obtain 53.3 mg of pale yellow solid, namely compound A51.

[M+H+]=496.33.

1H NMR (500 MHz, DMSO-d6) δ 8.42 (s, 1H), 8.29 (s, 1H), 7.67 (d, J = 8.5 Hz, 1H), 7.37-7.35 (m, 1H), 7.30-7.29 ( m, 1H), 7.24 - 7.17 (m, 4H), 5.48 (t, J = 6.0 Hz, 1H), 5.24 (s, 2H), 4.53 (d, J = 5.0 Hz, 2H), 4.40 (s, 2H) ), 3.90 (s, 1H), 3.88 - 3.82 (m, 2H), 3.24 - 3.13 (m, 2H), 3.08 (d, J = 15.5 Hz, 1H), 2.66 (d, J = 15.5 Hz, 1H) , 1.95-1.89 (m, 1H), 1.82-1.81

(m, 1H), 1.59-1.56 (m, 1H), 1.18-1.15 (m, 1H).

Example 52 Preparation of Compound A52 (S)-6-((3-(5-(1-amino-1,3-dihydrospiro[indene-2,4'-piperidin]-1'-yl)-6-(hydroxymethy l)pyrazin-2-yl)prop-2-yn-1-yl)oxy)-3,4-dihydroisoquinolin-1(2H)-one

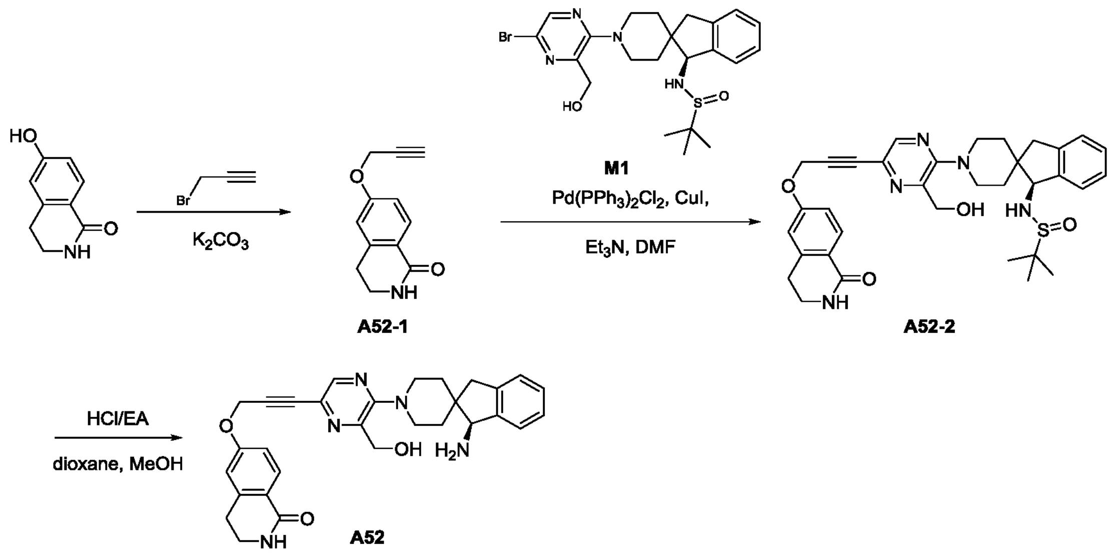


Step 1: Preparation of Compound A52-1

0.50g of 6-hydroxy-3,4-dihydroisoquinolin-1(2H)-one,0.437g of 3-bromopropyne and 0.847 g of potassium carbonate were dissolved in DMF (4 mL) andreacted at room temperature for 16 hours. TLC detected that the reaction was complete, cooled to room temperature, quenched by adding 20 mL of water, extracted with DCM (3×10 mL), washed with saturated brine, dried over anhydrous sodium sulfate, concentrated under reduced pressure, and the crude product was purified by column chromatography to obtain 570 mg of light yellow solid, Namely compound A52-1.

Step 2: Preparation of Compound A52-2

306 mg of compound A52-1, 600 mg of M1, 42 mg of $PdCl_2(PPh_3)_2$, and 11.6 mg of CuI were dissolved in DMF (4.0 mL) and $Et_3N$ (1.0 mL), and heated at 60 °C for 16 hours after nitrogen replacement. LC-MS and TLC detected that the reaction was complete, cooled to room temperature, quenched by adding 20 mL of water, extracted with ethyl acetate (3×20 mL), washed with saturated brine, dried over anhydrous sodium sulfate, concentrated in vacuo, and the crude product was subjected to column chromatography purified to obtain 700 mg of pale yellow solid, namely compound A52-2.

Step 3: Preparation of Compound A52

400 mg of compound A52-2 was dissolved in 4.0 mL of dioxane, 1 mL of MeOH was added, 0.49 mL of HCl in EA solution (2M) was added dropwise with stirring, and the mixture was stirred at room temperature for 1 hour. LC-MS detected the completion of the reaction, added saturated sodium bicarbonate dropwise to adjust the pH to 8~9, quenched by adding 20 mL of water, extracted with dichloromethane (3×10 mL), washed with saturated brine, dried over anhydrous sodium sulfate, concentrated in vacuo, and prepared with the plate was purified to obtain 95 mg of yellow solid, namely compound A52.

[M+H+]=510.37.

1H NMR (500 MHz, DMSO) δ 8.23 (s, 1H), 7.80 (d, J = 8.5 Hz, 1H), 7.76 (s, 1H), 7.30 (d, J = 6.2 Hz, 1H), 7.21 - 7.09 (m, 3H), 7.04 - 6.92 (m, 2H), 5.43 (t, J = 5.9 Hz, 1H), 5.15 (s, 2H), 4.47 (d, J = 5.9 Hz, 2H), 3.84 (s, 1H), 3.83 - 3.73 (m, 2H), 3.37 - 3.34 (m, 2H), 3.14 - 3.08 (m, 2H), 3.03 (d, J = 15.6 Hz, 1H), 2.88 (t, J = 6.5 Hz , 2H), 2.60 (d, J = 15.6 Hz, 1H), 1.91 - 1.81 (m, 1H), 1.80 - 1.69 (m, 1H), 1.55 - 1.44 (m, 1H), 1.17 - 1.05 (m, 1H) ).

Example 53 Preparation of Compound A53 (S)-3-((3-(5-(1-amino-1,3-dihydrospiro[indene-2,4'-piperidin]-1'-yl)-6-(hydroxymethy l)pyrazin-2-yl)prop-2-yn-1-yl)oxy)benzonitrile

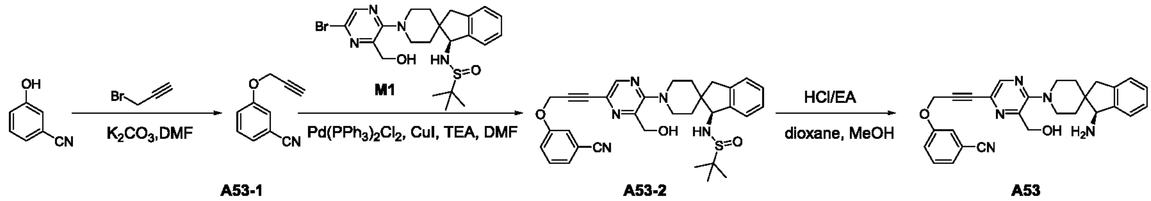


Step 1: Preparation of Compound A53-1

1.0 g of 3-cyanophenol was dissolved in DMF (10.0 mL), 1.74 g of $K_2CO_3$ was added, and the reaction was stirred at room temperature for 10 min. 0.98 mL of bromopropyne was added dropwise. After nitrogen replacement, the reaction was stirred at room temperature for 1 h. LC-MS and TLC After detecting that the reaction was complete, 40 mL of water was added, extracted with ethyl acetate (3×40 mL), washed with saturated brine, dried over anhydrous sodium sulfate, and concentrated in vacuo. The crude product was purified by column chromatography to obtain 0.92 g of white solid, namely compound A53- 1.

Step 2: Preparation of Compound A53-2

150 mg of compound M1, 21 mg of $PdCl_2(PPh_3)_2$, 3 mg of CuI, and 72 mg of compound A47-1 were dissolved in DMF (20.0 mL) and $Et_3N$ (0.75 mL), and after nitrogen replacement, the reaction was carried out at 60 °C for 3 hours. LC-MS and TLC detected that the reaction was complete, 20 mL of ethyl acetate was added, the filter residue was filtered off, the filtrate was dispersed in 20 mL of water, the organic phase was washed with saturated brine, dried over anhydrous sodium sulfate, and concentrated in vacuo. The crude product was purified by column chromatography to obtain 60 mg pale yellow solid, namely compound A53-2.

Step 3: Preparation of Compound A53

60 mg of compound A53-2 was dissolved in 6.0 mL of dioxane, 1.5 mL of MeOH was added, 0.2 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 20 mg of pale yellow solid, namely Compound A53.

[M+H+]=466.30.

1H NMR (500 MHz, CDCl3) δ 8.20 (s, 1H), 7.45-7.38 (m, 1H), 7.37-7.25 (m, 5H), 7.21 (s, 3H), 4.99 (s, 2H), 4.65 ( s, 2H), 4.00 (s, 1H), 3.66-3.52 (m, 2H), 3.25-3.12 (m, 2H), 3.11-3.02 (m, 1H), 2.77-2.64 (m, 1H), 1.97- 1.87 (m, 1H), 1.87-1.79 (m, 1H), 1.68-1.57 (m, 1H), 1.45-1.36 m, 1H), 1.22-1.16(m, 1H).

Example 54 Preparation of Compound A54 (S)-4-((3-(5-(1-amino-1,3-dihydrospiro[indene-2,4'-piperidin]-1'-yl)-6-(hydroxymethy l)pyrazin-2-yl)prop-2-yn-1-yl)oxy)-2-chlorobenzamide

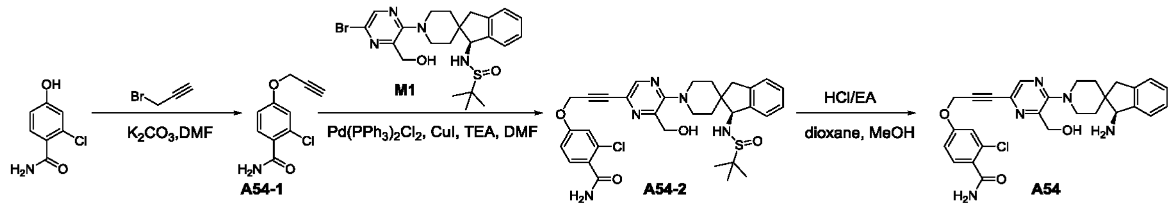

Step 1: Preparation of Compound A54-1

Dissolve 0.53 g of 2-chloro-4-hydroxybenzamide in DMF (10.0 mL), add 0.63 g of $K_2CO_3$ and stir at room temperature for 10 min, add dropwise 0.40 mL of bromopropyne, replace with nitrogen, and heat up to 60 °C to stir the reaction After 2.5 h, LC-MS and TLC detected the reaction was complete, 40 mL of water was added, extracted with ethyl acetate (3×40 mL), washed with saturated brine, dried over anhydrous sodium sulfate, and concentrated in vacuo. The crude product was purified by column chromatography to obtain 610 mg white solid, namely compound A54-1.

Step 2: Preparation of Compound A54-2

1.00 g of compound M1, 0.14 g of $PdCl_2(PPh_3)_2$, 20 mg of CuI, and 0.64 g of compound A54-1 were dissolved in DMF (20.0 mL) and $Et_3N$ (5.0 mL), and the reaction was carried out at 60 °C for 10 hours after nitrogen replacement. LC-MS and TLC detected that the reaction was complete, 50 mL of ethyl acetate was added, the filter residue was filtered off, the filtrate was dispersed in 50 mL of water, the organic phase was washed with saturated brine, dried over anhydrous sodium sulfate, and concentrated in vacuo. The crude product was purified by column chromatography to obtain 0.80 g pale yellow solid, namely compound A54-2.

Step 3: Preparation of Compound A54

100 mg of compound A54-2 was dissolved in 6.0 mL of dioxane, 1.5 mL of MeOH was added, 0.26 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 35 mg of yellow solid compound A54.

[M+H+]=518.26.

1H NMR (500 MHz, DMSO) δ 8.22 (s, 1H), 7.77 (s, 1H), 7.47 (d, J = 11.3 Hz, 1H), 7.44 (s, 1H), 7.30 (d, J = 6.5 Hz , 1H), 7.23 - 7.08 (m, 4H), 7.05 (dd, J = 8.6, 2.5 Hz, 1H), 5.43 (t, J = 5.9 Hz, 1H), 5.17 (s, 2H), 4.46 (d, J = 5.9 Hz, 2H), 3.83 (s, 1H), 3.82 - 3.74 (m, 2H), 3.19 - 3.06 (m, 2H), 3.03 (d, J = 15.6 Hz, 1H), 2.60 (d, J = 15.6 Hz, 1H), 1.89 - 1.82 (m, 1H), 1.79 - 1.72 (m, 1H), 1.54 - 1.48 (m, 1H), 1.13 - 1.07 (m, 1H).

Example 55 Preparation of Compound A55 (S)-4-((3-(5-(1-amino-1,3-dihydrospiro[indene-2,4'-piperidin]-1'-yl)-6-(hydroxymethy l)pyrazin-2-yl)prop-2-yn-1-yl)(methyl)amino)benzamide

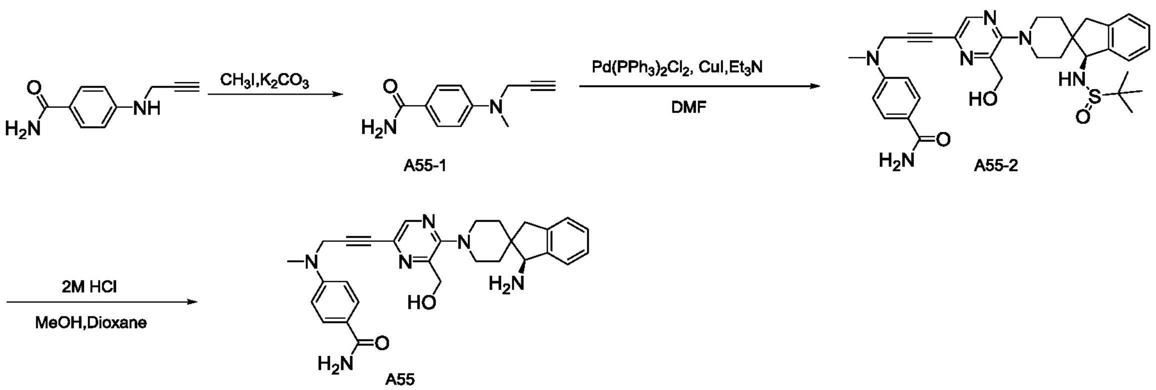

A55-1
A55-2
A55

Step 1: Preparation of Compound A55-1

0.18 g of 4-(prop-2-p-1-amino)benzamide, 0.17 g of methyl iodide and 0.21 g of potassium carbonate were dissolved in DMF (3 mL) and reacted at room temperature for 3 hours. TLC detected that the reaction was complete, 50 mL of water was added, 50 mL of ethyl acetate was added for extraction, the organic phase was washed with saturated brine, dried over anhydrous sodium sulfate, and concentrated in vacuo. The crude product was purified by column chromatography to obtain 0.12 g of a yellow solid, namely compound A55-1 .

Step 2: Preparation of Compound A55-2

120 mg of compound M1, 54.4 mg of A55-1, 8.5 mg of $PdCl_2(PPh_3)_2$ and 2.3 mg of CuI were dissolved in DMF (5 mL) and triethylamine (1 mL), and the reaction was performed at 60 °C for 2 hours after nitrogen replacement. LC-MS and TLC detected that the reaction was complete, quenched by adding 10 mL of water, extracted with ethyl acetate (3×20 mL), washed with saturated brine, dried over anhydrous sodium sulfate, and concentrated in vacuo. The crude product was purified by preparative plate to obtain 50 mg of yellow Solid compound A55-2.

Step 3: Preparation of Compound A55

50 mg of compound A55-2 was dissolved in dioxane (2 mL) and methanol (1 mL), 2N HCl (0.1 mL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), the solution was adjusted to pH=8 with saturated $NaHCO_3$, extracted with dichloromethane (3×3 mL), the organic phase was dried and concentrated in vacuo, and purified by preparative plate to obtain 34.2 mg of a pale yellow solid, namely compound A55 .

[M+H+]=497.37.

1H NMR (500 MHz, DMSO-d6) δ8.13 (s, 1H), 7.79 - 7.75 (m, 2H), 7.68 (s, 1H), 7.30-7.28 (m, 1H), 7.18-7.12 (m, 3H), 6.99 (s, 1H), 6.89-6.85 (m, 2H), 5.40 (t, J = 6.0 Hz, 1H), 4.50 (s, 2H), 4.43 (d, J = 6.0 Hz, 2H), 3.83 (s, 1H), 3.78-3.71 (m, 2H), 3.12 -2.97 (m, 6H), 2.65-2.56 (m, 1H), 1.90-1.82 (m, 1H), 1.78 - 1.72 (m, 1H) ), 1.54-1.48 (m, 1H), 1.11-1.08 (m, 1H).

Example 57 Preparation of Compound A57 (S)-(4-((3-(5-(1-amino-1,3-dihydrospiro[indene-2,4'-piperidin]-1'-yl)-6-(hydroxymeth yl))pyrazin-2-yl)prop-2-yn-1-yl)oxy)phenyl)dimethylphosphine oxide

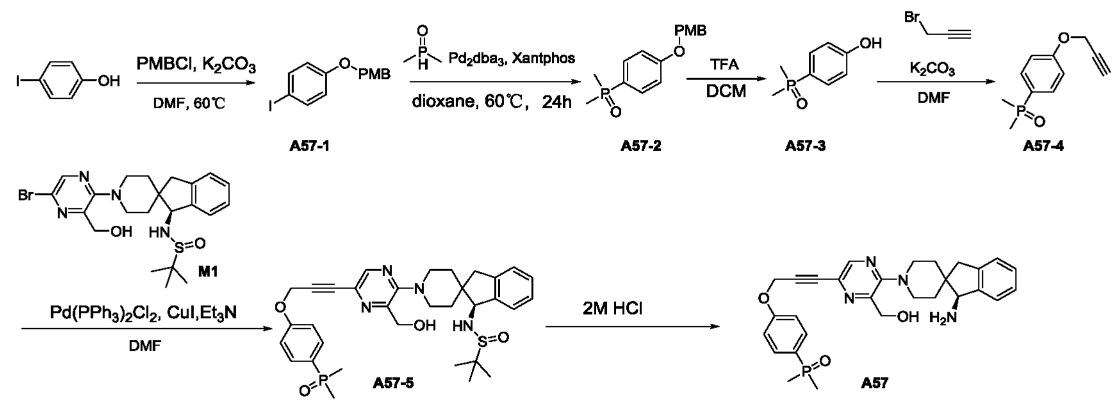


Step 1: Preparation of Compound A57-1

2.0 g of 4-iodophenol was dissolved in DMF (10.0 mL), 2.5 g of $K_2CO_3$ was added to stir the reaction for 3 min, 1.48 mL of PMBCl was added dropwise, and the reaction was stirred at 60 °C for 1.5 h. LC-MS and TLC detected the reaction was complete, and 40 mL of extracted with water, ethyl acetate (3×40 mL), washed with saturated brine, dried over anhydrous sodium sulfate, concentrated in vacuo, and the crude product was purified by column chromatography to obtain 2.38 g of white solid compound A57-1.

Step 2: Preparation of Compound A57-2

Dissolve 2.38 g of compound A57-1, 0.54 g of dimethyl phosphorus oxide in dioxane (10.0 mL), and stir under nitrogen protection for 10 min to obtain a raw material mixture. In addition, 0.13 g of $Pd_2dba_3$, 80 mg of Xantphos and 1.44 mL of tris Ethylamine was dissolved in dioxane (10.0 mL), and stirred under nitrogen protection for 10 min to obtain a catalyst mixture. The catalyst mixture solution was injected into the raw material mixture solution, and the temperature was raised to 60 °C and stirred for 24 h. After LC-MS and TLC were detected, the reaction was complete, added 40 mL of water, extracted with DCM (3×40 mL), washed with saturated brine, dried over anhydrous sodium sulfate, and concentrated in vacuo. The crude product was purified by column chromatography to obtain 1.96 g of off-white solid,

namely compound A57-2.

Step 3: Preparation of Compound A57-3

0.10 g of compound A57-3 was dissolved in DCM (5.0 mL), 0.3 mL of TFA was added dropwise in an ice bath, stirred at room temperature for 1 h, the reaction was complete by LC-MS and TLC, concentrated in vacuo, and the crude product was purified by column chromatography to obtain 0.55 g Light yellow oily liquid, namely compound A57-3.

Step 4: Preparation of Compound A57-4

0.60 g of compound A57-3 was dissolved in DMF (10.0 mL), 0.73 g of $K_2CO_3$ was added, and the reaction was stirred at room temperature for 10 min, and 0.41 mL of bromopropyne was added dropwise. TLC detected the completion of the reaction, added 40 mL of water, extracted with ethyl acetate (3×40 mL), washed with saturated brine, dried over anhydrous sodium sulfate, and concentrated in vacuo. The crude product was purified by column chromatography to obtain 0.57 g of a purple-red liquid, which is the compound A57-4.

Step 5: Preparation of Compound A57-5

200 mg of compound M1, 28 mg of $PdCl_2(PPh_3)_2$, 4 mg of CuI, and 126 mg of compound A47-1 were dissolved in DMF (20.0 mL) and $Et_3N$ (1.3 mL), and after nitrogen replacement, the reaction was carried out at 60 °C for 3 hours. LC-MS and TLC detected that the reaction was complete, 20 mL of ethyl acetate was added, the filter residue was filtered off, the filtrate was dispersed in 20 mL of water, the organic phase was washed with saturated brine, dried over anhydrous sodium sulfate, and concentrated in vacuo. The crude product was purified by column chromatography to obtain 0.17 g yellow solid, namely compound A57-5.

Step 6: Preparation of Compound A57

0.17 g of compound A57-5 was dissolved in 12.0 mL of dioxane, 3 mL of MeOH was added, 0.3 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 95 mg of pale yellow solid, namely Compound A57.

[M+H+]=517.35.

1H NMR (500 MHz, DMSO) δ 8.23 (s, 1H), 7.72 (dd, J = 11.0, 8.7 Hz, 2H), 7.30 (d, J = 6.9 Hz, 1H), 7.21-7.07 (m, 6H) , 5.43 (t, J = 5.9 Hz, 1H), 5.16 (s, 2H), 4.46 (d, J = 5.8 Hz, 2H), 3.79-3.73 (m, 1H), 3.72-3.68 (m, 2H), 3.18-3.06 (m, 2H), 3.03 (d, J = 15.6 Hz, 1H), 2.60 (d, J = 15.6 Hz, 1H), 1.89-1.82 (m, 1H), 1.79-1.72 (m, 1H) , 1.63 (s, 3H), 1.54-1.48 (m, 1H), 1.13-1.07 (m, 1H).

Example 58 Preparation of Compound A58 (S)-4-((3-(5-(1-amino-6-methoxy-1,3-dihydrospiro[indene-2,4'-piperidin]-1'-yl)-6-(hy droxymethyl)pyrazin-2-yl)prop-2-yn-1-yl)oxy)benzamide

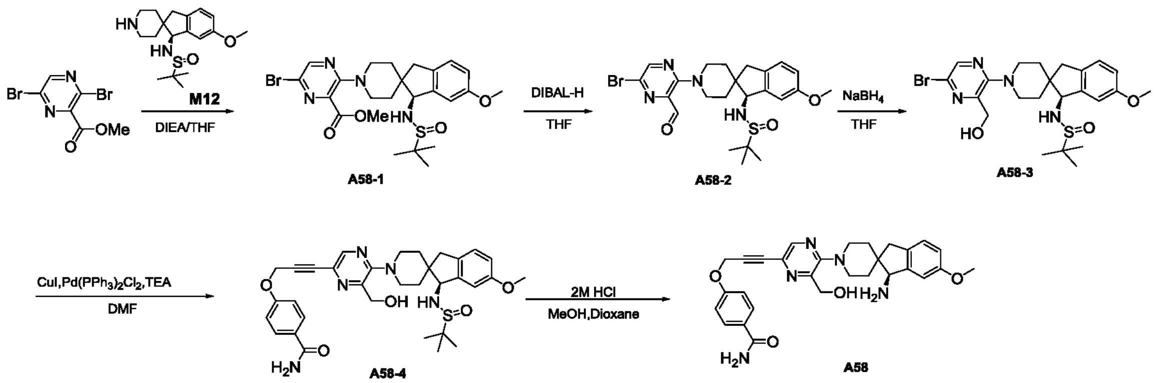

Step 1: Preparation of Compound A58-1

2.00 g of methyl 3,6-dibromopyrazine-2-carboxylate and 2.27 g of compound M12 were dissolved in 12 mL of tetrahydrofuran, 1.95 mL of DIEA was added dropwise, and the reaction was stirred at 60 °C. for 2 hours. TLC detected that the reaction was complete, concentrated under reduced pressure, added 50 mL of water, extracted with EtOAc (50 mL×3), combined the organic layers, dried over sodium sulfate, and desolvated, and purified by column chromatography to obtain 3.50 g, namely compound A58-1.

Step 2: Preparation of Compound A58-2

Under nitrogen protection, 3.00 g of compound A58-1 was dissolved in 40 mL of anhydrous dichloromethane, the temperature was lowered to -78 °C, a solution of DIBAL-H (1 M, 27 mL) in n-hexane was added dropwise, and the solution was stirred at -78 °C React for 1 hour. The temperature was slowly raised to -40°C, and the reaction was continued for 2 hours. TLC detected that the reaction of the raw materials was complete. At 0 °C, 1.08 mL of water was slowly added dropwise, then an aqueous sodium hydroxide solution (15%, 1.08 mL) was added dropwise, and 2.7 mL of water was added. Warm to room temperature and stir for 15 minutes. It was dried by adding sodium sulfate, stirred for ten minutes and filtered. The filtrate was concentrated and purified by column chromatography to obtain 2.80 g, namely compound A58-2

Step 3: Preparation of Compound A58-3

Under nitrogen protection, 2.80 g of compound A58-2 was dissolved in 20 mL of tetrahydrofuran, 0.41 g of sodium borohydride was added, and the reaction was carried out at room temperature for 0.5 hours. TLC detected that the reaction of the raw materials was complete. At 0 °C, 2 mL of saturated ammonium chloride was added to quench the reaction, 50 mL of water was added, and then extracted with EtOAc (50 mL × 3). The organic layers were combined, dried over sodium sulfate, and then desolvated. purified by column chromatography to obtain 2.30 g, namely compound A58-3.

Step 4: Preparation of Compound A58-4

300 mg of compound M1, 150 mg of 4-(prop-2-p-1-oxy)benzamide, 20.1 mg of $PdCl_2(PPh_3)_2$ and 5.5 mg of CuI were dissolved in DMF (10 mL) and triethylamine (1 mL), after nitrogen replacement, the reaction was carried out at 60 °C for 12 hours. LC-MS and TLC detected that the reaction was complete, quenched by adding 10 mL of water, extracted with ethyl acetate (3×20 mL), washed with saturated brine, dried over anhydrous sodium sulfate, concentrated in vacuo, and the crude product was purified by column chromatography to obtain 160 mg yellow solid, namely compound A58-4.

Step 5: Preparation of Compound A58

160 mg of compound A58-4 was dissolved in dioxane (3 mL) and methanol (1 mL), 2N HCl (2 mL, ethyl acetate solution) was added, and the reaction was stirred at RT for 1 hour. LC-MS and TLC detected the completion of the reaction, the reaction solution was concentrated under reduced pressure, the residue was dissolved in $H_2O$ (1.0 mL), the solution was adjusted to pH=8 with saturated $NaHCO_3$, extracted with dichloromethane (3×3 mL), the organic phase was dried and concentrated in vacuo, purified by preparative plate to obtain 81 mg of pale yellow solid compound A58.

[M+H+]=514.34.

1H NMR (500 MHz, DMSO-d6) δ 8.22 (s, 1H), 7.88-7.85 (m, 3H), 7.21 (s, 1H), 7.10-7.05 (m, 3H), 6.89 (d, J = 2.5 Hz, 1H), 6.69 (dd, J = 8.0, 2.5 Hz, 1H), 5.43 (t, J = 6.0 Hz, 1H), 5.15 (s, 2H), 4.46 (d, J = 6.0 Hz, 2H), 3.84-3.77 (m, 3H), 3.72 (s, 3H), 3.16-3.04 (m, 2H), 2.95 (d, J = 15.6 Hz, 1H), 2.52 (d, J = 15.6 Hz, 1H), 1.89 -1.83 (m, 1H), 1.77-1.70 (m, 1H), 1.54-1.49 (m, 1H), 1.11-1.08 (m, 1H).

Example 59 Preparation of Compound A59 (S)-2-(3-((3-(5-(1-amino-1,3-dihydrospiro[indene-2,4'-piperidin]-1'-yl)-6-(hydroxyme thyl)pyrazin-2-yl)prop-2-yn-1-yl)oxy)phenyl)acetamide

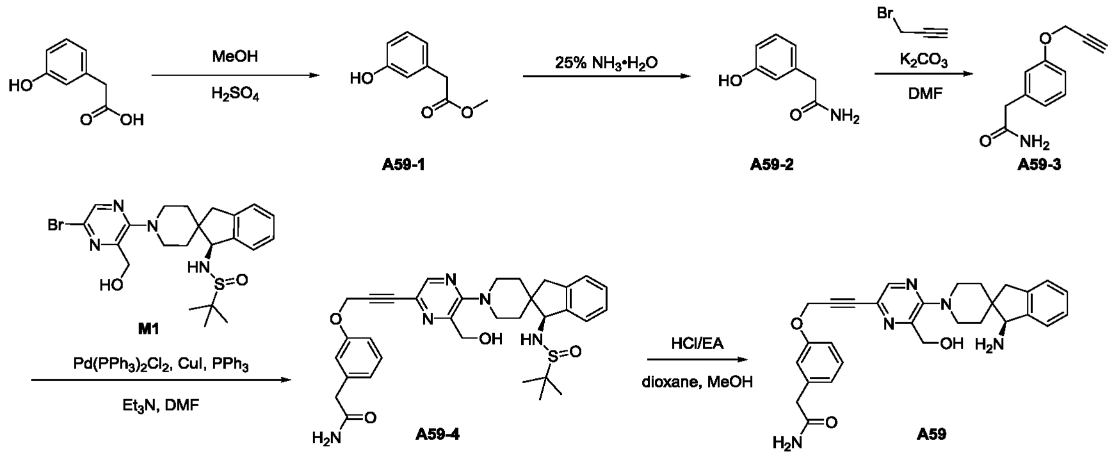

A59-1
A59-2
A59-3
M1
A59-4
A59

Step 1: Preparation of Compound A59-1

2.00 g m-hydroxyphenylacetic acid was dissolved in MeOH (30.0 mL), then concentrated sulfuric acid (0.1 mL) was slowly added dropwise, heated at 60 °C for 5 hours, TLC detected the reaction was complete, cooled to room temperature, the reaction solution was concentrated under reduced pressure, added 20 mL of dichloromethane, washed with saturated brine (3×10 mL), dried over anhydrous sodium sulfate, and concentrated under reduced pressure to obtain 2.15 g of an oily product, namely compound A59-1

Step 2: Preparation of Compound A59-2

2.15 g of compound A59-1 was dissolved in 25% ammonia water (11 mL) and reacted at room temperature for 6 hours. LC-MS detected that the reaction was complete, and concentrated under reduced pressure to obtain 2.22 g of a light yellow solid, namely compound A59-2.

Step 3: Preparation of Compound A59-3

1.00 g of compound A59-2, 0.866 g of 3-bromopropyne and 1.83 g of potassium carbonate were dissolved in DMF (8 mL) and reacted at 80 °C for 3 hours. The reaction was completed by TLC, cooled to room temperature, quenched by adding 20 mL of water, extracted with DCM (3×10 mL), washed with saturated brine, dried over anhydrous sodium sulfate, concentrated in vacuo, and the crude product was purified by column chromatography to obtain 135 mg of pale yellow solid, namely compound A59-3.

Step 4: Preparation of Compound A59-4

135 mg of compound A59-3, 234 mg of M1, 20 mg of $PdCl_2(PPh_3)_2$, 5.4 mg of CuI were dissolved in DMF (4.0 mL) and $Et_3N$ (1.0 mL), and the reaction was carried out at 60 °C for 16 hours after nitrogen replacement. LC-MS and TLC detected that the reaction was complete, cooled to room temperature, quenched by adding 20 mL of water, extracted with ethyl acetate (3×20 mL), washed with saturated brine, dried over anhydrous sodium sulfate, concentrated in vacuo, and the crude product was subjected to column chromatography purified to obtain 250 mg of pale yellow solid, namely compound A59-4.

Step 5: Preparation of Compound A59

250 mg of compound A59-4 was dissolved in 2.0 mL of dioxane, 0.5 mL of MeOH was added, 0.19 mL of HCl in EA solution (2M) was added dropwise with stirring, and the mixture was stirred at room temperature for 1 hour. LC-MS detected the completion of the reaction, added saturated sodium bicarbonate dropwise to adjust the pH to 8-9, quenched by adding 20 mL of water, extracted with dichloromethane (3×10 mL), washed with saturated brine, dried over anhydrous sodium sulfate, concentrated in vacuo, and prepared with plate purification yielded 33 mg of a yellow solid, compound A59.
[M+H+]=498.41.
1H NMR (500 MHz, DMSO) δ 8.23 (s, 1H), 7.44 (s, 1H), 7.30 (d, J = 6.6 Hz, 1H), 7.25 (t, J = 7.9 Hz, 1H), 7.20 - 7.11 (m, 3H), 6.96 - 6.83 (m, 4H), 5.44 (t, J = 6.0 Hz, 1H), 5.06 (s, 2H), 4.46 (d, J = 6.0 Hz, 2H), 3.83-3.76 (m, 2H),

3.35 (s, 2H), 3.18 - 2.97 (m, 3H), 2.61 (d, J = 15.6 Hz, 1H), 1.92 - 1.80 (m, 1H), 1.80 - 1.70 (m, 1H) , 1.56 - 1.44 (m, 1H), 1.16 - 1.07 (m, 1H).

Example 60 Preparation of Compound A60 (S)-(3-(1-amino-1,3-dihydrospiro[inden-2,4'-piperidin]-1'-yl)-6-(3-(oxazol-2-ylAmino ))prop-1-yn-1-yl)pyrazin-2-yl)methanol

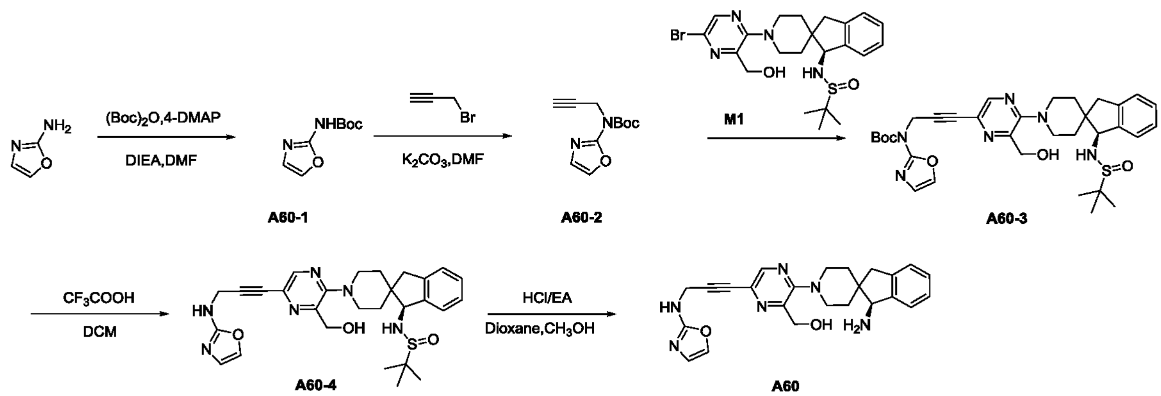


Step 1: Preparation of Compound A60-1

1.00 g of 2-Aminooxazole, 3.37 g of BOC acid anhydride and 0.15 g of 4-DMAP were dissolved in DMF (10.0 mL) and DMF (3.9 mL), and the reaction was stirred at room temperature for 4 h. LC-MS and TLC detected that the reaction was complete. 20 mL of water, extracted with ethyl acetate (3×20 mL), washed with saturated brine, dried over anhydrous sodium sulfate, concentrated in vacuo, and the crude product was purified by column chromatography to obtain 700 mg of white solid, namely compound A60-1.

Step 2: Preparation of Compound A60-2

Under nitrogen protection, 0.2 g of A60-1, 0.16 g of bromopropyne and 0.23 g of potassium carbonate were dissolved in DMF (4 mL) and reacted at 40 °C for 16 h. TLC detected that the reaction was complete, filtered, concentrated the filtrate, and purified the crude product by column chromatography to obtain 0.20 g of a yellow solid, namely compound A60-2.

Step 3: Preparation of Compound A60-3

160 mg of compound M1, 6.4 mg of $PdCl_2(PPh_3)_2$, 3.5 mg of CuI, and 108 mg of compound A60-2 were dissolved in DMF (4.0 mL) and $Et_3N$ (1.0 mL), and after nitrogen replacement, reacted at 60 °C for 2 h. LC-MS and TLC detected that the reaction was complete, 10 mL of ethyl acetate was added, the filter residue was filtered off, the filtrate was dispersed in 20 mL of water, the organic phase was washed with saturated brine, dried over anhydrous sodium sulfate, and concentrated in vacuo. The crude product was purified by preparative plate to obtain 190 mg of light yellow Solid, namely compound A60-3.

Step 4: Preparation of Compound A60-4

190 mg of compound A60-3 was dissolved in DCM (4.0 mL) and trifluoroacetic acid (0.5 mL) and stirred at room temperature for 2 h. LC-MS detected that the reaction was complete, the reaction solution was concentrated under reduced pressure, the residue was dissolved in 10 mL of water, and saturated sodium bicarbonate was added dropwise to adjust the pH to 8-9, solid was precipitated, filtered, and dried to obtain a solid, namely compound A60-4, which was directly proceed to the next reaction.

Step 5: Preparation of Compound A60

160 mg of compound A60-4 was dissolved in 6 mL of dioxane, 2 mL of MeOH was added, 0.30 mL of HCl in EA solution (2M) was added dropwise with stirring, and the mixture was stirred at room temperature for 30 min. LC-MS

detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 54.3 mg of a yellow solid, the compound A60.

[M+H+]=431.34.

1H NMR (500 MHz, DMSO) δ 8.17 (s, 1H), 7.67 (t, J = 6.1 Hz, 1H), 7.49 (d, J = 1.0 Hz, 1H), 7.30 (d, J = 7.1 Hz, 1H) ), 7.20 - 7.11 (m, 3H), 6.81 (d, J = 0.9 Hz, 1H), 5.42 (t, J = 6.0 Hz, 1H), 4.46 (d, J = 5.9 Hz, 2H), 4.23 (d , J = 6.1 Hz, 2H), 3.83 (s, 1H), 3.80-3.69 (m, 2H), 3.15 - 3.08 (m, 2H), 3.03 (d, J = 15.6 Hz, 1H), 2.59 (d, J = 15.6 Hz, 1H), 1.90 - 1.85 (m, 1H), 1.78 - 1.73 (m, 1H), 1.52 - 1.50 (m, 1H), 1.07 - 1.04 (m, 1H).

Example 61 Preparation of Compound A61 (S)-(3-(1-amino-1,3-dihydrospir o[indene-2,4'-piperidin]-1'-yl)-6-(3-((1-methyl-1H-pyrazol-5-yl)amino)prop-1-yn-1-yl) pyrazin-2-yl)methanol

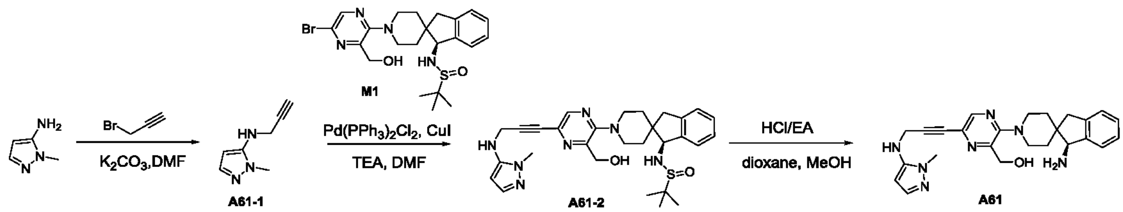


Step 1: Preparation of Compound A61-1

Dissolve 1.0 g of 1-methyl-5-Aminopyrazole in DMF (15.0 mL), add 2.13 g of $K_2CO_3$ and stir at room temperature for 10 min, drop 1.35 g of bromopropyne, replace with nitrogen, and stir at 60 °C for 18 h, LC-MS and TLC detected the reaction was complete, 20 mL of water was added, extracted with ethyl acetate (3×20 mL), washed with saturated brine, dried over anhydrous sodium sulfate, concentrated in vacuo, and the crude product was purified by column chromatography to obtain 700 mg of white solid compound A61-1.

Step 2: Preparation of Compound A61-2

150 mg of compound M1, 6.4 mg of $PdCl_2(PPh_3)_2$, 3.5 mg of CuI and 144 mg of compound A61-1 were dissolved in DMF (4.0 mL) and $Et_3N$ (1.0 mL), and the reaction was carried out at 60 °C for 14 h after nitrogen replacement. LC-MS and TLC detected that the reaction was complete, 20 mL of water was added, extracted with 10 mL of ethyl acetate, the organic phase was washed with saturated brine, dried over anhydrous sodium sulfate, and concentrated in vacuo. The crude product was purified by preparative plate to obtain 140 mg of pale yellow solid A61-2.

Step 3: Preparation of Compound A61

140 mg of compound A61-2 was dissolved in 9 mL of dioxane, 3 mL of MeOH was added, 0.26 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 71 mg of yellow solid compound A61.

[M+H+]=444.39.

1H NMR (500 MHz, DMSO) δ 8.16 (s, 1H), 7.30 (d, J = 7.1 Hz, 1H), 7.19-7.08 (m, 4H), 6.01 (t, J = 6.0 Hz, 1H), 5.51 (d, J = 1.8 Hz, 1H), 5.40 (t, J = 6.0 Hz, 1H), 4.46 (d, J = 5.9 Hz, 2H), 4.08 (d, J = 6.0 Hz, 2H), 3.83 (s , 1H), 3.81-3.70 (m, 2H), 3.54 (s, 3H), 3.14-3.05 (m, 2H), 3.02 (d, J = 15.7 Hz, 1H), 2.59 (d, J = 15.6 Hz, 1H), 1.90-1.83 (m, 1H), 1.79-1.73 (m, 1H), 1.5-1.50 (m, 1H), 1.12-1.08 (m, 1H).

Example 62 Preparation of Compound A62 (S)-4-((3-(5-(1-amino-1,3-dihydrospiro[inden-2,4'-piperidin]-1'-yl)pyrazin-2-yl)prop-2-yn-1-yl)oxy)-2-chlorobenzamide

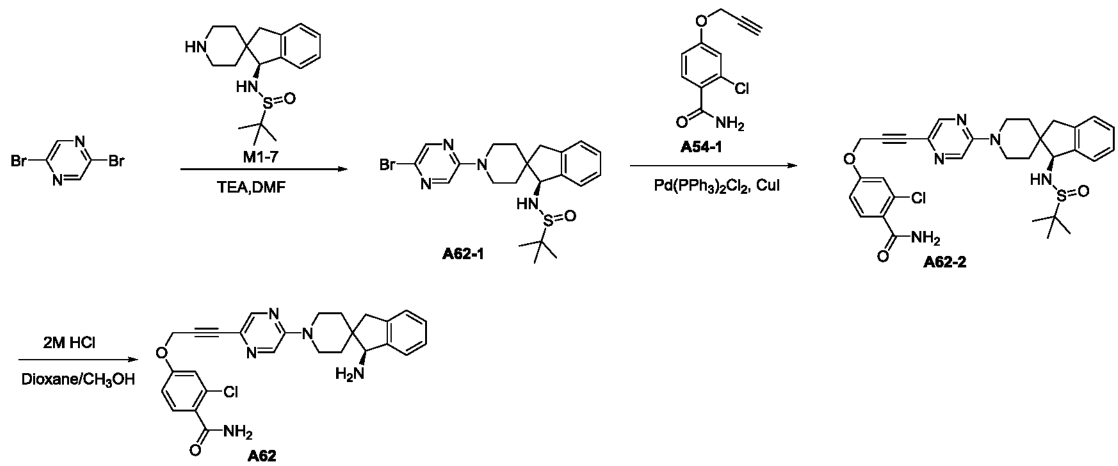


Step 1: Preparation of Compound A62-1

0.150 g of 2,4-dibromopyrazine and 0.203 g of compound M1-7 were dissolved in DMF (10 mL), 0.274 mL of triethylamine was added, and the reaction was heated at 80 °C overnight. TLC detected that the reaction of the raw materials was complete, the reaction solution was cooled to room temperature, 20 mL of water was added, a solid was precipitated, the solid was filtered, the filter cake was washed with water, and dried to obtain 0.175 g of a pale yellow solid, namely compound A62-1.

Step 2: Preparation of Compound A62-2

Dissolve 150 mg of compound A62-1, 121 mg of compound A54-1, 8 mg of $PdCl_2(PPh_3)_2$, 2.0 mg of CuI in DMF (4 mL) and triethylamine (1 mL), replace with nitrogen, and react at 60 °C overnight. LC-MS and TLC detected that the reaction of the raw materials was complete, 10 mL of water was added to precipitate a solid, filtered, the filter cake was dissolved in DCM, dried over sodium sulfate, and concentrated under reduced pressure.

Step 3: Preparation of Compound A62

95 mg of compound A62-2 was dissolved in dioxane (3 mL) and methanol (1.5 mL), 2N HCl (0.36 mL, ethyl acetate solution) was added, and the reaction was stirred

at RT for 1 hour. LC-MS and TLC detected that the reaction was complete, the reaction solution was adjusted to pH=8 with saturated $NaHCO_3$, and the reaction solution was concentrated under reduced pressure to obtain a crude product. The crude product was purified by a preparative plate to obtain 62.3 mg of a pale yellow solid, namely compound A62.

[M+H+]=488.23.

1H NMR (500 MHz, DMSO-d6) δ 8.32 (d, J = 1.5 Hz, 1H), 8.18 (d, J = 1.4 Hz, 1H), 7.76 (s, 1H), 7.47 (s, 1H), 7.44 (d, J = 8.5 Hz, 1H), 7.32-7.26 (m, 1H), 7.20-7.11 (m, 4H), 7.05 (dd, J = 8.5, 2.5 Hz, 1H), 5.14 (s, 2H), 4.30-4.18 (m, 2H), 3.82 (s, 1H), 3.26-3.12 (m, 2H), 3.07 (d, J = 15.7 Hz, 1H), 2.62 (d, J = 15.7 Hz, 1H), 1.77 -1.71 (m, 1H), 1.66-1.60 (m, 1H), 1.55-1.47 (m, 1H), 1.13-1.06 (m, 1H).

Example 81 Preparation of Compound A81 (S)-3-((3-(5-(1-amino-1,3-dihydrospiro[indene-2,4'-piperidin]-1'-yl)-6-(hydroxymethy 1)pyrazin-2-yl)prop-2-yn-1-yl)amino)benzamide

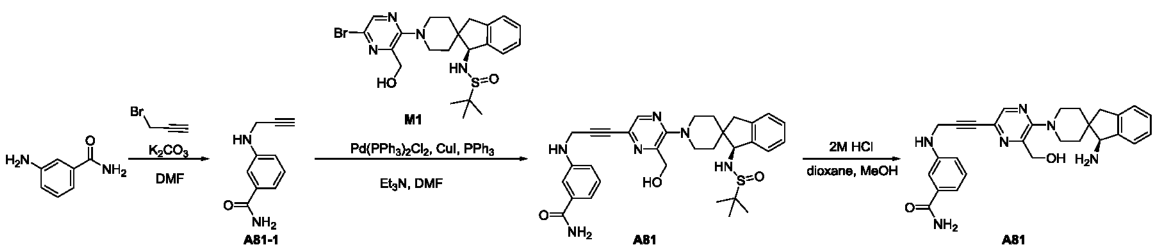

Step 1: Preparation of Compound A81-1

1 g of raw material 3-Aminobenzamide, 917 mg of 3-bromo-1-propyne, and 1.52 g of potassium carbonate were dissolved in 10 mL of DMF, and reacted at room temperature for 2 h. After TLC (DCM:MeOH=7:1) monitoring showed that the reaction was complete, 10 mL of water was added, extracted with 10 mL of EA, the organic phase was washed with 3×10 mL of saturated sodium chloride solution, the organic phase was separated, and dried over anhydrous sodium sulfate, concentrated under reduced pressure, and subjected to column chromatography (DCM:MeOH=10:1) to obtain 680 mg of light yellow powder of compound A81-1.

Step 2: Preparation of Compound A81-2

174 mg of compound A81-1, 150 mg of M1, 6.4 mg of $Pd(PPh_3)_2Cl_2$, and 3.5 mg of CuI were dissolved in 2 mL of DMF and 0.4 mL of triethylamine, replaced with N2, and reacted at 60 °C for 12 h. After TLC (DCM:MeOH=10:1) detection showed that the reaction of the raw materials was complete, 10 mL of ethyl acetate and 10 mL of water were added for extraction, the organic phase was separated, the organic phase was washed with 3×10 mL of saturated sodium chloride solution, separated and dried, separation and purification by column chromatography (DCM:MeOH=19:1) gave 121 mg of pale yellow powder, namely compound A81-2.

Step 3: Preparation of Compound A81

121 mg of compound A81-2 was dissolved in 4 mL of dioxane and 2 mL of methanol solution, replaced with nitrogen, 0.2 mL of HCl in EA solution (2 M) was added dropwise with stirring, and stirred at room temperature for 20 min. LC-MS detected that the reaction was complete, the reaction solution was concentrated under reduced pressure, the residue was dissolved in 5 mL of water, a saturated sodium bicarbonate solution was added dropwise to pH 8-9, the precipitated solid was collected, and 50.7 mg of yellow powder was obtained by separation and purification on a thick plate, namely compound A81.

[M+H+]=483.31.

1H NMR (500 MHz, DMSO-d6) δ 8.14 (s, 1H), 7.80 (s, 1H), 7.35-7.27 (m, 1H), 7.25-7.06 (m, 8H), 6.83 (dd, J = 8.0 , 2.4 Hz, 1H), 6.28 (t, J = 6.3 Hz, 1H), 5.41 (t, J = 6.0 Hz, 1H), 4.44 (d, J = 5.7 Hz, 2H), 4.20 (d, J = 6.2 Hz, 2H), 3.86 (s, 1H), 3.79-3.70 (m, 2H), 3.15-3.05 (m, 2H), 3.03 (d, J = 15.6 Hz, 1H), 2.61 (d, J = 15.8 Hz , 1H), 1.89-1.83 (m, 1H), 1.78-1.74 (m, 1H), 1.51-1.49 (m, 1H), 1.13-1.11 (m, 1H).

Example 82 Preparation of Compound A82 (S)-2-(4-((3-(5-(1-amino-1,3-dihydrospiro[indene-2,4'-piperidin]-1'-yl)-6-(hydroxyme thyl)pyrazin-2-yl)prop-2-yn-1-yl)oxy)-1H-pyrazol-1-yl)acetamide

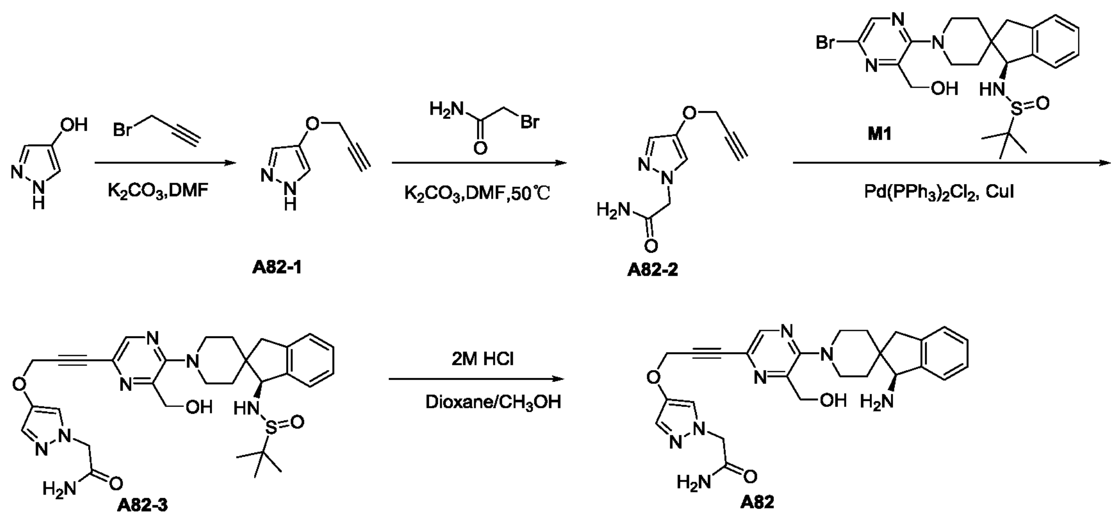


Step 1: Preparation of Compound A82-1

0.200 g of 4-hydroxypyrazole, 0.513 g of potassium carbonate and 0.340 g of bromopropyne were dissolved

in DMF (2 mL) and reacted overnight at room temperature. TLC detected that the reaction of the raw materials was complete, 10 mL of water was added, extracted with ethyl acetate (10 mL×3), the organic phases were combined, washed with saturated brine, dried over anhydrous sodium sulfate, and concentrated in vacuo. The crude product was purified by column chromatography to obtain a solid of 0.190 g, namely compound A82-1.

Step 2: Preparation of Compound A82-2

0.215 g of compound A82-1, 0.487 g of potassium carbonate and 0.364 g of bromopropyne were dissolved in DMF (4 mL), and heated at 50 °C to react for 4 hours. TLC detected that the reaction of the raw materials was complete, 10 mL of water was added, extracted with ethyl acetate (10 mL × 4), the organic phases were combined, washed with saturated brine (5 mL × 4), dried over anhydrous sodium sulfate, and concentrated in vacuo. The crude product was purified by column chromatography to obtain 0.211 g of solid, namely compound A82-2.

Step 3: Preparation of Compound A82-3

180 mg of compound M1, 98 mg of compound A82-2, 12.8 mg of $PdCl_2(PPh_3)_2$, 3.5 mg of CuI were dissolved in DMF (5 mL) and triethylamine (1 mL), and after nitrogen replacement, the reaction was carried out at 60 °C for 5 hours . LC-MS and TLC detected that the reaction was complete, 10 mL of water was added, (EA:THF=1:1) extraction (10 mL×4), the organic phase was washed with saturated brine, dried over anhydrous sodium sulfate, concentrated in vacuo, and the crude product was subjected to column chromatography purified to obtain solid 0.190 g, namely compound A82-3.

Step 4: Preparation of Compound A82

190 mg of compound A82-3 was dissolved in dioxane (5 mL) and methanol (1.5 mL), 2N HCl (0.45 mL, ethyl acetate solution) was added, and the reaction was stirred at RT for 1 hour. LC-MS and TLC detected that the reaction was complete, and the reaction solution was concentrated under reduced pressure. The residue was dissolved in $H_2O$ (1.0 mL), adjusted to pH=8 with saturated $NaHCO_3$, extracted with dichloromethane (5×3 mL), the organic phase was dried and concentrated in vacuo, and purified by preparative plate to obtain 85.0 mg of pale yellow solid, compound A82 .

[M+H+]=488.34.

1H NMR (500 MHz, DMSO-d6): δ 8.24 (s, 1H), 7.57 (s, 1H), 7.39 (s, 1H), 7.315-7.29 (m, 2H), 7.23 (s, 1H), 7.21 -7.11 (m, 3H), 5.45 (t, J = 5.5 Hz, 1H), 4.88 (s, 2H), 4.65 (s, 2H), 4.47 (d, J = 5.5 Hz, 2H), 3.87-3.75 ( m, 3H), 3.16-3.12 (m , 3H), 3.04 (d, J = 15.6 Hz, 1H), 2.61 (d, J = 15.6 Hz, 1H), 1.89-1.81 (m, 1H), 1.78-1.71 (m, 1H), 1.53-1.50 (m, 1H), 1.12-1.10 (m, 1H).

Through different reaction starting materials, suitable reagents (both starting materials and reagents are commercially available) and intermediates prepared by the present invention, the compounds in Table 2 were prepared by methods similar to the previous examples.

Table 2

| Example | Structure | Chemical Name | $[M+H]^+$ |
|---|---|---|---|
| Example5 (A05) | 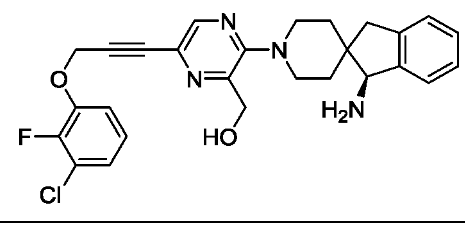 | (S)-(3-(1-amino-1,3-dihydrospiro [ indene-2,4'-piperidin]-1'-yl)-6-(3-( 3-chloro-2-fluorophenoxy)prop-1-yn-1-yl)pyrazin-2-yl)methanol | 493.17 |
| Example 10 (A10) | 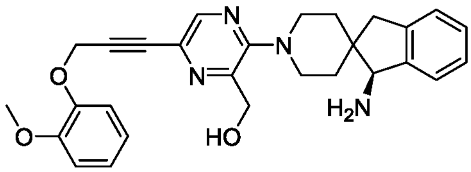 | (S)-(3-(1-amino-1,3-dihydrospiro [ indene-2,4'-piperidin]-1'-yl)-6-(3-( 2-methoxyphenoxy)prop-1-yn-1 -yl) pyrazin-2-yl)methanol | 471.23 |

(continued)

| Example | Structure | Chemical Name | [M+H]+ |
|---|---|---|---|
| Example 11 (A11) | 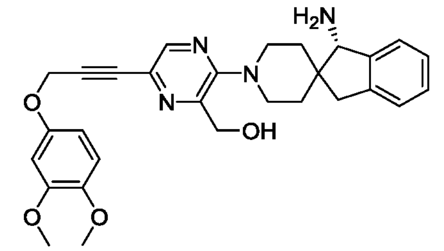 | (S)-(3-(1-amino-1,3-dihydrospiro [ indene-2,4'-piperidin]-1'-yl)-6-(3-( 3,4-dimethoxyphenoxy)prop-1-yn-1-y l)pyrazin-2-yl)methanol | 501.24 |
| Example 12 (A12) | 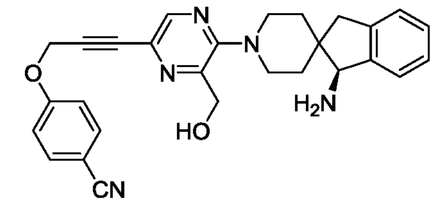 | (S)-4-((3-(5-(1-amino-1,3-dihydro spiro[indene-2,4'-piperidin]-1'-yl)-6-(hydroxymethyl)pyrazin-2-yl)pr op-2-yn-1-yl)oxy)benzonitrile | 466.22 |
| Example 14 (A14) | 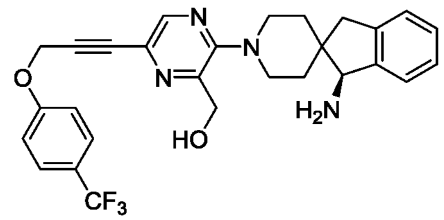 | (S)-(3-(1-amino-1,3-dihydrospiro [ indene-2,4'-piperidin]-1'-yl)-6-(3-( 4-(trifluoromethyl)phenoxy) prop-1-yn-1-yl)pyrazin-2-yl)methanol | 509.21 |
| Example 15 (A15) | 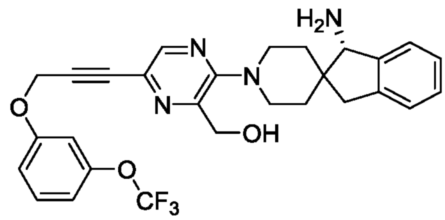 | (S)-(3-(1-amino-1,3-dihydrospiro [ indene-2,4'-piperidin]-1'-yl)-6-(3-( 3-(trifluoromethoxy)phenoxy) pro p-1-yn-1-yl)pyrazin-2-yl)methano 1 | 525.20 |
| Example 16 (A16) | 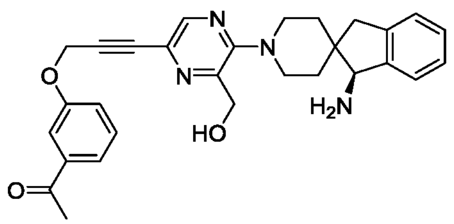 | (S)-1-(3-((3-(5-(1-amino-1,3-dihy drospiro[indene-2,4'-piperidin]-1'-yl)-6-(hydroxymethyl)pyrazin-2-y l)prop-2-yn-1-yl)oxy)phenyl)etha n-1-one | 483.23 |
| Example 19 (A19) | 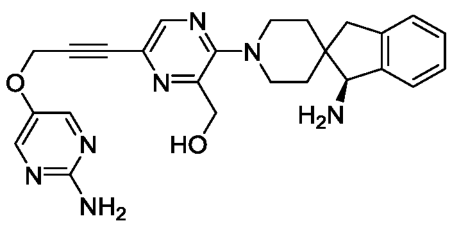 | (S)-(3-(1-amino-1,3-dihydrospiro [ indene-2,4'-piperidin]-1'-yl)-6-(3-( (2-Aminopyrimidine-5-yl)oxy)pro p-1-yn-1-yl)pyrazin-2-yl)methano 1 | 458.22 |

(continued)

| Example | Structure | Chemical Name | [M+H]+ |
|---|---|---|---|
| Example20 (A20) | 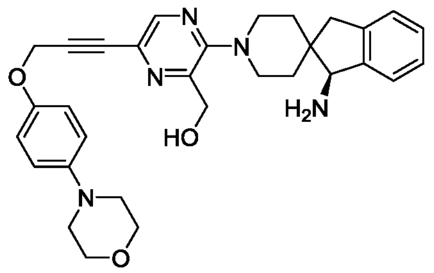 | (S)-(3-(1-amino-1,3-dihydrospiro [ indene-2,4'-piperidin]-1'-yl)-6-(3-( 4-morpholinoloxy)prop-1-yn-1-yl ) pyrazin-2-yl)methanol | 526.27 |
| Example23 (A23) | 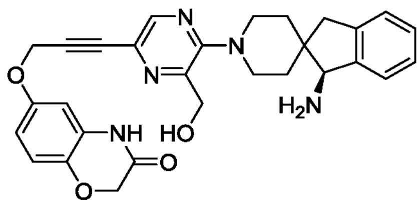 | (S)-6-((3-(5-(1-amino-1,3-dihydro spiro[indene-2,4'-piperidin]-1'-yl)-6-(hydroxymethyl)pyrazin-2-yl)pr op-2-yn-1-yl)oxy)-2Hbenzo[b] [1, 4] oxazin-3(4H)-one | 512.22 |
| Example24 (A24) | 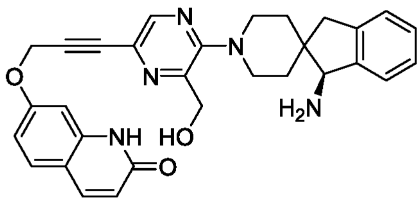 | (S)-7-((3-(5-(1-amino-1,3-dihydro spiro[indene-2,4'-piperidin]-1'-yl)-6-(hydroxymethyl)pyrazin-2-yl)pr op-2-yn-1-yl)oxy)quinolin-2(1H)-one | 508.23 |
| Example25 (A25) | 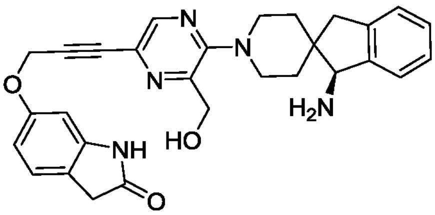 | (S)-6-((3-(5-(1-amino-1,3-dihydro spiro[indene-2,4'-piperidin]-1'-yl)-6-(hydroxymethyl)pyrazin-2-yl)pr op-2-yn-1-yl)oxy)indol-2-one | 496.23 |
| Example28 (A28) | 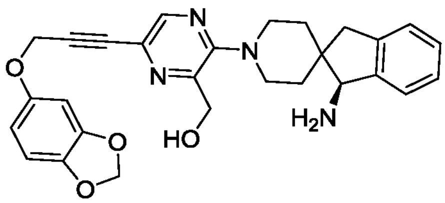 | (S)-(3-(1-amino-1,3-dihydrospiro [ indene-2,4'-piperidin]-1'-yl)-6-(3-( benzo[d][1,3]dioxyol-5-oxy) prop-1-yn-1-yl)pyrazin-2-yl)methanol | 485.21 |
| Example29 (A29) | 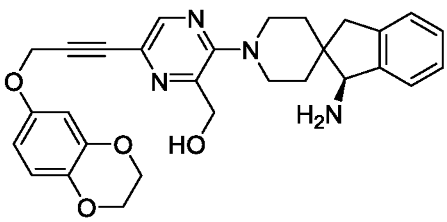 | (S)-(3-(1-amino-1,3-dihydrospiro [ indene-2,4'-piperidin]-1'-yl)-6-(3-( (( (2,3-dihydrobenzo[b][1,4]dioxin -6-yl) oxy)prop-1-yn-1-yl)pyrazin-2-yl) methanol | 499.23 |
| Example30 (A30) | 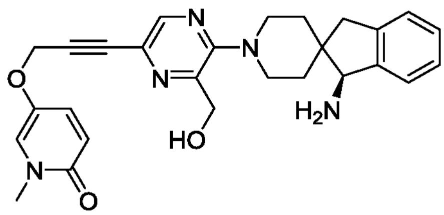 | (S)-5-((3-(5-(1-amino-1,3-dihydro spiro[indene-2,4'-piperidin]-1'-yl)-6-(hydroxymethyl)pyrazin-2-yl)pr op-2-yn-1-yl)oxy)-1-methylpyridi n-2 (1H)-one | 472.23 |

(continued)

| Example | Structure | Chemical Name | [M+H]+ |
|---|---|---|---|
| Example31 (A31) | 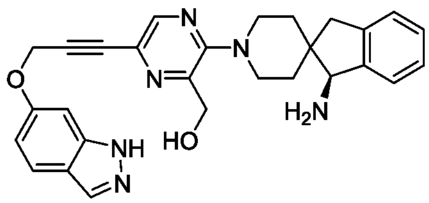 | (S)-(6-(3-((1H-Indolazol-6-yl)oxy ) prop-1-yn-1-yl)-3-(1-amino-1,3-d ihydrospiro[indene-2,4'-piperidin] -1'-yl)pyrazin-2-yl)methanol | 481.23 |
| Example34 (A34) | 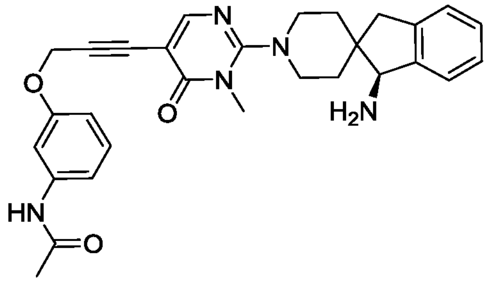 | (S)-N-(3-((3-(2-(1-amino-1,3-dihy drospiro [indene-2,4'-piperidin] -1'-yl)-1-methyl-6-oxo-1,6-dihydropy rimidin-5-yl)prop-2-yn-1-yl)oxy)p henyl) acetamide | 498.24 |
| Example35 (A35) | 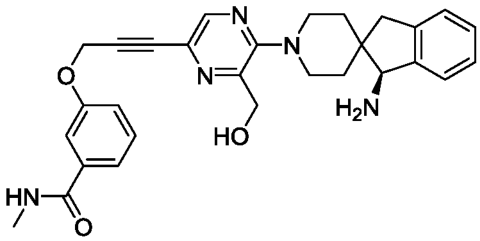 | (S)-3-((3-(5-(1-amino-1,3-dihydro spiro[indene-2,4'-piperidine]-1'-yl)-6-(hydroxymethyl)pyrazin-2-yl) prop-2-yn-1-yl)oxy)-N-methylben zamide | 498.24 |
| Example36 (A36) | 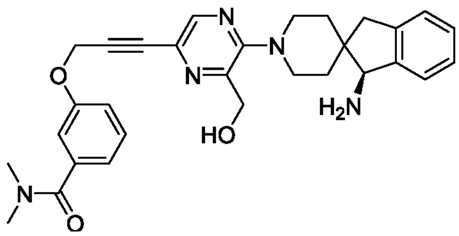 | (S)-3-((3-(5-(1-amino-1,3-dihydro spiro[indene-2,4'-piperidin]-1'-yl)-6-(hydroxymethyl)pyrazin-2-yl)pr op-2-yn-1-yl)oxy)-N,N-dimethylb enzamide | 512.26 |
| Example37 (A37) | 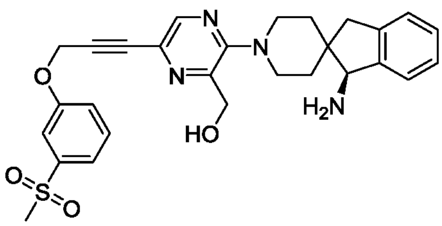 | (S)-(3-(1-amino-1,3-dihydrospiro [ indene-2,4'-piperidin]-1'-yl)-6-(3-( 3-(methylthio)phenoxy)prop-1-yn -1-yl)pyrazin-2-yl)methanol | 519.20 |
| Example3 8 (A38) | 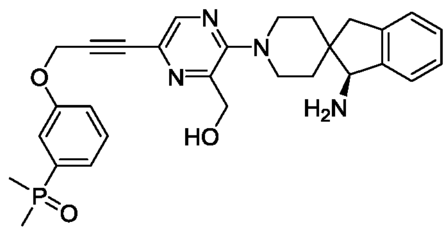 | (S)-(3-((3-(5-(1-amino-1,3-dihydr ospiro[indene-2,4'-piperidin]-1'-yl )-6-(hydroxymethyl)yl)pyrazin-2-yl) prop-2-yn-1-yl)oxy)phenyl)dim ethylphosphine oxide | 517.23 |

(continued)

| Example | Structure | Chemical Name | [M+H]+ |
|---|---|---|---|
| Example39 (A39) | 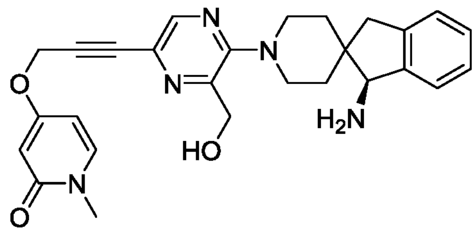 | (S)-4-((3-(5-(1-amino-1,3-dihydro spiro[indene-2,4'-piperidin]-1'-yl)-6-(hydroxymethyl)pyrazin-2-yl)pr op-2-yn-1-yl)oxy)-1-methylpyridi n-2 (1H)-one | 472.23 |
| Example40 (A40) | 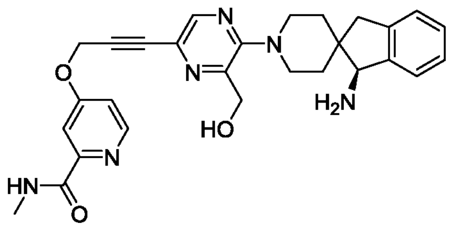 | (S)-4-((3-(5-(1-amino-1,3-dihydro spiro[indene-2,4'-piperidin]-1'-yl)-6-(hydroxymethyl)pyrazin-2-yl)pr op-2-yn-1-yl)oxy)-N-picoline amide | 499.24 |
| Example42 (A42) | 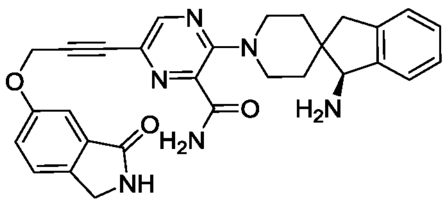 | (S)-3-(1-amino-1,3-dihydro spiro[i ndene-2,4'-piperidin]-1'-yl)-6-(3-( (3-oxoisooctanol)-5-yl)oxy)prop-1-yn-1-yl)pyrazine-2- formamide | 509.22 |
| Example43 (A43) | 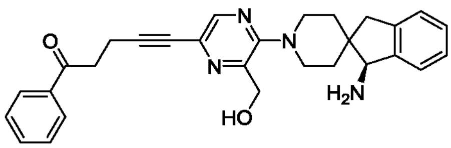 | (S)-5-(5-(1-amino-1,3-dihydrospir o [indene-2,4'-piperidin]-1'-yl)-6-( hydroxymethyl)pyrazine-2-yl)-1 -phenylpent-4-yn-1 -one | 467.24 |
| Example44 (A44) | 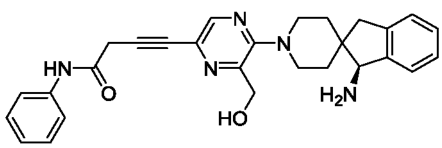 | (S)-4-(5-(1-amino-1,3-dihydrospir o [indene-2,4'-piperidin]-1'-yl)-6-( hydroxymethyl)pyrazine-2-yl)-N-phenyl-3-alkynamide | 468.23 |
| Example56 ( A56) | 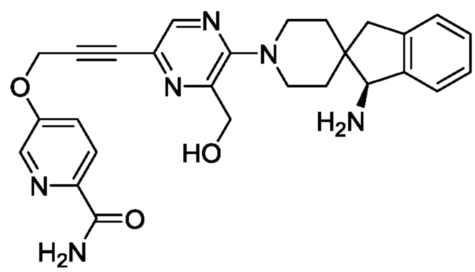 | (S)-5-((3-(5-(1-amino-1,3-dihydro spiro[indene-2,4'-piperidin]-1'-yl)-6-(hydroxymethyl)pyrazin-2-yl)pr op-2-yn-1-yl)oxy)pyridineamide | 485.34 |
| Example63 ( A63) | 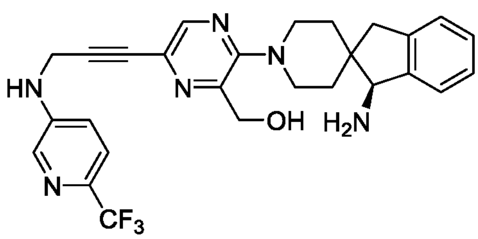 | (S)-(3-(1-amino-1,3-dihydrospiro [ indene-2,4'-piperidin]-1'-yl)-6-(3-( (6-(trifluoromethyl)pyridin-3-yl)a mino) prop-1-yn-1-yl)pyrazin-2-yl )methanol | 509.32 |

(continued)

| Example | Structure | Chemical Name | [M+H]+ |
|---|---|---|---|
| Example64 ( A64) | 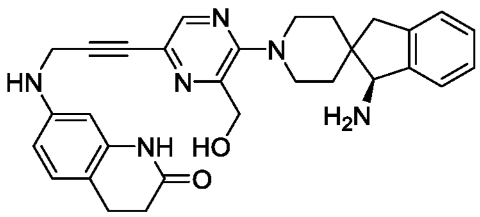 | (S)-7-((3-(5-(1-amino-1,3-dihydro spiro[indene-2,4'-piperidin]-1'-yl)-6-(hydroxymethyl)pyrazin-2-yl)pr op-2-yn-1-yl)amino)-3,4-dihydroq uinolin-2(1H)-one | 509.32 |
| Example65 ( A65) | 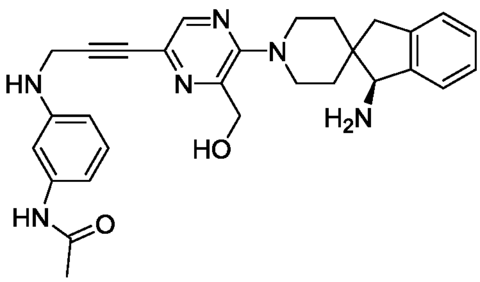 | (S)-N-(3-((3-(5-(1-amino-1,3-dihy drospiro [indene-2,4'-piperidin] -1'-yl)-6-(hydroxylmethyl)pyrazin-2-yl)prop-2-yn-1-yl)amino)phenyl)a cetamide | 497.33 |
| Example66 ( A66) | 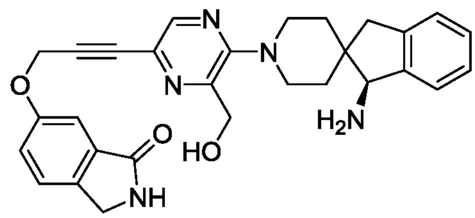 | (S)-6-((3-(5-(1-amino-1,3-dihydro spiro[indene-2,4'-piperidin]-1'-yl)-6-(hydroxymethyl)pyrazin-2-yl)pr op-2-yn-1 -yl)oxy)isoindol-1 -one | 496.31 |
| Example67 ( A67) | 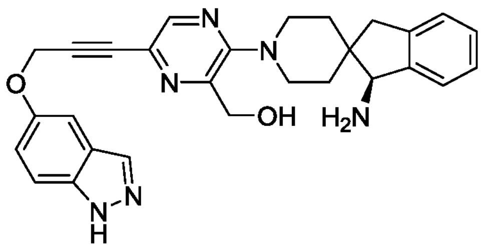 | (S)-(6-(3-((1H-Indazol-5-yl)oxy)p rop-1-yn-1-yl)-3-(1-amino-1,3-dih ydrospiro[Indene-2,4'-piperidin]-1 '-yl) pyrazin-2-yl)methanol | 481.32 |
| Example68 ( A68) | 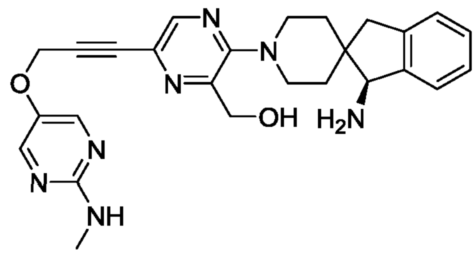 | (S)-(3-(1-amino-1,3-dihydrospiro [ indene-2,4'-piperidin]-1'-yl)-6-(3-( (2-(methylAmino)pyrimidin-5-yl) oxy) prop-1-yn-1-yl)pyrazin-2-yl) methanol | 472.34 |
| Example69 ( A69) | 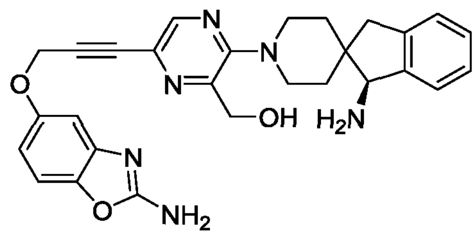 | (S)-(3-(1-amino-1,3-dihydrospiro [ inden-2,4'-piperidin]-1'-yl)-6-(3-(( 2-Aminobenzo[d]oxazol-5-yl)oxy )prop-1-yn-1-yl)pyrazin-2-yl)met hanol | 497.3 |

(continued)

| Example | Structure | Chemical Name | [M+H]+ |
|---|---|---|---|
| Example70 ( A70) | 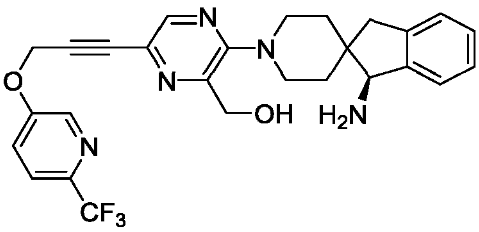 | (S)-(3-(1-amino-1,3-dihydrospiro [ indene-2,4'-piperidin]-1'-yl)-6-(3-( (6-(trifluoromethyl)pyridin-3-yl)o xy) prop-1-yn-1-yl)pyrazin-2-yl)m ethanol | 510.27 |
| Example71 ( A71) | 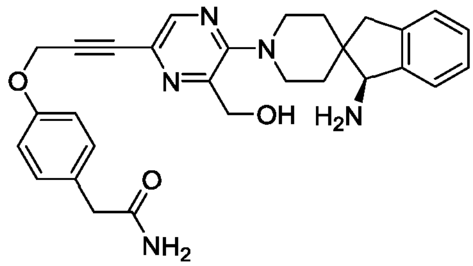 | (S)-2-(4-((3-(5-(1-amino-1,3-dihy drospiro[indene-2,4'-piperidin]-1'-yl)-6-(hydroxylmethyl)pyrazin-2-yl)prop-2-yn-1-yl)oxy)phenyl)ace tamide | 498.34 |
| Example72 ( A72) | 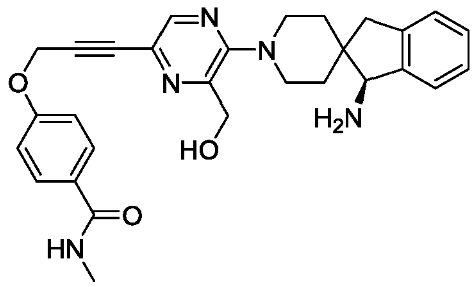 | (S)-4-((3-(5-(1-amino-1,3-dihydro spiro[indene-2,4'-piperidin]-1'-yl)-6-(hydroxymethyl)pyrazin-2-yl)pr op-2-yn-1-yl)oxy)-N-methylbenza mide | 498.33 |
| Example73 ( A73) | 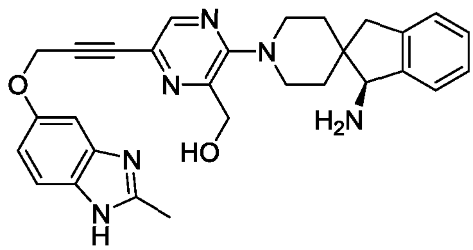 | (S)-(3-(1-amino-1,3-dihydrospiro [ indene-2,4'-piperidin]-1'-yl)-6-(3-( (2-methyl-1H-Benzo[d]imidazol-5-yl)oxy) prop-1-yn-1-yl)pyrazin-2-yl)methanol | 495.31 |
| Example74 ( A74) | 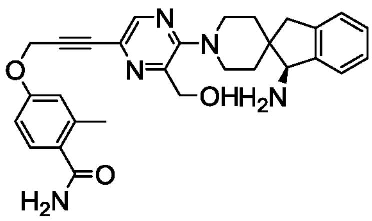 | (S)-4-((3-(5-(1-amino-1,3-dihydro spiro[indene-2,4'-piperidin]-1'-yl)-6-(hydroxymethyl)pyrazin-2-yl)pr op-2-yn-1-yl)oxy)-2-methylbenza mide | 498.38 |
| Example75 ( A75) | 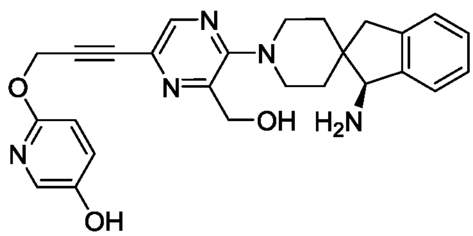 | (S)-6-((3-(5-(1-amino-1,3-dihydro spiro[indene-2,4'-piperidin]-1'-yl)-6-(hydroxymethyl)pyrazin-2-yl)pr op-2-yn-1-yl)oxy)pyridin-3-ol | 458.3 |

(continued)

| Example | Structure | Chemical Name | [M+H]+ |
|---|---|---|---|
| Example76 ( A76) | 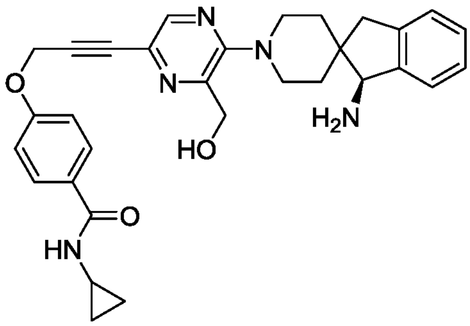 | (S)-4-((3-(5-(1-amino-1,3-dihydro spiro[indene-2,4'-piperidin]-1'-yl)-6-(hydroxymethyl)pyrazin-2-yl)pr op-2-yn-1-yl)oxy)-N-cyclopropyl benzamide | 524.33 |
| Example77 ( A77) | 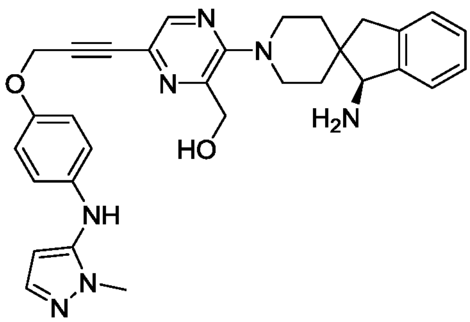 | (S)-(3-(1-amino-1,3-dihydrospiro [ indene-2,4'-piperidin]-1'-yl)-6-(3-( 4-((1-methyl)yl-1H-pyrazol-5-yl) a mino)phenoxy)prop-1-yn-1-yl)pyr azin-2-yl)methanol | 536.38 |
| Example83 (A83) | 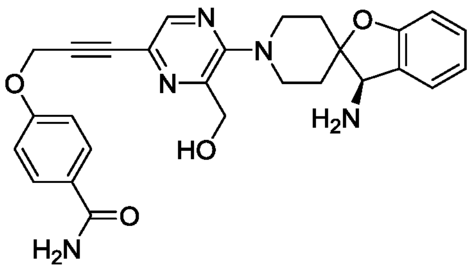 | (R)-4-((3-(5-(3-amino-3H-spiro[b enzofuran-2,4'-piperidin]-1'-yl)-6-(hydroxymethyl)pyrazine -2-yl)prop-2-yn-1-yl)oxy)benzami de | 486.21 |
| Example84 (A84) | 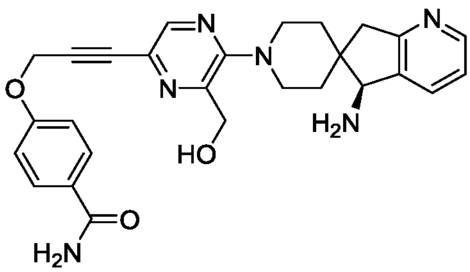 | (S)-4-((3-(5-(5-amino-5,7-dihydro spiro[cyclopenta[b]pyridin-6,4'-pi peridin]-1'-yl)-6-(hydroxymethyl) pyrazin-2-yl)pro p-2-yn-1-yl)oxy) benzamide | 485.22 |
| Example85 (A85) | 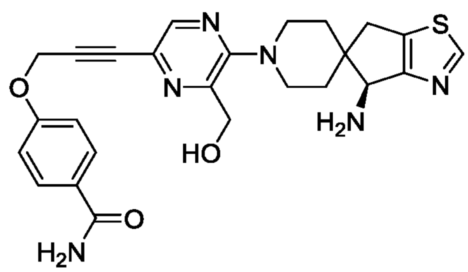 | (S)-4-((3-(5-(4-amino-4,6-dihydro spiro [cyclopenta[d]thiazol-5,4'-pi peridin]-1'-yl)-6-(hydroxymethyl) pyrazin-2-yl)pro p-2-yn-1-yl)oxy) benzamide | 491.18 |

(continued)

| Example | Structure | Chemical Name | [M+H]+ |
|---|---|---|---|
| Example86 (A86) | 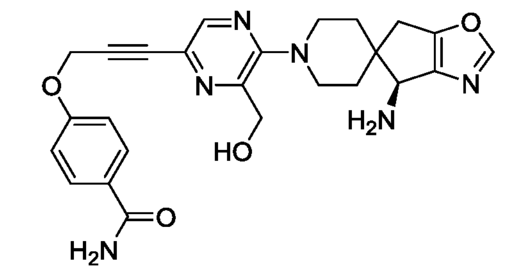 | (S)-4-((3-(5-(4-amino-4,6-dihydro spiro[cyclopenta[d]oxazol-5,4'-pi peridin]-1'-yl)-6 -(hydroxymethyl) pyrazin-2-yl)pro p-2-yn- 1-yl)oxy) benzamide | 475.20 |
| Example87 (A87) | 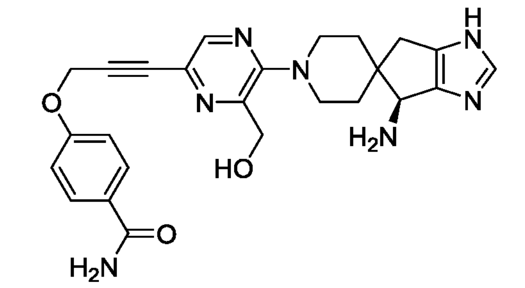 | (S)-4-((3-(5-(4-amino-4,6-dihydro -1H-spiro[cyclopenta[d]imidazol-5,4'-piperidin]-1'-yl)-6-(hydroxym ethyl) pyrazin-2-yl)prop-2-yn-1-yl )oxy) benzamide | 474.22 |

Compound A56: $^1$H NMR (500 MHz, DMSO) δ 8.39 (d, *J* = 2.8 Hz, 1H), 8.23 (s, 1H), 8.04 (d, *J* = 8.7 Hz, 1H), 7.96 (s, 1H), 7.66 - 7.62 (m, 1H), 7.51 (s, 1H), 7.30 (d, *J* = 6.6 Hz, 1H), 7.19 - 7.12 (m, 3H), 5.43 (t, *J* = 5.6 Hz, 1H), 5.28 (s, 2H), 4.46 (d, *J* = 5.3 Hz, 2H), 3.84 - 3.79 (m, 3H) , 3.17- 3.08 (m, 2H), 3.03 (d, *J* = 15.6 Hz, 1H), 2.60 (d, *J* = 15.6 Hz, 1H), 1.90 - 1.85 (m, 1H), 1.78 - 1.73 (m, 1H), 1.52 - 1.50 (m, 1H), 1.12- 1.10 (m, 1H)

Example 80 Preparation of Compound A80 1-(4-((3-(5-((S)-1-amino-1,3-dihydrospiro[indene-2,4'-piperidin]-1'-yl)-6-(hydroxyme thyl)pyrazin-2-yl)prop-2-yn-1-yl)oxy)phenyl)-2,2,2-trifluoroethane-1-ol

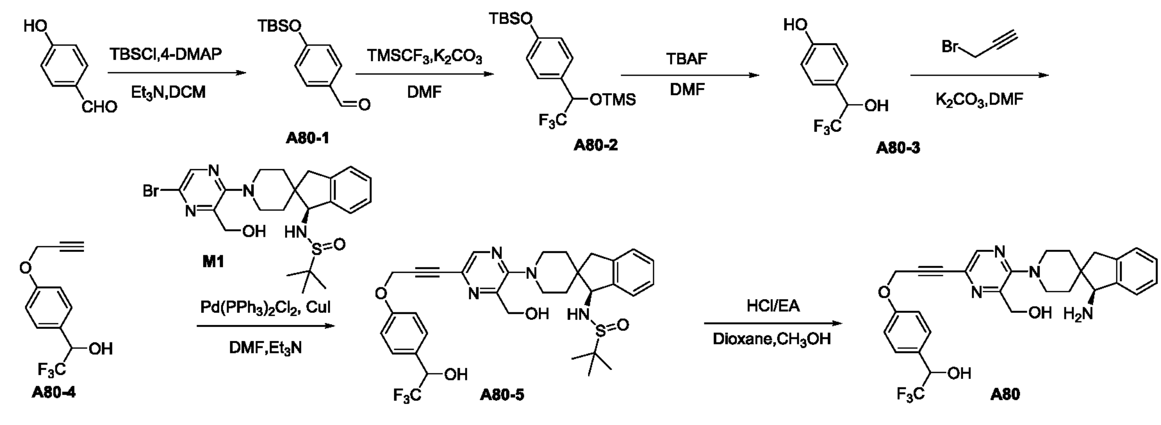


Step 1: Preparation of Compound A80-1

2.00 g of p-hydroxybenzaldehyde, 3.70 g of TBSCl and 0.20 g of DMAP were dissolved in DMF (50.0 mL) and $Et_3N$ (3.6 mL), and the reaction was stirred at room temperature for 4 h. LC-MS and TLC detected that the reaction was complete, 20 mL of water was added, It was extracted with ethyl acetate (3×20 mL), washed with saturated brine, dried over anhydrous sodium sulfate, and concentrated in vacuo. The crude product was purified by column chromatography to obtain 3.02 g of colorless transparent liquid, namely compound A80-1.

Step 2: Preparation of Compound A80-2

Under nitrogen protection, 1.50 g of compound A80-1, 1.35 g of $TMSCF_3$ and 0.088 g of potassium carbonate were dissolved in DMF (20 mL) and reacted at room temperature for 4 h. TLC detected the completion of the reaction, added 20 mL of water, extracted with ethyl acetate (3×20 mL), washed with saturated brine, dried over anhydrous sodium sulfate, and the organic phase was concentrated in vacuo to obtain 1.80 g of colorless and transparent liquid, namely compound A80-2 , proceed directly to the next reaction.

Step 3: Preparation of Compound A80-3

Under nitrogen protection, 1.80 g of compound A80-2 was dissolved in DMF (20 mL), and then TBAF (2M, 0.76 mL) was added to react at room temperature for 4 h. TLC detected the completion of the reaction, added 20 mL of water, extracted with ethyl acetate (3 × 20 mL), washed with saturated brine, dried over anhydrous sodium sulfate, concentrated the organic phase in vacuo, and purified by column chromatography to obtain 0.50 g of white solid, namely compound A80 -3.

Step 4: Preparation of Compound A80-4

Under nitrogen protection, 0.50 g of compound A80-3, 0.37 g of bromopropyne and 0.54 g of potassium carbonate were dissolved in DMF (10 mL) and reacted at 40 °C for 4 h. TLC detected the completion of the reaction, added 20 mL of water, extracted with ethyl acetate (3×20 mL), washed with saturated brine, dried over anhydrous sodium sulfate, concentrated the organic phase in vacuo, and obtained 0.55 g of colorless and transparent liquid by column chromatography, which is the compound A80-4.

Step 5: Preparation of Compound A80-5

150 mg of compound M22-2, 6.4 mg of $PdCl_2(PPh_3)_2$, 3.5 mg of CuI and 105 mg of compound A80-4 were dissolved in DMF (4.0 mL) and $Et_3N$ (1.0 mL), and after nitrogen replacement, the reaction was carried out at 60 °C for 2 h. LC-MS and TLC detected that the reaction was complete, 10 mL of ethyl acetate was added, the insolubles were filtered off, the filtrate was dispersed in 20 mL of water, the organic phase was washed with saturated brine, dried over anhydrous sodium sulfate, and concentrated in vacuo. The crude product was purified by column chromatography to obtain 180 mg Light yellow solid, namely compound A80-5.

Step 6: Preparation of Compound A80

180 mg of compound A80-5 was dissolved in 6 mL of dioxane, 2 mL of MeOH was added, 0.28 mL of HCl in EA solution (2M) was added dropwise with stirring, and the mixture was stirred at room temperature for 30 min. LC-MS detected that the reaction was complete, the reaction solution was concentrated under reduced pressure, the residue was dissolved in 10 mL of water, and saturated sodium bicarbonate was added dropwise to adjust the pH to 8-9, the precipitated solid was collected, and purified with a preparative plate to obtain 101.5 mg of light yellow solid, namely Compound A80.

[M+H+]=431.34.

1H NMR (500 MHz, DMSO) δ 8.22 (s, 1H), 7.44 (d, J = 8.2 Hz, 2H), 7.30 (d, J = 6.1 Hz, 1H), 7.16 (d, J = 6.7 Hz, 3H) ), 7.07 (d, J = 8.2 Hz, 2H), 6.74 (d, J = 5.1 Hz, 1H), 5.43 (s, 1H), 5.10 (s, 3H), 4.46 (d, J = 4.6 Hz, 2H) ), 3.86 - 3.72 (m, 3H), 3.16 - 3.09 (m, 2H), 3.03 (d, J = 15.8 Hz, 1H), 2.61 (d, J = 15.7 Hz, 1H), 1.90 - 1.85 (m, 1H), 1.78 - 1.73 (m, 1H), 1.52 - 1.50 (m, 1H), 1.07 - 1.04 (m, 1H).

Through different reaction starting materials, suitable reagents (both starting materials and reagents are commercially available) and intermediates prepared by the present invention, the compounds in Table 3 were prepared by methods similar to Example 80.

Table 3

| Example | Structure | Name | [M+H]+ |
|---|---|---|---|
| Example78 (A78) | 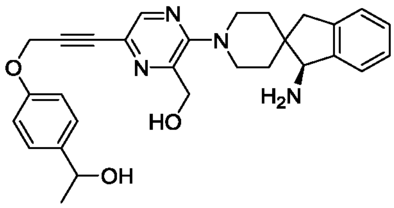 | 1-(4-((3-(5-((S)-1-amino-1,3-dihy drospiro [indene-2,4'-piperidin]-1'-yl)-6-(hydroxyl) methyl)pyrazin-2-yl)prop-2-yn-1-yl)o xy) phenyl)ethan-1-ol | 485.25 |
| Example79 (A79) | 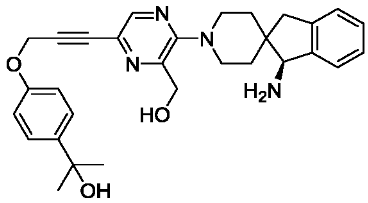 | (S)-2-(4-((3-(5-(1-amino-1,3-dihydros piro [indene-2,4'-piperidin]-1'-yl)-6-(hy droxyl Methyl)pyrazin-2-yl)prop-2-yn-1-yl)o xy) phenyl)propan-2-ol | 499.26 |

## PHARMACOLOGICAL TEST

### Example A: SHP2 allosteric inhibitory enzyme activity assay

SHP2 is allosterically activated by binding of a bis-tyrosyl-phosphorylated peptide to its Src homology 2 (SH2) domain. This later activation step results in the release of the autoinhibitory interface of SHP2, which in turn activates the SHP2 protein tyrosine phosphatase (PTP) and is available for substrate recognition and reaction catalysis. The catalytic activity of SHP2 was monitored using the surrogate DiFMUP in a rapid fluorescence assay format.
experiment procedure:

(1) Compound preparation:
The compounds of the present invention (10 mM stock solution) were diluted to appropriate times with 100% DMSO, and the final test concentrations of the compounds of the present invention were 10 μM, 3.3333 μM, 1.1111 μM, 0.3704 μM, 0.1235 μM, 0.0412 μM, 0.0137 μM, 0.0046 μM, 0.0015 μM, 0.00 μM;
(2) Prepare the enzyme reaction working solution:
Perform SHP2 enzymatic assays in 96-well black polystyrene plates (flat bottom, low flange, non-binding surface) (Perki Elmer, Cat#6005270) at room temperature using a final reaction volume of 50 μL and the following assay buffer conditions: 60 mM HEPES, 75 mM NaCl, 75 mM KCl, 0.05% BRIJ-35, 1 mM EDTA, 5 mM DTT.
(3) Enzyme-catalyzed reaction and data monitoring:
Add the compound of the present invention to the corresponding 96-well plate, and set the blank test well without compound and enzyme only with buffer. Thaw SHP2 Activating Peptide (IRS1_pY1172(dPEG8)pY1222) on ice, add 25 μM to each well, then add 0.2 ng of SHP2 protein sample to the corresponding well plate and incubate at room temperature for 1 hour. The surrogate substrate DiFMUP (Invitrogen, Cat #D6567) was added to the reaction and allowed to react for 1 hour at room temperature. Fluorescence signals were monitored using a microplate reader (Envision, Perki Elmer) using excitation and emission wavelengths of 340 nM and 450 nM, respectively.
(4) Data analysis:
Calculation Formula:

$$\text{Suppression rate }\% = [1-(\text{Conversion_sample}-\text{Conversion_min})/(\text{Conversion_max}-\text{Conversion_min})]\times 100\%$$

Among them: Conversion_sample is the conversion rate reading of the sample; Conversion_min is the average value of blank control wells, representing the conversion rate readings of wells without enzymatic activity; Conversion_max is the mean ratio of positive control wells, representing the conversion rate readings of wells without compound inhibition. The log(inhibitor) vs. response-Variable slope of the analysis software GraphPad Prism was used to fit the dose-response curve, and the $IC_{50}$ value of the compound for the enzymatic activity was calculated.
The $IC_{50}$ data of some examples are shown in Table 4.

Table 4

| No. | SHP2 enzymatic activity $IC_{50}$ (nM) | No. | SHP2 enzymatic activity $IC_{50}$ (nM) |
|---|---|---|---|
| A01 | 1.2 | A47 | 4 |
| A02 | 0.7 | A48 | 2 |
| A03 | 1.1 | A49 | 1.4 |
| A04 | 2.9 | A50 | 1.7 |
| A05 | 4.9 | A51 | 2.4 |
| A06 | 6.8 | A52 | 1.6 |
| A07 | 2.5 | A53 | 3.3 |
| A08 | 3.3 | A54 | 0.7 |
| A09 | 0.9 | A55 | 2.7 |
| A10 | 3.7 | A57 | 4.3 |
| A11 | 1.5 | A58 | 1.5 |
| A12 | 1 | A59 | 1.1 |
| A13 | 5.8 | A60 | 1.6 |
| A15 | 11 | A61 | 1.8 |
| A16 | 2.4 | A62 | 1.5 |
| A17 | 2.4 | A63 | 5 |
| A18 | 35.0 | A64 | 2.6 |
| A20 | 3.8 | A65 | 3.5 |
| A21 | 2.1 | A66 | 2.1 |
| A22 | 4.6 | A67 | 3.1 |
| A24 | 2.4 | A68 | 2.1 |
| A25 | 1.7 | A69 | 2.1 |
| A26 | 1.6 | A70 | 2.6 |
| A27 | 1.9 | A71 | 2.9 |
| A28 | 1.5 | A72 | 2.6 |
| A32 | 1.3 | A73 | 3.1 |
| A34 | 5.3 | A74 | 1.1 |
| A35 | 2.2 | A75 | 7.8 |
| A36 | 7.2 | A76 | 3.4 |
| A37 | 2 | A77 | 5.9 |
| A41 | 14 | A80 | 2.8 |
| A42 | 15 | | |
| A45 | 4.7 | | |

The compounds of the present invention have an allosteric inhibitory effect on SHP2 protein.

**Example B: Cell Proliferation Assay**

The effects of the compounds of the invention on the proliferation of leukemia cells MV-4-11 and lung cancer

cells NCI-H358 were evaluated using in vitro cell assays. The detection method used in the experiment is the CELL TITER-GLO (CTG) luminescence method, which can detect the number of viable cells by quantifying ATP. Because ATP participates in a variety of enzymatic reactions in vivo, it is an indicator of the metabolism of living cells, and its content directly reflects the number and state of cells. During the experiment, CellTiter-Glo™ reagent was added to the cell culture medium to measure the luminescence value. The value is proportional to the amount of ATP, and ATP is positively related to the number of living cells, so the cell viability can be examined by detecting the ATP content. experiment procedure:

(1) Cell plating:

Take a bottle of MV-4-11 cells in logarithmic growth phase, resuspend the cells by centrifugation and count them. After adjusting the cell density, they were seeded into a 96-well plate (Corning #3917). After culturing in a 5% $CO_2$ incubator for 24hrs, the compound of the present invention was added for treatment;
Take a bottle of NCI-H358 cells in logarithmic growth phase, digest and resuspend the cells, count them, adjust the cell density, and inoculate them into a 96-well transparent ultra-low adsorption cell culture plate (Corning #3474), inoculate 2000 cells per well. The plate was placed in an incubator at 37°C and 5% $CO_2$ for 24hrs, and then the compound of the present invention was added for treatment;

(2) Cell compound treatment:
An appropriate amount of the compound of the present invention was prepared for cell treatment. The final concentration of the compound from high to low was 1000 nM, 333.3 nM, 111.1 nM, 37.04 nM, 12.35 nM, 4.115 nM, 1.372 nM, 0.4572 nM, 0.1524 nM, and 0 nM. The plate was placed in a 37 °C, 5% $CO_2$ incubator for 120 hrs. Only adding medium without cell wells was set as the blank group; the group with compound concentration of 0 nM was the zero-adjusting group.
(3) CTG detection:

After NCI-H358 cells were cultured for 96 hrs, 50 μL of CellTiter-Glo® Luminescent Cell Viability Assay solution was added to each well, shaken gently for 2 mins, and incubated at room temperature for 10 mins. The cell reaction system was transferred to a white-bottomed 96-well plate. Read the detection value of each well on a multi-function microplate reader.
After MV-4-11 cells were cultured for 120 hrs, 50 μL of CellTiter-Glo® Luminescent Cell Viability Assay solution was added to each well, gently shaken for 2 mins, and incubated at room temperature for 10 mins. Check the value.

(4) Data analysis:
Calculate the inhibition rate from the luminescence value readings,

$$\text{Inhibition rate } \% = (1 - (\text{administration group value} - \text{blank group value})/(\text{zero adjustment group value} - \text{blank group value}) \times 100$$

The log(inhibitor) vs. response-Variable slope of GraphPad Prism was fitted to dose-response curves and IC50s of compounds that inhibited cell proliferation were calculated.
The experimental data are shown in Table 5.

Table 5

| No. | MV-4-11 cells IC50(nM) | NCI-H358 cells $IC_{50}$(nM) |
|---|---|---|
| A01 | 2.6 | 7.3 |
| A02 | 5.0 | 7.8 |
| A03 | 1.5 | 2.5 |
| A04 | 3.6 | 4 |
| A05 | 14 | 15 |
| A06 | 8 | 11 |

(continued)

| No. | MV-4-11 cells IC50(nM) | NCI-H358 cells $IC_{50}$(nM) |
|---|---|---|
| A07 | 5.5 | 12 |
| A08 | 2.1 | 3.8 |
| A09 | 2.6 | 4.9 |
| A10 | 4.4 | 4.3 |
| A11 | 2.6 | 6.3 |
| A12 | 3.8 | 7.4 |
| A13 | 18 | 28 |
| A16 | 6.9 | 11 |
| A17 | 1 | 1.5 |
| A20 | 12 | 14 |
| A21 | 11 | 12 |
| A22 | 2.9 | 2.3 |
| A24 | 1.3 | 1.5 |
| A25 | 0.9 | 2.3 |
| A26 | 1.8 | 5.2 |
| A27 | 1.4 | 1.5 |
| A28 | 6.1 | 7 |
| A32 | 3.4 | 9 |
| A34 | 25 | 51 |
| A35 | 3.0 | 3.6 |
| A36 | 14 | 48 |
| A37 | 11 | 9.6 |
| A45 | 20 | 40 |
| A47 | 17 | 48 |
| A48 | 1.7 | 12 |
| A49 | 6.7 | 8.5 |
| A50 | 2.2 | 3.3 |
| A51 | 1.0 | 0.8 |
| A52 | 1.5 | 4.6 |
| A53 | 8.1 | 27 |
| A54 | 1.6 | 2.2 |
| A55 | 9.6 | 26 |
| A57 | 10 | 22 |
| A58 | 0.8 | 4.0 |
| A59 | 1 | 5.8 |
| A60 | 13 | 41 |
| A61 | 5.1 | 9.4 |
| A62 | 14 | 56 |

(continued)

| No. | MV-4-11 cells IC50(nM) | NCI-H358 cells $IC_{50}$(nM) |
|---|---|---|
| A63 | 11 | 36 |
| A64 | 0.9 | 2.1 |
| A65 | 2.8 | 25 |
| A66 | 2.7 | 6.4 |
| A67 | 1.0 | 0.9 |
| A68 | 8.6 | 26 |
| A69 | 0.7 | 2.1 |
| A70 | 6.2 | 17 |
| A71 | 2.3 | 4.3 |
| A72 | 1.8 | 3.5 |
| A73 | 1.1 | 3.4 |
| A74 | 1.7 | 6.4 |
| A75 | 7.3 | 78 |
| A76 | 3.3 | 19 |
| A77 | 11 | 36 |
| A80 | 10.4 | / |
| A81 | 1.8 | 6.3 |
| A82 | 9 | 40 |
| Note: "/" means not tested. | | |

The compounds of the present invention have a good inhibitory effect on the proliferation of MV-4-11 cells and NCI-H358 cells.

**Example C Patch Clamp Experiments to Assess the Effects of Compounds on the hERG Ion Channel**

**Test solution Formulation:**

Dilute the test sample diluent in sequence with extracellular fluid to prepare detection solutions with final concentrations of 0.3 μM, 1μM, 3μM, 10 μM and 30 μM. The solubility of the samples to be tested was visually checked.

**Cell culture and plating:**

The cell line was derived from HEK293 cells and cultured in a 37 °C, 5% $CO_2$ incubator. In order to prevent cell senescence caused by contact inhibition, when the cell culture confluence should not exceed 80%, the cells should be passaged every 3/4 days, and the inoculation density of each T175 flask should be 2*106 cells. Pre-wash with Phosphate Buffered Saline (PBS), then trypsin/EDTA for 2-3 minutes, stop digestion by adding cell culture medium, and transfer to a new culture bottle.

HEK293 cells overexpressing the hERG potassium channel were cultured overnight at a cell density below 50%. Experimental cells were transferred to a cell bath embedded in an inverted microscope stage(Diaphot, Nikon) and perfused with extracellular fluid. The extracellular fluid contained 130 mM NaCl, 4 mM KCl, 1.8 mM $CaCl_2$, 1 mM $MgCl_2$, 10 mM glucose and 10 mM HEPES (pH 7.4 with NaOH) at a perfusion rate of 4 ml/min. The inner tube contains 130 mM KCl, 1 mM $MgCl_2$, 5 mM EGTA, 5 mM MgATP and 10 mM HEPES (pH 7.2 with KOH). Membrane currents were recorded using a HEKA EPC-10 patch-clamp amplifier and PATCHMASTER acquisition system (HEKA Instruments Inc., D-67466 Lambrecht, Pfalz, Germany). All experiments were done at room temperature (22-23 °C).

Electrodes (BF150-86-10) were straightened during the experiments using a P-97 microelectrode puller (Sutter Instrument Company, One Digital Drive, Novato, CA 94949). The inner diameter of the electrode is 1-1.5 mm, and the

water entry resistance after filling with internal liquid is 2-4 MΩ.

**Electrophysiological stimulation protocol:**

When the whole-cell seal is formed, wait for the current to stabilize for 2 minutes (the current decay is less than 5% within 5 minutes, and the tail current value is greater than 500 pA). At this time, the peak value of the tail current is the control current value. The extracellular fluid containing the drug to be tested is then perfused. The same procedure was repeated 3-5 times, exposing each cell to 4-6 increasing concentrations of compounds. Blocking and deactivation of hERG during compound exposure and washout were continuously recorded.

When the whole-cell seal was formed, the cell membrane voltage was clamped at -80 mV, depolarized for 2 seconds every 12 seconds, and the clamping voltage was depolarized from -80 mV to -50 mV under a 5-second repolarization pulse of -50 mV. Measure the peak tail current.

Parametric analysis:

Peak hERG tail currents were measured under a 5 s repolarization pulse of -50 mV. Each drug concentration was plotted against the current inhibition rate as a function of the logarithm of the compound concentration. The following Hill equation was used to fit a concentration-response curve to fit $IC_{50}$.

$$Y = Bottom + \frac{Top - Bottom}{1 + (\frac{x}{EC50})^{Hillcoefficient}}$$

Y: test value; Bottom: the lowest test value (0); Top: the highest test value (1); Hillcoefficient: the maximum absolute value of the slope of the curve.

**Data Analysis and Statistics**

Experimental data were collected using PATCHMASTER (HEKA Instruments Inc., D-67466 Lambrecht, Pfalz, Germany) and analyzed and statistically analyzed using Origin (OriginLab Corporation, Northampton, MA).

Data are presented as mean ± standard deviation. Using t-Test test, whether there is a significant difference compared with the control group, when $p<0.05$, it is considered to have a significant difference.

The experimental data are shown in Table 6.

Table 6

| No. | hERG (μM) |
|---|---|
| A18 | >20 |
| A32 | >20 |
| A41 | >20 |
| A48 | >20 |
| A71 | >20 |
| A57 | >20 |
| A59 | 26 |

**Example D: In vitro metabolic stability of human and rat liver microsomes**

**Test buffer preparation:**

Dissolve 1900 mg of $MgCl_2$ in 400 mL of ultrapure water.

Dissolve 17.42 g $K_2HPO_4$ and 13.65 g $KH_2PO_4$ into 1000 mL of ultrapure water, respectively. Mix a certain proportion of $K_2HPO_4$ and $KH_2PO_4$ to adjust the pH to 7.30 ± 0.10.

**Reaction stop solution preparation:**

Prepare a solution of 10 ng/mL labetalol and 10 ng/mL glyburide in acetonitrile and store at 4 °C.

**Compound working solution preparation:**

Prepare 10 mM verapamil (positive control) and test article stock solutions in DMSO. Then, with MeOH/ACN/$H_2O$ solution (1:1:2 by volume), diluted to 50 μM and 200 μM working solutions, respectively.
experiment procedure:

1) Add 40 μL $MgCl_2$ and 306 μL PBS to 96 wells (set blank control group, positive control group, test compound sample group, and negative control group at the same time)
2) Add 4 μL of compound working solution to each well (4 μL of PBS buffer for blank group) (Note: the final concentration of DMSO volume in the system is ≤ 0.5%)
3) Add 10 μL of liver microsomes (concentration: 20 mg/mL) to each well, mix well, and pre-incubate at 37 °C for 10 minutes.
4) Add 40 μL of 10 mM NADPH working solution to each well to start the reaction (add equal volume of PBS buffer for blank control group and negative control group respectively), the total reaction volume is 400 μL.
5) Take out 50 μL samples from the above reaction solution at 0, 5, 15, and 45 minutes, and add 400 μL of reaction stop solution to stop the reaction
6) Shake the sample after the termination of the reaction on a shaker for 5 minutes.
7) Centrifuge the sample at 3200 rcf for 10 minutes. After the centrifugation is complete, transfer 50 μL of the supernatant to 200 μL of $H_2O$ for dilution for LC-MS/MS analysis.

data analysis
Calculations of $t_{1/2}$ and $CL_{int}$ are performed using the first-order kinetic equations:

$$k = -slope$$

$$t_{1/2} = 0.693/k$$

$$CL_{int} = k/C_{protein}$$

where k represents the elimination constant, calculated from a log-linear plot of percentage remaining versus time. $t_{1/2}$ represents half-life. $C_{protein}$ is the concentration of liver microsomes.

The experimental data are shown in Table 7.

Table 7

| No. | $CL_{int}$ (μL/min/mg protein) | | | | |
|---|---|---|---|---|---|
| | human | rat | mouse | monkey | dog |
| A12 | 0.2 | 5.5 | 8.3 | 7.3 | 6.2 |
| A13 | 6.3 | 2.4 | 6.6 | 2.6 | 0.9 |
| A14 | 1.4 | 1.8 | 6.4 | 3.1 | 7.8 |
| A17 | 0.4 | 15.9 | 12.0 | 5.0 | 10.6 |
| A19 | 8.2 | 31.6 | 21.4 | 21.2 | 18.3 |
| A22 | 1.6 | 20.7 | 13.2 | 21.0 | 13.6 |
| A24 | 1.6 | 11.0 | 8.3 | 9.9 | 8.8 |
| A25 | 9.0 | 33.2 | 5.1 | 19.7 | 13.2 |
| A26 | 2.2 | 9.0 | 9.8 | 6.3 | 1.2 |

(continued)

| No. | $CL_{int}$ (μL/min/mg protein) | | | | |
|---|---|---|---|---|---|
| | human | rat | mouse | monkey | dog |
| A27 | 4.0 | 7.5 | 17.7 | 15.9 | 4.8 |
| A32 | 2.2 | 14.7 | 10.6 | 10.1 | 1.9 |
| A37 | 19.1 | 16.7 | 18.9 | 28.7 | 10.8 |
| A40 | 11.0 | 11.7 | 11.0 | 15.5 | 7.8 |
| A45 | 5.2 | 7.9 | 6.3 | 3.2 | 9.6 |
| A81 | 14.2 | 18.4 | 11.5 | 4.0 | 13.5 |
| A48 | 7.3 | 15.8 | 15.2 | 8.6 | 26.3 |
| A50 | 7.4 | 11.4 | 3.1 | 5.9 | 5.2 |
| A51 | 9.9 | 12.5 | 16.7 | 26.2 | 22.2 |
| A54 | 12.1 | 12.8 | 10.4 | 13.7 | 12.8 |
| A55 | 14.7 | 24.7 | 14.3 | 20.0 | 20.7 |
| A59 | 10.7 | 16.2 | 16.8 | 15.9 | 14.9 |
| A82 | 8.6 | 5.3 | 10.5 | 4.5 | 3.9 |
| A60 | 9.9 | 10.4 | 15.1 | 16.9 | 10.8 |
| A61 | 13.0 | 15.9 | 14.1 | 6.1 | 14.5 |
| A62 | 2.6 | 8.1 | 9.7 | 19.2 | 2.9 |
| A80 | 2.7 | 14.0 | 9.0 | 1.0 | 3.1 |

**Example E: In vivo efficacy of MIA-PACA2 cell subcutaneous xenograft tumor model**

BALB/c nude mice, female, 6-8 weeks, weighing about 18-22 grams. Each mouse was subcutaneously inoculated with 0.2 mL (1×107 cells) MIA-$PaCa_2$ cells (plus Matrigel, 1:1 by volume). Dosing was initiated when the mean tumor volume reached approximately 100-150 cubic millimeters. Test compounds were administered orally daily at a dose of 10 mpk QD. Tumor volume was measured twice a week, and the volume was measured in cubic millimeters and was calculated by the following Formula: $V=0.5a\times b^2$, where a and b were the long and short diameters of the tumor, respectively. The tumor-inhibitory efficacy of the compounds was evaluated by TGI (%). TGI (%), reflecting tumor growth inhibition rate. Calculation of TGI (%): TGI (%) = [(1-(average tumor volume at the end of administration of a certain treatment group - average tumor volume at the beginning of administration of this treatment group))/(average tumor volume at the end of treatment in the solvent control group Volume - the average tumor volume of the solvent control group at the beginning of treatment)] × 100%.

In conclusion, most of the compounds listed in the present invention are highly potent and show significant improvement in safety and pharmacokinetics and excellent antitumor activity in in vivo models.

Although the present invention has been fully described in terms of its embodiments, it is worth noting that various changes and modifications will be apparent to those skilled in the art. Such changes and modifications are intended to be included within the scope of the appended claims of the present invention.

## Claims

1. A compound shown in Formula I, or a pharmaceutically acceptable salt, deuterated substance, cis-trans isomer, tautomer, solvate, chelate, non-covalent complex or prodrug thereof:

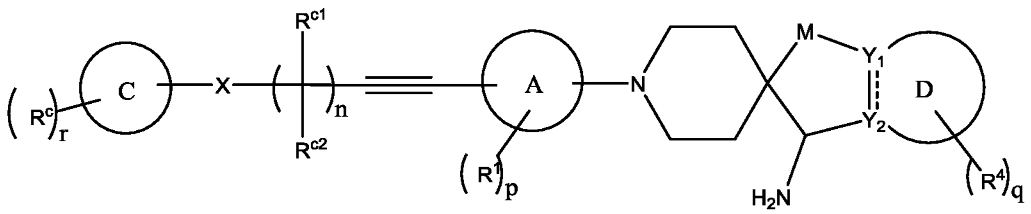

Formula I

- - - - - - is a single bond or a double bond;

ring A is selected from 5- to 10- membered heteroaryl or 5- to 10- membered heterocyclyl; wherein, the 5- to 10- membered heteroaryl or the 5- to 10- membered heterocyclyl contains one or more O, N or S heteroatom; the 5- to 10- membered heterocyclyl contains at least one double bond;

ring C is selected from 3- to 10- membered saturated or partially saturated heterocyclyl, $C_{6-10}$ aryl and 5- to 10- membered heteroaryl;

each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$, $OC_{2-6}$ alkenyl $C_{1-6}$ alkoxy, $-OC_{2-6}$ alkenyl $C_{6-10}$ aryl,

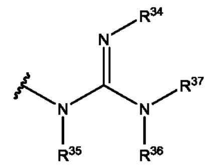 and 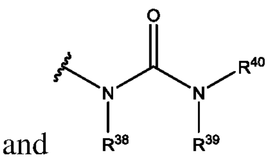

; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, or 5- to 10-membered heteroaryl is optionally substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$;

two $R^c$ with the atoms they are attached form $C_{3-8}$ carbocyclyl, 3- to 10-membered heterocyclyl, $C_{6-10}$ aryl, or 5- to 10- membered heteroaryl; wherein the $C_{3-8}$ carbocyclyl, 3- to 10- membered heterocyclyl, $C_{6-10}$ membered aryl, and 5- to 10-membered heteroaryl are optionally substituted by one or more substituents; the substituents are independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$, $OC_{2-6}$ alkenyl $C_{1-6}$ alkoxy, $-OC_{2-6}$ alkenyl $C_{6-10}$ aryl,

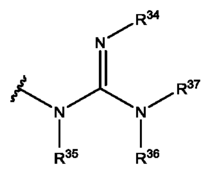

and

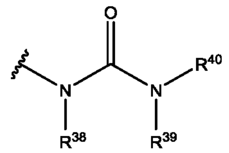

; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, or 5- to 10-membered heteroaryl is optionally substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5-to 10- membered heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C({=}O)R^{26}$, $-OS({=}O)_2R^{27}$, $-NR^{28}S({=}O)_2R^{29}$, $-OC({=}O)R^{30}$, $-S({=}O)R^{31}$, $-P({=}O)R^{32}R^{33}$;

$R^{c1}$, $R^{c2}$ are independently selected from hydrogen, halogen, $C_{1-4}$ alkyl and $-OR^5$;

or $R^{c1}$ and $R^{c2}$ together with the atoms to which they are attached form $C_{3-6}$ carbocyclyl, or 3- to 6- membered heterocyclyl;

each $R^1$ is independently selected from hydrogen, halogen, -CN, $-NO_2$, oxo, $C_{1-4}$ alkyl, $C_{2-4}$ alkenyl, $C_{2-4}$ alkynyl, $C_{3-6}$ carbocyclyl, 3- to 6- membered heterocyclyl, $C_{6-10}$ aryl, 5- to 10- membered heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C({=}O)R^{26}$, $-OS({=}O)_2R^{27}$, $-NR^{28}S({=}O)_2R^{29}$, $-OC({=}O)R^{30}$, $-S({=}O)R^{31}$, and $-P({=}O)R^{32}R^{33}$, wherein, the $C_{1-4}$ alkyl, $C_{2-4}$ alkenyl, $C_{2-4}$ alkynyl, $C_{3-6}$ carbocyclyl, 3-to 6- membered heterocyclyl, $C_{6-10}$-membered aryl, or 5- to 10- membered heteroaryl is optionally substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-6}$ carbocyclyl, 3- to 6- membered heterocyclyl, $C_{6-10}$ aryl, 5- to 10- membered heteroaryl, $-OR^5$, $-SR^6$, $-NR^7R^8$, $-(C{=}O)R^9$, $-C({=}O)OR^{10}$ and $-C({=}O)NR^{11}R^{12}$;

X is selected from -O-, $-N(R^{1X})-$, -S-, -C(=O)-, -C(=O)O-, $-C({=}O)N(R^{2X})-$, $-S({=}O)_2-$, $-S({=}O)_2O-$, $-S({=}O)_2N(R^{3X})-$, -S(=O)-, -S(=O)O-, $-S({=}O)N(R^{4X})-$, $-OS({=}O)_2O-$,

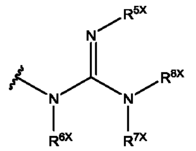 and 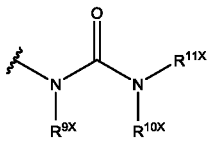 ;

$R^{1X}$, $R^{2X}$, $R^{3X}$, $R^{4X}$, $R^{5X}$, $R^{6X}$, $R^{7X}$, $R^{8X}$, $R^{9X}$, $R^{10X}$, $R^{11X}$ are independently selected from hydrogen, $C_{1-3}$ alkyl, $C_{3-6}$ cycloalkyl and 3- to 6- membered heterocyclyl;

M is selected from -O-, $-NR^{1a}-$ and $-CR^{1b}R^{1c}-$;

$R^{1a}$ is selected from hydrogen, $C_{1-3}$ alkyl, $C_{3-6}$ cycloalkyl, 3- to 6- membered heterocyclyl or absent;

$R^{1b}$, $R^{1c}$ are independently selected from hydrogen, $C_{1-3}$ alkyl, $C_{3-6}$ cycloalkyl, 3-to 6- membered heterocyclyl, $-OR^{1d}$, $-NR^{1e}R^{1f}$ or absent;

$R^{1d}$, $R^{1e}$, $R^{1f}$ are independently selected from hydrogen, $C_{1-3}$ alkyl, $C_{3-6}$ cycloalkyl and 3- to 6- membered heterocyclyl;

$Y_1$ is selected from $-NR^{3a}$ or $-CR^{3b}R^{3c}$;

$R^{3a}$ is selected from hydrogen, $C_{1-3}$ alkyl, $C_{3-6}$ cycloalkyl, 3- to 6- membered heterocyclyl or absent;

$R^{3b}$, $R^{3c}$ are independently selected from hydrogen, $C_{1-3}$ alkyl, $C_{3-6}$ cycloalkyl, 3-to 6- membered heterocyclyl or absent;

$Y_2$ is selected from $-NR^{4a}$ or $-CR^{4b}R^{4c}$;

$R^{4a}$ is selected from hydrogen, $C_{1-3}$ alkyl, $C_{3-6}$ cycloalkyl, 3- to 6- membered heterocyclyl or absent;

$R^{4b}$, $R^{4c}$ are independently selected from hydrogen, $C_{1-3}$ alkyl, $C_{3-6}$ cycloalkyl, 3-to 6- membered heterocyclyl or absent;

ring D is a $C_{6-10}$ aryl, a 5- to 10- membered heteroaryl, a $C_{3-8}$ carbocyclyl, or a 3-to 8- membered heterocyclyl; wherein, the $C_{3-8}$ carbocyclyl or a 3- to 8- membered heterocyclyl optionally contains a double bond;

each $R^4$ is independently selected from hydrogen, halogen, -CN, $-NO_2$, oxo, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C({=}O)OR^{10}$, $-C({=}O)NR^{11}R^{12}$, $-S({=}O)_2R^{13}$, $-S({=}O)_2OR^{14}$, $-S({=}O)_2NR^{15}R^{16}$, $-O(CH_2)_sOR^{17}$, $-O(CH_2)_sNR^{18}R^{19}$, $-NR^{20}(CH_2)_tNR^{21}R^{22}$, and $-NR^{23}(CH_2)_tOR^{24}$; wherein, the $C_{1-8}$ alkyl, $C_{2-8}$ alkenyl, $C_{2-8}$ alkynyl, $C_{3-8}$ carbocyclyl, 3- to 10-membered heterocyclyl, $C_{6-10}$ membered aryl, or 5- to 10- membered heteroaryl

is optionally substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, and 5- to 10- membered heteroaryl;

$R^5$, $R^6$, $R^7$, $R^8$, $R^9$, $R^{10}$, $R^{11}$, $R^{12}$, $R^{13}$, $R^{14}$, $R^{13}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, or $R^{40}$ is independently selected from hydrogen, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, and 5- to 10- membered heteroaryl; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, or 5- to 10- heteroaryl is optionally substituted with one or more substituents; the substituents are selected from hydrogen, halogen, -CN, $-NO_2$, oxo, -OH, $-NH_2$, $C_{1-4}$ alkyl, $C_{1-4}$ alkoxy, $C_{2-4}$ alkenyl, $C_{2-4}$ alkynyl, $C_{3-10}$ carbocyclyl, 3- to 10- membered heterocyclyl, $C_{6-10}$ aryl, and 5- to 10- membered heteroaryl;

n is 0, 1, 2 or 3;

p is 0, 1, 2, or 3;

q is 0, 1, 2, 3 or 4;

r is 0, 1, 2, 3 or 4;

s is 1, 2, 3 or 4; and

t is 1, 2, 3 or 4.

2. The compound or the pharmaceutically acceptable salt, deuterated substance, cis-trans isomer, tautomer, solvate, chelate, non-covalent complex or prodrug thereof of claim 1, **characterized in that** ring A is selected from a 6-membered heteroaryl or a 6- membered heterocyclyl; the 6-membered heterocyclyl contains at least one double bond.

3. The compound or the pharmaceutically acceptable salt, deuterated substance, cis-trans isomer, tautomer, solvate, chelate, non-covalent complex or prodrug thereof of any one of claims 1-2, **characterized in that** ring A is

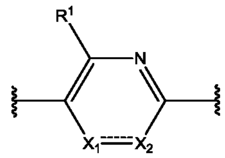

;

wherein, $X_1$ and $X_2$ are independently selected from $-NR^1-$ or $-CR^1R^1-$; - - - - - - is single bond or double bond.

4. The compound or the pharmaceutically acceptable salt, deuterated substance, cis-trans isomer, tautomer, solvate, chelate, non-covalent complex or prodrug thereof of any one of claims 1-3, **characterized in that** ring A is selected from

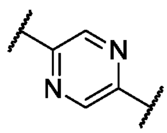

or

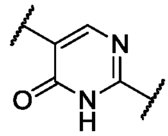

.

5. The compound or the pharmaceutically acceptable salt, deuterated substance, cis-trans isomer, tautomer, solvate, chelate, non-covalent complex or prodrug thereof of any one of claims 1-4, **characterized in that** ring D is

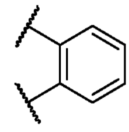, 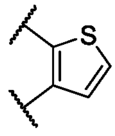,

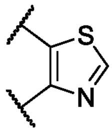, 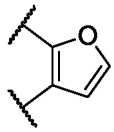, 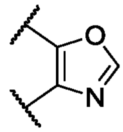, 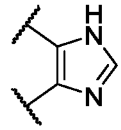, 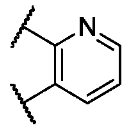 or 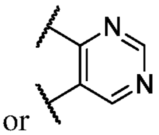.

**6.** The compound or the pharmaceutically acceptable salt, deuterated substance, cis-trans isomer, tautomer, solvate, chelate, non-covalent complex or prodrug thereof of any one of claims 1-5, **characterized in that** ring D is

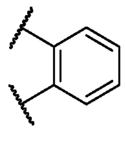.

**7.** The compound or the pharmaceutically acceptable salt, deuterated substance, cis-trans isomer, tautomer, solvate, chelate, non-covalent complex or prodrug thereof of any one of claims 1-6, **characterized in that** the compound is selected from Formula II:

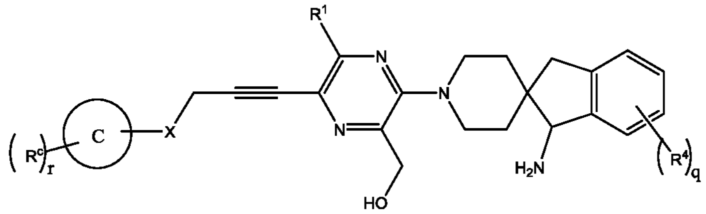

Formula II

wherein

ring C is selected from 3- to 10- membered saturated or partially saturated heterocyclyl, $C_{6-10}$ aryl and 5- to 10-membered heteroaryl;

each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$, $-OC_{2-6}$ alkenyl $C_{1-6}$ alkoxy, $-OC_{2-6}$ alkenyl $C_{6-10}$ aryl,

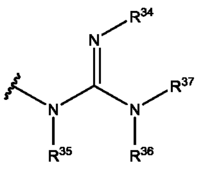 and 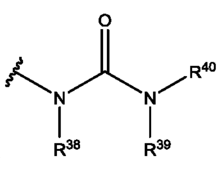

; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, or 5- to 10-membered heteroaryl is optionally substituted with one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$,

$-OC(=O)R^{30}$, $-S(=O)R^{31}$, and $-P(=O)R^{32}R^{33}$;

two $R^c$ with the atoms they are attached form $C_{3-8}$ carbocyclyl, 3- to 10-membered heterocyclyl, $C_{6-10}$ aryl, or 5- to 10- membered heteroaryl; wherein the $C_{3-8}$ carbocyclyl, 3- to 10- membered heterocyclyl, $C_{6-10}$ membered aryl, or 5- to 10-membered heteroaryl is optionally substituted by one or more substituents; the substituents are independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, $-P(=O)R^{32}R^{33}$, $-OC_{2-6}$ alkenyl $C_{1-6}$ alkoxy, $-OC_{2-6}$ alkenyl $C_{6-10}$ aryl,

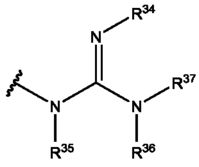 and 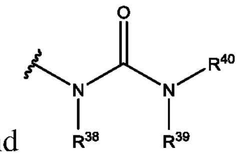 ;

; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ membered aryl, or 5- to 10-membered heteroaryl is optionally substituted with one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- member heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$, and $-P(=O)R^{32}R^{33}$;

$R^1$ is selected from hydrogen, halogen, -CN, $-NO_2$, oxo, $C_{1-4}$ alkyl, $C_{2-4}$ alkenyl, $C_{2-4}$ alkynyl, $-OR^5$, $-NR^7R^8$, $-O(CH_2)_sOR^{17}$, $-O(CH_2)_sNR^{18}R^{19}$, $-NR^{20}(CH_2)_tNR^{21}R^{22}$, and $-NR^{23}(CH_2)_tOR^{24}$; wherein, the $C_{1-4}$ alkyl, $C_{2-4}$ alkenyl or $C_{2-4}$ alkynyl is optionally substituted by one or more substituents; the substituent is selected from hydrogen, halogen, -CN, $-NO_2$, oxo, $C_{1-4}$ alkyl, $C_{2-4}$ alkenyl, $C_{2-4}$ alkynyl, $C_{3-6}$ carbocyclyl, 3- to 6- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$ and $-C(=O)NR^{11}R^{12}$;

X is selected from -O-, $-N(R^{1X})-$, -S-, -C(=O)-, -C(=O)O-, $-C(=O)N(R^{2X})-$, $-S(=O)_2-$, $-S(=O)_2O-$, $-S(=O)_2N(R^{3X})-$, -S(=O)-, -S(=O)O-, $-S(=O)N(R^{4X})-$, $-OS(=O)_2O-$,

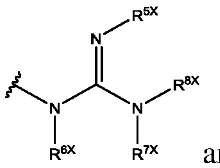 and 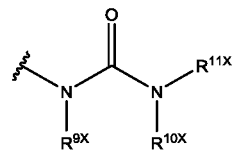 ;

$R^{1X}$, $R^{2X}$, $R^{3X}$, $R^{4X}$, $R^{5X}$, $R^{6X}$, $R^{7X}$, $R^{8X}$, $R^{9X}$, $R^{10X}$, and $R^{11X}$ are independently selected from hydrogen, $C_{1-3}$ alkyl, $C_{3-6}$ cycloalkyl and 3- to 6- membered heterocyclyl;

each $R^4$ is independently selected from hydrogen, halogen, -CN, $-NO_2$, oxo, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, and $-NR^{23}(CH_2)_tOR^{24}$, wherein, the $C_{1-8}$ alkyl, $C_{2-8}$ alkenyl, $C_{2-8}$ alkynyl, $C_{3-8}$ carbocyclyl, 3- to 10-membered heterocyclyl, $C_{6-10}$ membered aryl, or 5- to 10- membered heteroaryl is optionally substituted by one or more substituents; the substituents are selected from hydrogen, halogen, -CN, $-NO_2$, oxo, $C_{1-4}$ alkyl, $C_{2-4}$ alkenyl, $C_{2-4}$ alkynyl, $C_{3-10}$ carbocyclyl, 3- to 10- membered heterocyclyl, $C_{6-10}$ aryl, 5- to 10- membered heteroaryl, $-OR^5$, $-SR^6$, $-NR^7R^8$, $-(C=O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, and $-NR^{23}(CH_2)_tOR^{24}$;

$R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, and $R^{40}$ are independently selected from hydrogen, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, and 5- to 10- membered heteroaryl; wherein, the $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, or 5- to 10- heteroaryl is optionally substituted with one or more substituents; the substituents are selected from hydrogen, halogen, -CN, $-NO_2$, oxo, -OH, $-NH_2$, $C_{1-4}$ alkyl, $C_{1-4}$ alkoxy, $C_{2-4}$

alkenyl, $C_{2-4}$ alkynyl, $C_{3-10}$ carbocyclyl, 3- to 10- membered heterocyclyl, $C_{6-10}$ aryl, and 5- to 10- membered heteroaryl;
q is 0, 1, 2, 3 or 4;
r is 0, 1, 2, 3 or 4;
s is 1, 2, 3 or 4; and
t is 1, 2, 3 or 4.

**8.** The compound or the pharmaceutically acceptable salt, deuterated substance, cis-trans isomer, tautomer, solvate, chelate, non-covalent complex or prodrug thereof of any one of claims 1-7, wherein ring C is selected from phenyl, pyrrolyl, furyl, thienyl, oxazolyl, imidazolyl, pyrazolyl, thiazolyl, pyridyl, pyrimidinyl, pyridazine base, pyrazinyl, pyridone, indolyl, benzoxazolyl, indazolyl, benzimidazolyl, benzotriazolyl, pyrrolopyridyl, quinolyl, naphthyl, dihydroisophenyl furanyl, benzobisoxazolyl, tetrahydroquinolinyl, dihydropyrrolopyridyl, dihydronaphthyridinyl, dihydropyridopyrimidinonyl, isoindolinyl, dihydropyrazolopyrimidine base, cyclohexenyl, tetrahydrobenzazepine, dihydrobenzimidazolone, indolinone, isoindolinone, dihydroquinolinone, dihydroisoquinolinone, benzene oxazinone, quinolinone, benzobisoxazolyl, dihydrobenzodioxin, pyridinone, and indazolyl.

**9.** The compound or the pharmaceutically acceptable salt, deuterated substance, cis-trans isomer, tautomer, solvate, chelate, non-covalent complex or prodrug thereof of any one of claims 1-8, **characterized in that** ring C is selected from

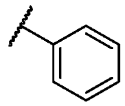,

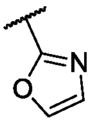, 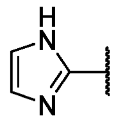, 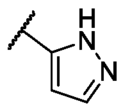, 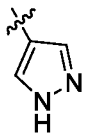, 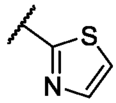, 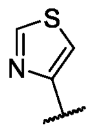, 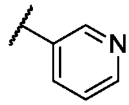,

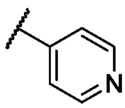 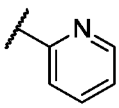, 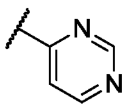, 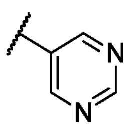, 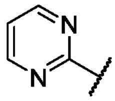, 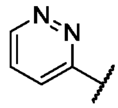, 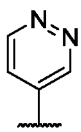, 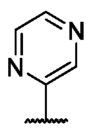,

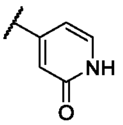, 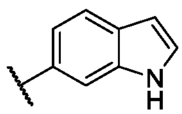, 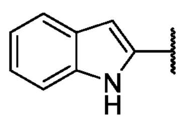, 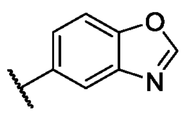, 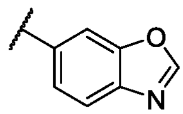,

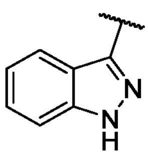, 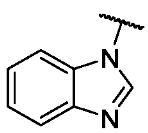, 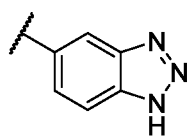, 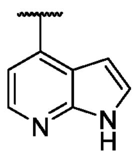, 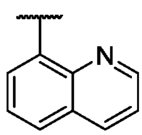, 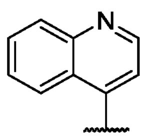,

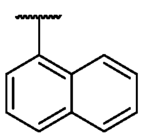, 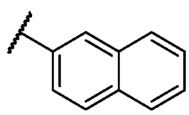, 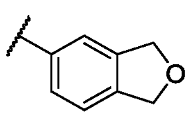, 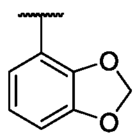, 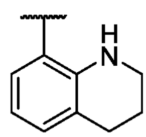, 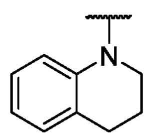,

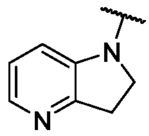, 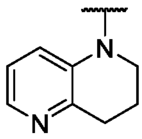, 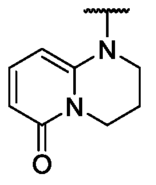, 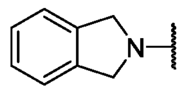, 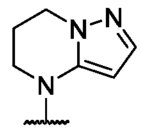, 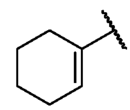,

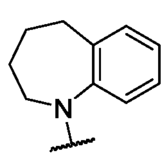, 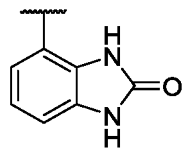, 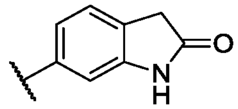, 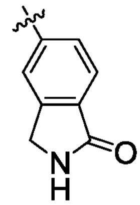, 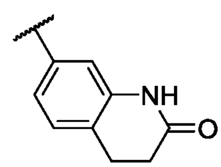,

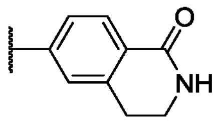, 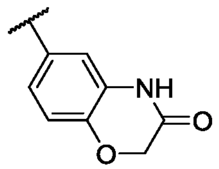, 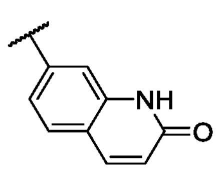, 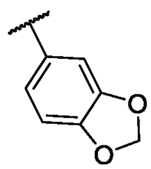, 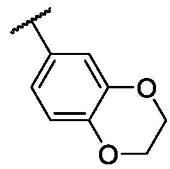,

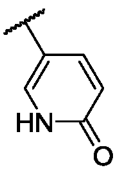, 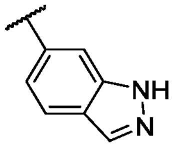 and 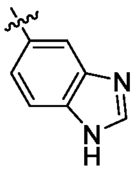.

**10.** The compound or the pharmaceutically acceptable salt, deuterated substance, cis-trans isomer, tautomer, solvate, chelate, non-covalent complex or prodrug thereof of any one of claims 1-9, **characterized in that** ring C is

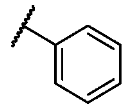.

**11.** The compound or the pharmaceutically acceptable salt, deuterated substance, cis-trans isomer, tautomer, solvate, chelate, non-covalent complex or prodrug thereof of any one of claims 1-10, wherein each $R^c$ is independently selected from hydrogen, halogen, -CN, $-NO_2$, oxo, $-SF_5$, $C_{1-8}$ alkyl, $C_{2-8}$ alkenyl, $C_{2-8}$ alkynyl, $C_{3-8}$ carbocyclyl, 3- to 10- membered heterocyclyl, $C_{6-10}$ aryl, 5- to 10- membered heteroaryl, $-OR^5$, $-SR^6$, $-NR^7R^8$, $-(C{=}O)R^9$, $-C(=O)OR^{10}$, $-C(=O)NR^{11}R^{12}$, $-S(=O)_2R^{13}$, $-S(=O)_2OR^{14}$, $-S(=O)_2NR^{15}R^{16}$, $-O(CH_2)_sOR^{17}$, $-O(CH_2)_sNR^{18}R^{19}$, $-NR^{20}(CH_2)_tNR^{21}R^{22}$, $-NR^{23}(CH_2)_tOR^{24}$, $-NR^{25}C(=O)R^{26}$, $-OS(=O)_2R^{27}$, $-NR^{28}S(=O)_2R^{29}$, $-OC(=O)R^{30}$, $-S(=O)R^{31}$ and $-P(=O)R^{32}R^{33}$.

**12.** The compound or the pharmaceutically acceptable salt, deuterated substance, cis-trans isomer, tautomer, solvate, chelate, non-covalent complex or prodrug thereof of any one of claims 1-11, wherein each $R^c$ is independently selected from hydrogen, oxo, $-CH_3$, $-OCH_3$, -OH, $-OCF_3$, -CN, -F, -Cl, $-CF_3$, $-NH_2$, $-NHCH_3$, $-NH(C{=}O)CH_3$, $-CH_2OH$, $-C(=O)CH_3$, $-C(=O)NH_2$, $-C(=O)NHCH_3$, $-C(=O)N(CH_3)_2$, -COOH, -COOEt, $-S(=O)_2CH_3$, $-S(=O)_2NH_2$, $-P(=O)(CH_3)_2$, $-CH_2OCH_3$, $-CH_2C(=O)NH_2$,

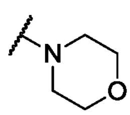, 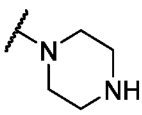, 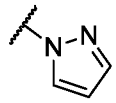, 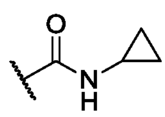, 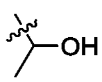, 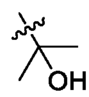, 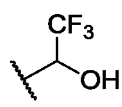,

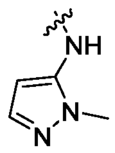 and 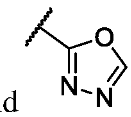 .

**13.** The compound or the pharmaceutically acceptable salt, deuterated substance, cis-trans isomer, tautomer, solvate, chelate, non-covalent complex or prodrug thereof of any one of claims 1-12, wherein each $R^c$ is independently selected from hydrogen, $-CH_3$, -F, -Cl, $-NH(C{=}O)CH_3$, $-C(=O)NH_2$ and $-C(=O)NHCH_3$.

**14.** The compound or the pharmaceutically acceptable salt, deuterated substance, cis-trans isomer, tautomer, solvate, chelate, non-covalent complex or prodrug thereof of any one of claims 1-13, **characterized in that** $R^1$ is selected from hydrogen, halogen and $C_{1-4}$ alkyl.

**15.** The compound or the pharmaceutically acceptable salt, deuterated substance, cis-trans isomer, tautomer, solvate, chelate, non-covalent complex or prodrug thereof of any one of claims 1-14, **characterized in that** $R^1$ is hydrogen.

**16.** The compound or the pharmaceutically acceptable salt, deuterated substance, cis-trans isomer, tautomer, solvate, chelate, non-covalent complex or prodrug thereof of any one of claims 1-15, wherein each $R^4$ is independently selected from hydrogen, halogen, oxo, $C_{1-8}$ alkyl and $-OR^5$.

**17.** The compound or the pharmaceutically acceptable salt, deuterated substance, cis-trans isomer, tautomer, solvate, chelate, non-covalent complex or prodrug thereof of any one of claims 1-16, wherein each $R^4$ is independently selected from hydrogen or $-OCH_3$.

**18.** The compound or a pharmaceutically acceptable salt, deuterated substance, cis-trans isomer, tautomer, solvate, chelate, non-covalent complex or prodrug thereof of any one of claims 1-17, wherein X is -O-.

**19.** The compound or the pharmaceutically acceptable salt, deuterated substance, cis-trans isomer, tautomer, solvate, chelate, non-covalent complex or prodrug thereof of any one of claims 1-17, wherein X is $-N(R^{1X})-$, wherein $R^{1X}$ is selected from hydrogen or methyl.

**20.** The compound or the pharmaceutically acceptable salt, deuterated substance, cis-trans isomer, tautomer, solvate, chelate, non-covalent complex or prodrug thereof of any one of claims 1-19, wherein q is 0 or 1.

**21.** The compound or the pharmaceutically acceptable salt, deuterated substance, cis-trans isomer, tautomer, solvate, chelate, non-covalent complex or prodrug thereof of any one of claims 1-20, wherein r is 0, 1 or 2.

**22.** The compound or the pharmaceutically acceptable salt, deuterated substance, cis-trans isomer, tautomer, solvate, chelate, non-covalent complex or prodrug thereof of any one of claims 1-6, **characterized in that** the compound is selected from any of the following Formulas III-V:

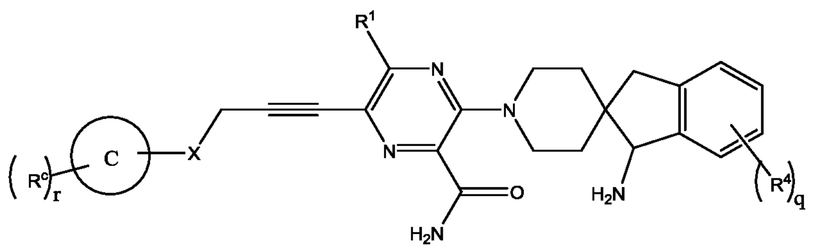

Formula III

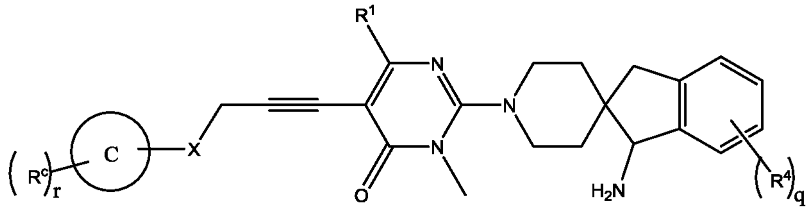

Formula IV

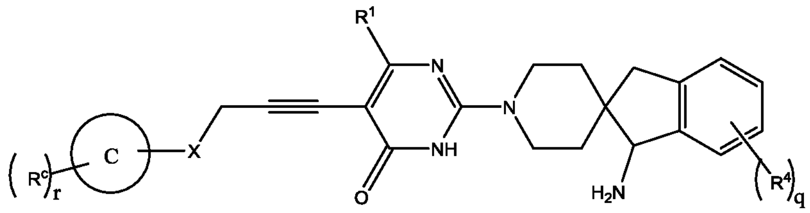

Formula V.

**23.** The compound or the pharmaceutically acceptable salt, deuterated compound, cis-trans isomer, tautomer, solvate, chelate, non-covalent complex or prodrug thereof of claim 1, **characterized in that** the compound is selected from:

(S)-(3-(1-amino-1,3-dihydrospiro[indene-2,4'-piperidin]-1'-yl)-6-(3-phenoxyprop-1-yn-1-yl)pyrazin-2-yl)methanol;
(S)-(3-(1-amino-1,3-dihydrospiro[indene-2,4'-piperidin]-1'-yl)-6-(3-(3-methoxybenzene)oxy)prop-1-yn-1-yl)pyrazin-2-yl)methanol;
(S)-(3-(1-amino-1,3-dihydrospiro[indene-2,4'-piperidin]-1'-yl)-6-(3-(3-Aminophenoxy))prop-1-yn-1-yl)pyrazin-2-yl)methanol;
(S)-N-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-(3-(1-amino-1,3-dihydrospiro[indene-2,4'-piperidin]-1'-yl)-6-(3-(3-chloro-2-fluoro)phenoxy)prop-1-yn-1-yl)pyrazin-2-yl)methanol;
(S)-(3-(1-amino-1,3-dihydrospiro[indene-2,4'-piperidin]-1'-yl)-6-(3-((3-methoxyphenyl)thio)prop-1-yn-1-yl)pyrazin-2-yl)methanol;
(S)-(3-(1-amino-1,3-dihydrospiro[indene-2,4'-piperidin]-1'-yl)-6-(3-(phenylamino)prop-1-yn-1-yl)pyrazin-2-yl)methanol;
(S)-N-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-(3-(1-amino-1,3-dihydrospiro[indene-2,4'-piperidin]-1'-yl)-6-(3-(4-methoxybenzene)oxy)prop-1-yn-1-yl)pyrazin-2-yl)methanol;
(S)-(3-(1-amino-1,3-dihydrospiro[indene-2,4'-piperidin]-1'-yl)-6-(3-(2-methoxybenzene)oxy)prop-1-yn-1-yl)pyrazin-2-yl)methanol;
(S)-(3-(1-amino-1,3-dihydrospiro[indene-2,4'-piperidin]-1'-yl)-6-(3-(3,4-dimethylform)oxyphenoxy)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)benzonitrile;
(S)-(3-(1-amino-1,3-dihydrospiro[indene-2,4'-piperidin]-1'-yl)-6-(3-(3-(trifluoromethyl)phenoxy)prop-1-yn-1-yl)pyrazin-2-yl)methanol;
(S)-(3-(1-amino-1,3-dihydrospiro[indene-2,4'-piperidin]-1'-yl)-6-(3-(4-(trifluoromethyl)phenoxy)prop-1-yn-1-yl)pyrazin-2-yl)methanol;
(S)-(3-(1-amino-1,3-dihydrospiro[indene-2,4'-piperidin]-1'-yl)-6-(3-(3-(trifluoromethyl)oxy)phenoxy)prop-1-yn-1-yl)pyrazin-2-yl)methanol;
(S)-1-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)ethan-1-one;
(S)-4-((3-(5-(1-amino-1,3-dihydrospiro[indene-2,4'-piperidin]-1'-yl)-6-(hydroxymethyl))pyrazin-2-yl)prop-2-yn-1-yl)oxy)benzamide;
(S)-(3-(1-amino-1,3-dihydrospiro[indene-2,4'-piperidin]-1'-yl)-6-(3-(pyridin-4-oxyl)prop-1-yn-1-yl)pyrazin-2-yl)methanol;
(S)-(3-(1-amino-1,3-dihydrospiro[indene-2,4'-piperidin]-1'-yl)-6-(3-((2-aminopyrimidine-5-yl)oxy)prop-1-yn-1-yl)pyrazin-2-yl)methanol;
(S)-(3-(1-amino-1,3-dihydrospiro[indene-2,4'-piperidin]-1'-yl)-6-(3-(4-morpholinoloxy)yl)prop-1-yn-1-yl)pyrazin-2-yl)methanol;
(S)-1-(4-(4-((3-(5-(1-amino-1,3-dihydrospiro[indene-2,4'-piperidin]-1'-yl)-6-(hydroxymethyl)pyrazin-2-yl)prop-2-yn-1-yl)oxy)piperazin-1-yl)ethan-1-one;
(S)-7-((3-(5-(1-amino-1,3-dihydrospiro[indene-2,4'-piperidin]-1'-yl)-6-(hydroxymethyl))pyrazin-2-yl)prop-2-yn-1-yl)oxy)-3,4-dihydroquinolin-2(1H)-one;
(S)-6-((3-(5-(1-amino-1,3-dihydrospiro[indene-2,4'-piperidin]-1'-yl)-6-(hydroxymethyl))pyrazin-2-yl)prop-2-yn-1-yl)oxy)-2Hbenzo[b][1,4]oxazin-3(4H)-one;
(S)-7-((3-(5-(1-amino-1,3-dihydrospiro[indene-2,4'-piperidin]-1'-yl)-6-(hydroxymethyl))pyrazin-2-yl)prop-2-yn-1-yl)oxy)quinolin-2(1H)-one;
(S)-6-((3-(5-(1-amino-1,3-dihydrospiro[indene-2,4'-piperidin]-1'-yl)-6-(hydroxymethyl))pyrazin-2-yl)prop-2-yn-1-yl)oxy)indol-2-one;
(S)-(3-(1-amino-1,3-dihydrospiro[indene-2,4'-piperidin]-1'-yl)-6-(3-((2-Aminopyridine-4-yl)oxy)prop-1-yn-1-yl)pyrazin-2-yl)methanol;
(S)-3-((3-(5-(1-amino-1,3-dihydrospiro[indene-2,4'-piperidin]-1'-yl)-6-(hydroxymethyl))pyrazin-2-yl)prop-2-yn-1-yl)oxy)benzamide;
(S)-(3-(1-amino-1,3-dihydrospiro[indene-2,4'-piperidin]-1'-yl)-6-(3-(benzo[d][1,3]Dioxyol-5-oxy)prop-1-yn-1-yl)pyrazin-2-yl)methanol;
(S)-(3-(1-amino-1,3-dihydrospiro[indene-2,4'-piperidin]-1'-yl)-6-(3-((((2,3-dihydrobenzo[b][1,4]dioxin-6-yl)oxy)prop-1-yn-1-yl)pyrazin-2-yl)methanol;
(S)-5-((3-(5-(1-amino-1,3-dihydrospiro[indene-2,4'-piperidin]-1'-yl)-6-(hydroxymethyl)pyrazin-2-yl)prop-2-yn-1-yl)oxy)-1-methylpyridin-2(1H)-one;
(S)-(6-(3-((1H-Indolazol-6-yl)oxy)prop-1-yn-1-yl)-3-(1-amino-1,3-dihydro spiro[indene-2,4'-piperidin]-1'-yl)pyrazin-2-yl)methanol;
(S)-4-((3-(5-(1-amino-1,3-dihydrospiro[indene-2,4'-piperidin]-1'-yl)-6-(hydroxymethyl))pyrazin-2-yl)prop-2-yn-1-yl)oxy)benzenesulfonamide;
(S)-3-((3-(2-(1-amino-1,3-dihydrospiro[indene-2,4'-piperidin]-1'-yl)-6-oxo-1 ,6-dihydropyrimidin-5-yl)prop-2-yn-1-yl)oxy)benzamide;
(S)-N-(3-((3-(2-(1-amino-1,3-dihydrospiro[indene-2,4'-piperidin]-1'-yl)-1-methyl)yl-6-oxo-1,6-dihydropyrimidin-5-yl)prop-2-yn-1-yl)oxy)phenyl)acetamide;
(S)-3-((3-(5-(1-amino-1,3-dihydrospiro[indene-2,4'-piperidin]-1'-yl)-6-(hydroxymethyl))pyrazin-2-yl)prop-2-yn-1-yl)oxy)-N-methylbenzamide;
(S)-3-((3-(5-(1-amino-1,3-dihydrospiro[indene-2,4'-piperidin]-1'-yl)-6-(hydroxymethyl))pyrazin-2-yl)prop-2-yn-1-yl)oxy)-N,N-dimethylbenzamide;
(S)-(3-(1-amino-1,3-dihydrospiro[indene-2,4'-piperidin]-1'-yl)-6-(3-(3-(methylthio)acyl)phenoxy)prop-1-yn-1-yl)pyrazin-2-yl)methanol;
(S)-(3-((3-(5-(1-amino-1,3-dihydrospiro[indene-2,4'-piperidin]-1'-yl)-6-(hydroxymethyl)yl)pyrazin-2-yl)prop-2-yn-1-yl)oxy)phenyl)dimethylphosphine oxide;
(S)-4-((3-(5-(1-amino-1,3-dihydrospiro[indene-2,4'-piperidin]-1'-yl)-6-(hydroxymethyl))pyrazin-2-yl)prop-2-yn-1-yl)oxy)-1-methylpyridin-2(1H)-one;
(S)-4-((3-(5-(1-amino-1,3-dihydrospiro[indene-2,4'-piperidin]-1'-yl)-6-(hydroxymethyl))pyrazin-2-yl)prop-2-yn-1-yl)oxy)-N-picoline amide;
(S)-6-(3-(3-acetamidophenoxy)prop-1-yn-1-yl)-3-(1-amino-1,3-dihydrospiro[indene-2,4]'-piperidin]-1'-yl)pyrazine-2-carboxamide;
(S)-3-(1-amino-1,3-dihydrospiro[indene-2,4'-piperidin]-1'-yl)-6-(3-((3-oxoisooctanol)-5-yl)oxy)prop-1-yn-1-yl)pyrazine-2-carboxamide;
(S)-5-(5-(1-amino-1,3-dihydrospiro[indene-2,4'-piperidin]-1'-yl)-6-(hydroxymethyl)pyrazine-2-yl)-1-phenylpent-4-yn-1-one;
(S)-4-(5-(1-amino-1,3-dihydrospiro[indene-2,4'-piperidin]-1'-yl)-6-(hydroxymethyl)pyrazine-2-yl)-N-phenyl-3-ynamide;
(S)-N-(3-(5-(1-amino-1,3-dihydrospiro[indene-2,4'-piperidin]-1'-yl)-6-(hydroxymethyl))pyrazin-2-yl)prop-2-yn-1-yl)benzamide;
(S)-(3-(1-amino-1,3-dihydrospiro[indene-2,4'-piperidin]-1'-yl)-6-(4-(phenylamino)butyl-1-yn-1-yl)pyrazin-2-yl)methanol;
(S)-(3-(1-amino-1,3-dihydrospiro[indene-2,4'-piperidin]-1'-yl)-6-(3-((3,4-dimethyl oxyphenyl)amino)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)amino)benzamide;
(S)-(3-(1-amino-1,3-dihydrospiro[indene-2,4'-piperidin] -1'-yl)-6-(3-((2-methoxypyridine-4-yl)oxy)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-2-fluorobenzamide;
(S)-5-((3-(5-(1-amino-1,3-dihydrospiro[indene-2,4'-piperidin]-1'-yl)-6-(hydroxymethyl)pyrazin-2-yl)prop-2-yn-1-yl)oxy)isoindolin-1-one;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)-3,4-dihydroisoquinolin-1(2H)-one;
(S)-3-((3-(5-(1-amino-1,3-dihydrospiro[indene-2,4'-piperidin]-1'-yl)-6-(hydroxymethyl)pyrazin-2-yl)prop-2-yn-1-yl)oxy)benzonitrile;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-2-chlorobenzamide;
(S)-4-((3-(5-(1-amino-1,3-dihydrospiro[indene-2,4'-piperidin]-1'-yl)-6-(hydroxymethyl)pyrazin-2-yl)prop-2-yn-1-yl)(methyl) amino)benzamide;
(S)-5-((3-(5-(1-amino-1,3-dihydrospiro[indene-2,4'-piperidin]-1'-yl)-6-(hydroxymethyl)pyrazin-2-yl)prop-2-yn-1-yl)oxy)pyridineamide;
(S)-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)dimethylphosphine oxide;
(S)-4-((3-(5-(1-amino-6-methoxy-1,3-dihydrospiro[indene-2,4'-piperidin]-1'-yl)-6-(hydroxymethyl)pyrazin-2-yl)prop-2-yn-1-yl)oxy)benzamide;
(S)-2-(3-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-(3-(1-amino-1,3-dihydrospiro[inden-2,4'-piperidin]-1'-yl)-6-(3-(oxazol-2-ylamino))prop-1-yn-1-yl)pyrazin-2-yl)methanol;
(S)-(3-(1-amino-1,3-dihydrospiro[indene-2,4'-piperidin]-1'-yl)-6-(3-((1-methyl-1H-pyrazol-5-yl)amino)propyly-1-yn-1-yl)pyrazin-2-yl)methanol;
(S)-4-((3-(5-(1-amino-1,3-dihydrospiro[inden-2,4'-piperidin]-1'-yl)pyrazin-2-yl)prop-2-yn-1-yl)oxy)-2-chlorobenzamide;
(S)-(3-(1-amino-1,3-dihydrospiro[indene-2,4'-piperidin]-1'-yl)-6-(3-((6-(trifluoromethyl)yl)pyridin-3-yl)amino)prop-1-yn-1-yl)pyrazin-2-yl)methanol;
(S)-7-((3-(5-(1-amino-1,3-dihydrospiro[indene-2,4'-piperidin]-1'-yl)-6-(hydroxymethyl)pyrazin-2-yl)prop-2-yn-1-yl)amino)-3,4-dihydroquinolin-2(1H)-one;
(S)-N-(3-((3-(5-(1-amino-1,3-dihydrospiro[indene-2,4'-piperidin]-1'-yl)-6-(hydroxymethyl)pyrazin-2-yl)prop-2-yn-1-yl)amino)phenyl)acetamide;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)isoindol-1-one;
(S)-(6-(3-((1H-Indazol-5-yl)oxy)prop-1-yn-1-yl)-3-(1-amino-1,3-dihydrospiro[indene-2,4'-piperidin]-1'-yl)pyrazin-2-yl)methanol;
(S)-(3-(1-amino-1,3-dihydrospiro[indene-2,4'-piperidin]-1'-yl)-6-(3-((2-(methylAmino)pyrimidin-5-yl)oxy)prop-1-yn-1-yl)pyrazin-2-yl)methanol;
(S)-(3-(1-amino-1,3-dihydrospiro[inden-2,4'-piperidin]-1'-yl)-6-(3-((2-Aminobenzo[d]oxazol-5-yl)oxy)prop-1-yn-1-yl)pyrazin-2-yl)methanol;
(S)-(3-(1-amino-1,3-dihydrospiro[indene-2,4'-piperidin]-1'-yl)-6-(3-((6-(trifluoromethyl)yl)pyridin-3-yl)oxy)prop-1-yn-1-yl)pyrazin-2-yl)methanol;
(S)-2-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)acetamide;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-N-methylbenzamide;
(S)-(3-(1-amino-1,3-dihydrospiro[indene-2,4'-piperidin]-1'-yl)-6-(3-((2-methyl-1H-benzo[d]imidazol-5-yl)oxy)prop-1-yn-1-yl)pyrazin-2-yl)methanol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-2-methylbenzamide;
(S)-6-((3-(5-(1-amino-1,3-dihydrospiro[indene-2,4'-piperidin]-1'-yl)-6-(hydroxymethyl)pyrazin-2-yl)prop-2-yn-1-yl)oxy)pyridin-3-ol;
(S)-4-((3-(5-(1-amino-1,3-dihydrospiro[indene-2,4'-piperidin]-1'-yl)-6-(hydroxymethyl)pyrazin-2-yl)prop-2-yn-1-yl)oxy)-N-cyclopropylbenzamide;
(S)-(3-(1-amino-1,3-dihydrospiro[indene-2,4'-piperidin]-1'-yl)-6-(3-(4-((1-methyl)yl)-1H-pyrazol-5-yl)amino)phenoxy)prop-1-yn-1-yl)pyrazin-2-yl)methanol;
1-(4-((3-(5-((S)-1-amino-1,3-dihydrospiro[indene-2,4'-piperidin]-1'-yl)-6-(hydroxyl)methyl)pyrazin-2-yl)prop-2-yn-1-yl)oxy)phenyl)-2,2,2-trifluoroethane-1-ol;
1-(4-((3-(5-((S)-1-amino-1,3-dihydrospiro[indene-2,4'-piperidin]-1'-yl)-6-(hydroxyl)methyl)pyrazin-2-yl)prop-2-yn-1-yl)oxy)phenyl)ethan-1-ol;
(S)-2-(4-((3-(5-(1-amino-1,3-dihydrospiro[indene-2,4'-piperidin]-1'-yl)-6-(hydroxymethyl)pyrazin-2-yl)prop-2-yn-1-yl)oxy)phenyl)propan-2-ol;
(S)-3-((3-(5-(1-amino-1,3-dihydrospiro[indene-2,4'-piperidin]-1'-yl)-6-(hydroxymethyl)pyrazin-2-yl)prop-2-yn-1-yl)amino)benzamide;
(S)-2-(4-((3-(5-(1-amino-1,3-dihydrospiro[indene-2,4'-piperidin]-1'-yl)-6-(hydroxymethyl)pyrazin-2-yl)prop-2-yn-1-yl)oxy)-1H-pyrazol-1-yl)acetamide;
(R)-4-((3-(5-(3-amino-3H-spiro[benzofuran-2,4'-piperidin]-1'-yl)-6-(hydroxymethyl)pyrazine-2-yl)prop-2-yn-1-yl)oxy)benzamide;
(S)-4-((3-(5-(5-amino-5,7-dihydrospiro[cyclopenta[b]pyridin-6,4'-piperidin]-1'-yl)-6-(hydroxymethyl)pyrazin-2-yl)prop-2-yn-1-yl)oxy)benzamide;
(S)-4-((3-(5-(4-amino-4,6-dihydrospiro[cyclopenta[d]thiazol-5,4'-piperidin]-1'-yl)-6-(Hydroxymethyl)pyrazin-2-yl)prop-2-yn-1-yl)oxy)benzamide;
(S)-4-((3-(5-(4-amino-4,6-dihydrospiro[cyclopenta[d]oxazol-5,4'-piperidin]-1'-yl)-6-(hydroxymethyl)pyrazin-2-yl)prop-2-yn-1-yl)oxy)benzamide; and
(S)-4-((3-(5-(4-amino-4,6-dihydro-1H-spiro[cyclopenta[d]imidazol-5,4'-piperidin]-1'-yl)-6-(hydroxymethyl)pyrazin-2-yl)prop-2-yn-1-yl)oxy)benzamide.

**24.** A pharmaceutical composition comprising a therapeutically effective amount of the compound of any one of claims 1-23 and at least one pharmaceutically acceptable adjuvant, wherein a mass ratio of the compound to the adjuvant is 0.0001: 1-10.

**25.** Use of the compound of any one of claims 1-23 or the pharmaceutical composition of claim 24 in the preparation of a medicament.

**26.** The use of claim 25, wherein the medicament is used for treating, preventing, delaying or preventing cancer, cancer metastasis, cardiovascular disease, immune disease, fibrosis or eye disease.

**27.** The use of claim 25, wherein the medicament is used for treating a disease mediated by SHP2; preferably, the disease is cancer.

**28.** The use of claim 27, wherein the cancer is selected from Noonan syndrome, leopard spot syndrome, juvenile myelomonocytic leukemia, neuroblastoma, melanoma, head and neck squamous cell carcinoma, acute myeloid leukemia, breast cancer, esophageal tumor, lung cancer, colon cancer, head cancer, gastric cancer, lymphoma, glioblastoma, and pancreatic cancer, or a combination thereof.

**29.** A method of treating and/or preventing a disease mediated by SHP2, comprising administering to a subject a therapeutically effective amount of at least one compound of any one of claims 1-23 or pharmaceutical composition.

**30.** A method of treating cancer comprising administering to a subject a therapeutically effective amount of at least one compound of any one of claims 1-23 or pharmaceutical composition.

**31.** The method of claim 30, wherein the cancer is selected from Noonan syndrome, leopard spot syndrome, juvenile myelomonocytic leukemia, neuroblastoma, melanoma, head and neck squamous cells carcinoma, acute myeloid leukemia, breast cancer, esophageal tumor, lung cancer, colon cancer, head cancer, gastric cancer, lymphoma, glioblastoma, and pancreatic cancer, or a combination thereof.

**32.** The method of claim 29 or 30, wherein the subject is human.

**INTERNATIONAL SEARCH REPORT**

International application No.
**PCT/CN2021/107808**

**A. CLASSIFICATION OF SUBJECT MATTER**

C07D 401/14(2006.01)i; C07D 491/10(2006.01)i; C07D 413/14(2006.01)i; C07D 513/10(2006.01)i; C07D 471/20(2006.01)i; C07D 487/04(2006.01)i; A61K 31/497(2006.01)i; A61P 35/02(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

WPI, STN, EPODOC, CNPAT, CNKI, 万方, 百度学术, 贝达药业, 吴颢, 吴文茂, 李玲, 张展, 王丰, 袁丁, 吴云飞, 陈强, 酪氨酸磷酸酶, 肿瘤, 癌症, 结肠癌, 二氢螺, 哌啶, 吡嗪, 炔, 吲哚, SHP, inhibitor, SRC, phosphatase, tyrosine, tumor, cancer, dihydrospirocycl+, pyrazin+, alkyne, indol+, structural formula search in STN

**C. DOCUMENTS CONSIDERED TO BE RELEVANT**

| Category* | Citation of document, with indication, where appropriate, of the relevant passages | Relevant to claim No. |
|---|---|---|
| X | WO 2019183367 A1 (RELAY THERAPEUTICS, INC. et al.) 26 September 2019 (2019-09-26)<br>abstract and description pages 22-24 and page 81 | 1-32 |
| A | CN 105916845 A (NOVARTIS AG) 31 August 2016 (2016-08-31)<br>abstract and description paragraphs [0008]-[0010] and [0531]-[0533] | 1-32 |
| A | WO 2020063760 A1 (JACOBIO PHARMACEUTICALS CO., LTD.) 02 April 2020 (2020-04-02)<br>abstract and description pages 1-4 and page 90 | 1-32 |
| A | WO 2019118909 A1 (REVOLUTION MEDICINES, INC.) 20 June 2019 (2019-06-20)<br>abstract and description paragraphs [0008]-[0009] and paragraph [00176] | 1-32 |
| A | CN 110143949 A (BEIJING JACOBIO PHARMACEUTICALS CO., LTD.) 20 August 2019 (2019-08-20)<br>abstract and description paragraphs [0005]-[0037] and paragraphs [0442]-[0443] | 1-32 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 September 2021** | **28 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/107808**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29-32**
because they relate to subject matter not required to be searched by this Authority, namely:

[1] The subject matter of claim 29 relates to a method for treating and/or preventing SHP2-mediated diseases. The subject matter of claims 30-32 relates to a method for treating cancer. They all pertain to methods for treating or preventing diseases on the living human or animal body. The present report is provided on the basis of the pharmaceutical uses.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/107808**

| Patent document cited in search report | Publication date (day/month/year) | Patent family member(s) | Publication date (day/month/year) |
|---|---|---|---|
| WO 2019183367 A1 | 26 September 2019 | SG 11202009245 T A | 29 October 2020 |
| | | US 10934302 B1 | 02 March 2021 |
| | | BR 112020019385 A2 | 30 March 2021 |
| | | TW 202003471 A | 16 January 2020 |
| | | KR 20210015758 A | 10 February 2021 |
| | | IL 277434 D0 | 30 November 2020 |
| | | CN 112166110 A | 01 January 2021 |
| | | EP 3768668 A1 | 27 January 2021 |
| | | AU 2019240299 A1 | 15 October 2020 |
| | | CA 3094690 A1 | 26 September 2019 |
| CN 105916845 A | 31 August 2016 | EP 3094628 B1 | 08 August 2018 |
| | | JO 3517 B1 | 05 July 2020 |
| | | PH 12016501336 A1 | 15 August 2016 |
| | | DO P2016000177 A | 30 September 2016 |
| | | PT 3094628 T | 21 November 2018 |
| | | CA 2935695 A1 | 23 July 2015 |
| | | IL 246436 A | 31 October 2019 |
| | | TW 201612170 A | 01 April 2016 |
| | | JP 6534389 B2 | 26 June 2019 |
| | | AP 2016009299 A0 | 30 June 2016 |
| | | IL 246436 D0 | 31 August 2016 |
| | | AU 2015207757 A1 | 07 July 2016 |
| | | CR 20200617 A | 11 February 2021 |
| | | AP 201609299 A0 | 30 June 2016 |
| | | SI 3094628 T1 | 30 November 2018 |
| | | CY 1121155 T1 | 29 May 2020 |
| | | US 2020181168 A1 | 11 June 2020 |
| | | AU 2015207757 B8 | 11 May 2017 |
| | | DK 3094628 T3 | 03 December 2018 |
| | | SV 2016005245 A | 21 November 2016 |
| | | ES 2695242 T3 | 02 January 2019 |
| | | MA 39282 A1 | 29 June 2018 |
| | | US 10968235 B2 | 06 April 2021 |
| | | KR 20160103137 A | 31 August 2016 |
| | | HR P20181832 T1 | 11 January 2019 |
| | | CR 20160328 A | 10 November 2016 |
| | | RS 57757 B1 | 31 December 2018 |
| | | PH 12016501336 B1 | 15 August 2016 |
| | | EP 3094628 A1 | 23 November 2016 |
| | | SG 11201605272S A | 30 August 2016 |
| | | GT 201600147 A | 27 November 2018 |
| | | NZ 721598 A | 31 August 2018 |
| | | JP 2017502993 A | 26 January 2017 |
| | | PE 20170011 A1 | 25 March 2017 |
| | | TW I657083 B | 21 April 2019 |
| | | PL 3094628 T3 | 31 January 2019 |
| | | US 2017015680 A1 | 19 January 2017 |
| | | AR 100033 A1 | 07 September 2016 |
| | | CN 105916845 B | 12 November 2019 |
| | | UY 35957 A | 31 August 2015 |

Form PCT/ISA/210 (patent family annex) (January 2015)

**INTERNATIONAL SEARCH REPORT**
**Information on patent family members**

International application No.
**PCT/CN2021/107808**

| Patent document cited in search report | | | Publication date (day/month/year) | Patent family member(s) | | | Publication date (day/month/year) |
|---|---|---|---|---|---|---|---|
| | | | | LT | 3094628 | T | 25 October 2018 |
| | | | | EA | 201691442 | A1 | 30 December 2016 |
| | | | | CL | 2016001790 | A1 | 30 December 2016 |
| | | | | AU | 2015207757 | A8 | 11 May 2017 |
| | | | | US | 10336774 | B2 | 02 July 2019 |
| | | | | US | 10077276 | B2 | 18 September 2018 |
| | | | | WO | 2015107495 | A1 | 23 July 2015 |
| | | | | EA | 031573 | B1 | 31 January 2019 |
| | | | | AU | 2015207757 | B2 | 06 April 2017 |
| WO | 2020063760 | A1 | 02 April 2020 | CN | 112839935 | A | 25 May 2021 |
| WO | 2019118909 | A1 | 20 June 2019 | CA | 3084058 | A1 | 20 June 2019 |
| | | | | AU | 2018385713 | A1 | 18 June 2020 |
| | | | | SG | 11202004090 Y | A | 28 May 2020 |
| | | | | BR | 112020009757 | A2 | 03 November 2020 |
| | | | | JP | 2021506776 | A | 22 February 2021 |
| | | | | CN | 111433205 | A | 17 July 2020 |
| | | | | TW | 201927791 | A | 16 July 2019 |
| | | | | IL | 275089 | D0 | 30 July 2020 |
| | | | | PH | 12020550857 | A1 | 17 May 2021 |
| | | | | EP | 3724189 | A1 | 21 October 2020 |
| | | | | KR | 20200099530 | A | 24 August 2020 |
| | | | | US | 2020407372 | A1 | 31 December 2020 |
| | | | | CO | 2020005887 | A2 | 10 August 2020 |
| CN | 110143949 | A | 20 August 2019 | CN | 112409334 | A | 26 February 2021 |
| | | | | CN | 112174935 | A | 05 January 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019183367 A


### Non-patent literature cited in the description


- **CHAN R J et al.** PTPN11 is the first identified proto-oncogene that encodes a tyrosine phosphatase. *Blood,* 2007, vol. 109, 862-867
- **DANCE M et al.** The molecular functions of SHP2 in the RAS/mitogen-activated protein kinase (ERK1/2) pathway. *Cell Signal,* 2008, vol. 20, 453-459
- **TARTAGLIA M et al.** Mutations in PTPN11, encoding the protein tyrosine phosphatase SHP-2, cause Noonan sydrome. *Nat Genet,* 2001, vol. 29, 465-468
- **TARTAGLIA M et al.** *Nat Genet,* 2003, vol. 34, 148-150
- **LOH ML et al.** *Blood,* 2004, vol. 103, 2325-2331
- **TARTAGLIA M et al.** *Br J Haematol,* 2005, vol. 129, 333-339
- **XU R et al.** *Blood,* 2005, vol. 106, 3142-3149
- **TANG CHUNLAN et al.** *Chinese Journal of Lung Cancer,* 2010, vol. 13, 98-101
- **HIGUCHI M et al.** *Cancer Sci,* 2004, vol. 95, 442-447
- **BENTIRES-ALJ M et al.** *Cancer Res,* 2004, vol. 64, 8816-8820
- **MARTINELLI S et al.** *Cancer Genet Cytogenet,* 2006, vol. 166, 124-129
- **H. BUNDGAARD.** Design of Prodrugs. Elsevier, 1985